# EUROPEAN PATENT APPLICATION

(11) **EP 4 501 363 A1**
(43) Date of publication of application: **05.02.2025**
(21) Application number: 23778380.8
(22) Date of filing: 30.03.2023
(51) Int. Cl.: A61K 47/68, A61K 39/00, C07K 16/18, A61P 35/00, A61K 39/395

(54) **B7H4 ANTIBODY-DRUG CONJUGATE AND USE THEREOF**

(30) Priority: 30.03.2022 CN 202210334522
(71) Applicant: Duality Biologics (Suzhou) Co., Ltd., Suzhou, Jiangsu 215000 (CN)
(72) Inventor: ZHU, Zhongyuan, Shanghai 201204 (CN); ZHONG, Chen, Shanghai 201204 (CN); ZHANG, Yu, Shanghai 201204 (CN); ZHOU, Yunhua, Shanghai 201204 (CN)
(74) Representative: Mewburn Ellis LLP
(86) International application number: PCT/CN2023/085097
(87) International publication number: WO 2023/186015

(57) **Abstract**

The present invention provides an anti-B7H4 antibody-drug conjugate specifically binding to B7H4, and a composition comprising same, wherein the structure of the anti-B7H4 antibody-drug conjugate is shown as formula (1-1). The present invention further provides a method and use of the antibody-drug conjugate provided herein. The anti-B7H4 antibody-drug conjugate of the present invention has better inhibitory activity against tumour cell proliferation in vitro and a better tumour inhibition effect in vivo.

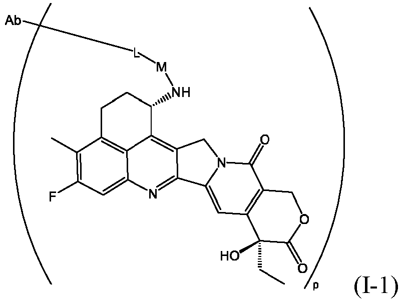

## Description

The present application claims the right of priority for the Chinese patent application 202210334522X with the filing date of 30 March 2022. This Chinese patent application is incorporated herein by reference in its entirety.

### Technical Field

The present invention provides an antibody-drug conjugate specifically binding to B7H4, and a composition comprising same. Also provided are a method and use of the antibody-drug conjugate provided herein.

### Background Art

Immune checkpoint inhibitors are the most studied form of immunotherapy for tumours. Immune checkpoint molecules are often highly expressed in tumour microenvironment, which promotes tumours to evade the attack from the immune system by inhibiting T cell activation and inducing T cell exhaustion. The B7 family and the TNF family are the two major families of co-stimulatory molecules, with the B7 family currently comprising 10 molecules: CD80 (B7.1), CD86 (B7.2), B7H1 (PD-L1/CD274), B7-DC (PD-L2/CD273), B7H2 (ICOSL), B7H3 (CD276), B7H4 (B7S1/B7x/Vtcn1), B7H5 (VISTA), B7H6 and B7H7 (HHLA2). Several members of the B7 family and their receptors, such as PD-L1/PD1, CTLA4 and VISTA, have been demonstrated to be immune checkpoints.

B7H4 is a relatively new member of the B7 family. Although it is widely expressed in cells of organisms at the mRNA level, its expression at the protein level is very limited, with low expression only on epithelial cells of some ducts in organisms, such as the lactiferous ducts, lobules of the mammary gland and fallopian tube, and tissues such as the endometrium. In contrast, B7H4 is abundantly expressed in a variety of tumour tissues, such as breast cancer tumour cells, particularly triple negative breast cancer tumour cells, ovarian cancer tumour cells, and endometrial tumour cells. In terms of expression profile, B7H4 can be considered a highly specific tumour-associated antigen. On the other hand, B7H4 is a new immune checkpoint molecule, and *in vitro* experiments demonstrate that B7H4 inhibits T cell proliferation, activation and cytokine production by interacting with its unknown T cell receptor. With the help of highly expressed B7H4 molecules and suppressive macrophages with high expression of B7H4 molecules in the tumour microenvironment, tumour cells inhibit T cell activation so as to achieve immune evasion. The expression profile of B7H4 on tumours does not overlap with that of PD-L1. It is a promising means to treat tumours positive for B7H4 expression by reactivating the immune system through the treatment with B7H4-targeting antibodies and the blockade of the negative regulatory effect of B7H4.

Monoclonal antibodies or antibody-drug conjugates or bispecific antibodies against B7-H4 are currently being developed by several pharmaceutical companies. Antibody-drug conjugates that have been marketed are Adcetris and Kadcyla. Several multinational pharmaceutical companies are currently developing monoclonal antibodies or antibody-drug conjugates against B7-H4 to enhance the immune response of the patient's immune system against tumours and to achieve direct tumour cell killing. Related patents are WO2013025779, US 20140322129, etc. Anti-B7-H4 monoclonal antibodies from companies such as Medimmune and FivePrime are currently in preclinical development, and the anti-B7-H4 antibody-drug conjugate from Genentech is also in preclinical development.

### Summary of the Invention

A technical problem to be solved by the present invention is to provide an anti-B7H4 antibody-drug conjugate, a preparation method therefor and the use thereof for overcoming the defects of deficient anti-B7H4 antibody-drug conjugates in the prior art. Compared with the prior art, the anti-B7H4 antibody-drug conjugate of the present invention has one or more advantageous effects selected from the following group: (1) better inhibitory activity against tumour cell proliferation *in vitro*; (*2*) better endocytosis; (3) a better tumour inhibition effect *in vivo*; (4) better affinity for human and monkey B7H4; (5) a better B7H4-targeting property; (6) a better bystander killing effect; (7) improved plasma stability; and (8) better safety.

The present invention solves the above-mentioned technical problems mainly by the following technical means.

In one aspect, the present application provides an anti-B7H4 antibody-drug conjugate, an isomer thereof, a pharmaceutically acceptable salt thereof or a mixture thereof, wherein the structure of the anti-B7H4 antibody-drug conjugate is shown as formula (I):

Ab-(L-M-D)ₚ (I)

wherein,
L and M are linker units;
D is a cytotoxic drug;
p represents the average connection number, and p is an integer or decimal selected from 1 to 10, preferably an integer or decimal from 3 to 8;
Ab is an anti-B7H4 antibody or an antigen-binding fragment thereof comprising a heavy chain variable region and a light chain variable region, wherein the heavy chain variable region comprises HCDR1, HCDR2 and HCDR3 having the amino acid sequences as shown in SEQ ID NO: 1, SEQ ID NO:2 and SEQ ID NO:3, respectively, and the light chain variable region comprises LCDR1, LCDR2 and LCDR3 having the amino acid sequences as shown in SEQ ID NO:4, SEQ ID NO:5 and SEQ ID NO:6, respectively.

In one aspect, the present application provides an anti-B7H4 antibody-drug conjugate, an isomer thereof, a pharmaceutically acceptable salt thereof or a mixture thereof, wherein the structure of the anti-B7H4 antibody-drug conjugate is shown as formula (I):

Ab-(L-M-D)ₚ (I)

wherein,
L and M are linker units;
-M-D is a cytotoxic drug;
p represents the average connection number, and p is an integer or decimal selected from 1 to 10, preferably an integer or decimal from 3 to 8;
Ab is an anti-B7H4 antibody or an antigen-binding fragment thereof comprising a heavy chain variable region and a light chain variable region, wherein the heavy chain variable region comprises HCDR1, HCDR2 and HCDR3 having the amino acid sequences as shown in SEQ ID NO: 1, SEQ ID NO:2 and SEQ ID NO:3, respectively, and the light chain variable region comprises LCDR1, LCDR2 and LCDR3 having the amino acid sequences as shown in SEQ ID NO:4, SEQ ID NO:5 and SEQ ID NO:6, respectively.

In one aspect, the present application provides an anti-B7H4 antibody-drug conjugate, an isomer thereof, a pharmaceutically acceptable salt thereof or a mixture thereof, wherein the structure of the anti-B7H4 antibody-drug conjugate is shown as formula (I-1): 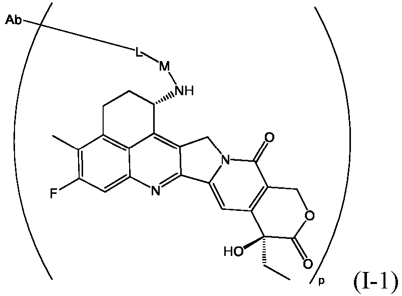 wherein,
M is -L²-L¹-C(O)-;
L² is -O- or -S-;
L¹ is -(C(R^{1a})(R^{1b}))ₘ-CH₂-, saturated C₃-C₆ cycloalkyl or 3- to 6-membered saturated heterocyclyl, wherein the saturated C₃-C₆ cycloalkyl and 3- to 6-membered saturated heterocyclyl are each independently and optionally substituted with one or more R^{2a};
m is selected from 1, 2, 3 or 4; the 3- to 6-membered saturated heterocyclyl has 1-3 heteroatoms selected from N, O and S;
each R^{1a} is independently selected from hydrogen, halogen, hydroxyl, amino and C₁-C₆ alkyl, wherein the C₁-C₆ alkyl is optionally substituted with one or more R;
R^{1b} and R^{2a} are each independently selected from hydrogen, halogen, hydroxyl, amino and C₁-C₆ alkyl, wherein the C₁-C₆ alkyl is optionally substituted with one or more R;
each R is independently hydrogen or halogen;
L is a linker unit;
p represents the average connection number, and p is an integer or decimal selected from 1 to 10;
Ab is an anti-B7H4 antibody or an antigen-binding fragment thereof.

In some embodiments, the average connection number p is preferably any integer or decimal from 3 to 8.

In certain preferred embodiments of the present invention, in the compound or pharmaceutically acceptable salt thereof shown as formula (I-1), (II-1) or (II-2), some groups are as defined below, and the groups not mentioned are as defined in any scheme in the present application (abbreviated as "in some embodiments").

In some embodiments, the present invention provides the anti-B7H4 antibody-drug conjugate, the isomer thereof, the pharmaceutically acceptable salt thereof or the mixture thereof as described herein, wherein each R^{1a} is independently selected from halogen, hydroxyl, amino and C₁-C₆ alkyl, the C₁-C₆ alkyl is optionally substituted with one or more R, and each R is independently hydrogen or halogen.

In some embodiments, the anti-B7H4 antibody or the antigen-binding fragment thereof of the present invention comprises a heavy chain variable region and a light chain variable region, wherein the heavy chain variable region comprises HCDR1, HCDR2 and HCDR3 having the amino acid sequences as shown in SEQ ID NO:1, SEQ ID NO:2 and SEQ ID NO:3, respectively, and the light chain variable region comprises LCDR1, LCDR2 and LCDR3 having the amino acid sequences as shown in SEQ ID NO:4, SEQ ID NO:5 and SEQ ID NO:6, respectively.

In some embodiments, the anti-B7H4 antibody or the antigen-binding fragment thereof of the present invention comprises: a heavy chain variable region with an amino acid sequence as shown in SEQ ID NO:7 or an amino acid sequence having at least 95%, 96%, 97%, 98% or 99% identity to SEQ ID NO:7, and a light chain variable region with an amino acid sequence as shown in SEQ ID NO:8 or an amino acid sequence having at least 95%, 96%, 97%, 98% or 99% identity to SEQ ID NO:8.

In some embodiments, the anti-B7H4 antibody or the antigen-binding fragment thereof of the present invention comprises a heavy chain variable region with an amino acid sequence as shown in SEQ ID NO:7, and a light chain variable region with an amino acid sequence as shown in SEQ ID NO:8.

In some embodiments, the anti-B7H4 antibody or the antigen-binding fragment thereof of the present invention is a murine antibody or a fragment thereof, a chimeric antibody or antigen-binding fragment, a humanized antibody or antigen-binding fragment, or a fully human antibody or antigen-binding fragment.

In some embodiments, the anti-B7H4 antibody or the antigen-binding fragment thereof of the present invention is a humanized antibody or a fragment thereof.

In some embodiments, the anti-B7H4 antibody or the antigen-binding fragment thereof of the present invention is selected from Fab, Fab', Fab'-SH, Fv, scFv, F(ab')₂, sdAb, diabodies or linear antibodies.

In some embodiments, the anti-B7H4 antibody of the present invention is a monoclonal antibody.

In some embodiments, the antibody of the present invention is an antibody in the form of IgG1, IgG2, IgG3 or IgG4.

In some embodiments, the antibody of the present invention is an antibody in the form of IgG1.

In some embodiments, the anti-B7H4 antibody or the antigen-binding fragment thereof of the present invention comprises: a heavy chain with an amino acid sequence as shown in SEQ ID NO: 9 or an amino acid sequence having at least 95%, 96%, 97%, 98% or 99% identity to SEQ ID NO: 9, and a light chain with an amino acid sequence as shown in SEQ ID NO: 10 or an amino acid sequence having at least 95%, 96%, 97%, 98% or 99% identity to SEQ ID NO:10.

In some embodiments, the anti-B7H4 antibody or the antigen-binding fragment thereof of the present invention comprises a heavy chain with an amino acid sequence as shown in SEQ ID NO: 9, and a light chain with an amino acid sequence as shown in SEQ ID NO: 10.

In some embodiments, the anti-B7H4 antibody of the present invention is an anti-B7H4 antibody DB1001. The amino acid sequence of DB1001 is as shown in the sequence listing. In some embodiments, the present invention provides the anti-B7H4 antibody-drug conjugate, the isomer thereof, the pharmaceutically acceptable salt thereof or the mixture thereof as described herein, wherein the L² end of M is linked to the linker unit L.

In some embodiments, the present invention provides the anti-B7H4 antibody-drug conjugate, the isomer thereof, the pharmaceutically acceptable salt thereof or the mixture thereof as described herein, wherein L¹ is -(C(R^{1a})(R^{1b}))ₘ-CH₂-; R^{1a} is selected from: hydrogen, halogen and C₁-C₆ alkyl; R^{1b} is selected from: hydrogen, halogen and C₁-C₆ alkyl; for example, R^{1a} is selected from: halogen and C₁-C₆ alkyl; R^{1b} is selected from: hydrogen, halogen and C₁-C₆ alkyl.

In some embodiments, the present invention provides the anti-B7H4 antibody-drug conjugate, the isomer thereof, the pharmaceutically acceptable salt thereof or the mixture thereof as described herein, wherein L¹ is -(C(R^{1a})(R^{1b}))ₘ-CH₂-; R^{1a} is hydrogen or -CH₃; R^{1b} is selected from: hydrogen and -CH₃; for example, R^{1a} is - CH₃; R^{1b} is selected from: hydrogen and -CH₃.

In some embodiments, the present invention provides the anti-B7H4 antibody-drug conjugate, the isomer thereof, the pharmaceutically acceptable salt thereof or the mixture thereof as described herein, wherein L¹ is -(C(R^{1a})(R^{1b}))ₘ-CH₂-; m is 1 or 2.

In some embodiments, the present invention provides the anti-B7H4 antibody-drug conjugate, the isomer thereof, the pharmaceutically acceptable salt thereof or the mixture thereof as described herein, wherein L¹ is selected from: 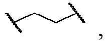 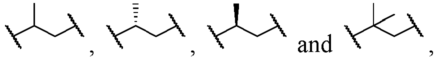 wherein the left side of the structural fragment is preferably linked to L².

In some embodiments, the present invention provides the anti-B7H4 antibody-drug conjugate, the isomer thereof, the pharmaceutically acceptable salt thereof or the mixture thereof as described herein, wherein L¹ is saturated C₃-C₆ cycloalkyl or 3- to 6-membered saturated heterocyclyl, wherein the saturated C₃-C₆ cycloalkyl and 3- to 6-membered saturated heterocyclyl are each independently and optionally substituted with one or more R^{2a}, and each R^{2a} is independently selected from: hydrogen, halogen and C₁-C₆ alkyl.

In some embodiments, the present invention provides the anti-B7H4 antibody-drug conjugate, the isomer thereof, the pharmaceutically acceptable salt thereof or the mixture thereof as described herein, wherein L² and -C(O)- are linked to different atoms in saturated C₃-C₆ cycloalkyl or 3- to 6-membered saturated heterocyclyl.

In some embodiments, the present invention provides the anti-B7H4 antibody-drug conjugate, the isomer thereof, the pharmaceutically acceptable salt thereof or the mixture thereof as described herein, wherein L¹ is saturated C₃-C₆ cycloalkyl optionally substituted with one or more R^{2a}; each R^{2a} is independently selected from: hydrogen, halogen and C₁-C₆ alkyl.

In some embodiments, the present invention provides the anti-B7H4 antibody-drug conjugate, the isomer thereof, the pharmaceutically acceptable salt thereof or the mixture thereof as described herein, wherein L¹ is saturated C₃-C₆ cycloalkyl. For example, L¹ is cyclopropyl, cyclobutyl, cyclopentyl or cyclohexyl, preferably is cyclobutyl.

In some embodiments, the present invention provides the anti-B7H4 antibody-drug conjugate, the isomer thereof, the pharmaceutically acceptable salt thereof or the mixture thereof as described herein, wherein L¹ is: 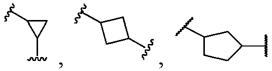 and 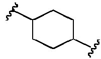 optionally substituted with 1, 2 or 3 R^{2a}; each R^{2a} is independently selected from: hydrogen, halogen and C₁-C₆ alkyl.

In some embodiments, the present invention provides the anti-B7H4 antibody-drug conjugate, the isomer thereof, the pharmaceutically acceptable salt thereof or the mixture thereof as described herein, wherein L¹ is selected from:

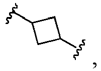

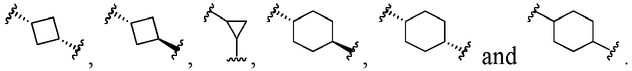

In some embodiments, the present invention provides the anti-B7H4 antibody-drug conjugate, the isomer thereof, the pharmaceutically acceptable salt thereof or the mixture thereof as described herein, wherein
M is -L²-L¹-C(O)-;
L² is -O-;
L¹ is -(C(R^{1a})(R^{1b}))ₘ-CH₂- or saturated C₃-C₆ cycloalkyl, wherein the saturated C₃-C₆ cycloalkyl is optionally substituted with one or more R^{2a};
m is selected from 1 or 2;
each R^{1a} is independently selected from hydrogen, halogen and C₁-C₆ alkyl, wherein the C₁-C₆ alkyl is optionally substituted with one or more R;
R^{1b} and R^{2a} are each independently selected from hydrogen, halogen and C₁-C₆ alkyl, wherein the C₁-C₆ alkyl is optionally substituted with one or more R;
each R is independently hydrogen or halogen.

In some embodiments, the present invention provides the anti-B7H4 antibody-drug conjugate, the isomer thereof, the pharmaceutically acceptable salt thereof or the mixture thereof as described herein, wherein
M is -L²-L¹-C(O)-;
L² is -O-;
L¹ is -(C(R^{1a})(R^{1b}))ₘ-CH₂-, or 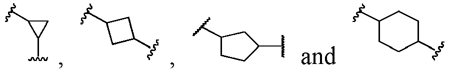 optionally substituted with 1, 2 or 3 R^{2a};
m is selected from 1 or 2;
each R^{1a} is independently selected from halogen and C₁-C₆ alkyl, wherein the C₁-C₆ alkyl is optionally substituted with one or more R;
R^{1b} and R^{2a} are each independently selected from hydrogen, halogen and C₁-C₆ alkyl, wherein the C₁-C₆ alkyl is optionally substituted with one or more R;
each R is independently hydrogen or halogen.

In some embodiments, the present invention provides the anti-B7H4 antibody-drug conjugate, the isomer thereof, the pharmaceutically acceptable salt thereof or the mixture thereof as described herein, wherein the -M- is selected from:

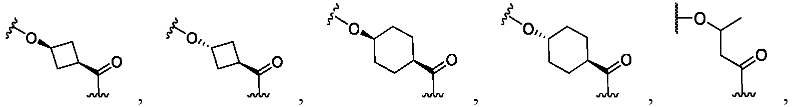

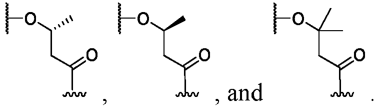

In some embodiments, the present invention provides the anti-B7H4 antibody-drug conjugate, the isomer thereof, the pharmaceutically acceptable salt thereof or the mixture thereof as described herein, wherein the -M- is:

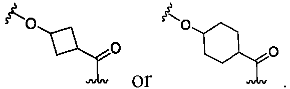

In some embodiments, the present invention provides the anti-B7H4 antibody-drug conjugate, the isomer thereof, the pharmaceutically acceptable salt thereof or the mixture thereof as described herein, wherein the -M- is:

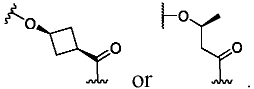

In some embodiments, the present invention provides the anti-B7H4 antibody-drug conjugate as described herein, wherein the cytotoxic drug is selected from any one of the following structures:

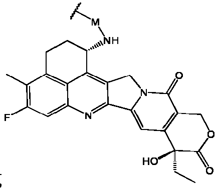

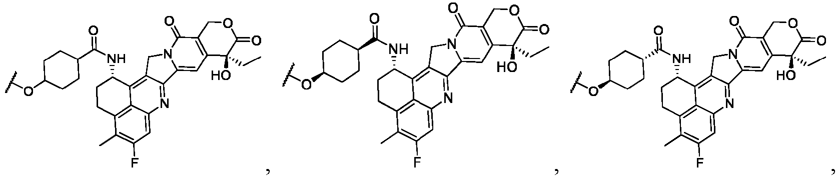

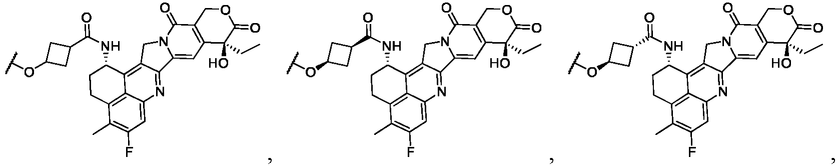

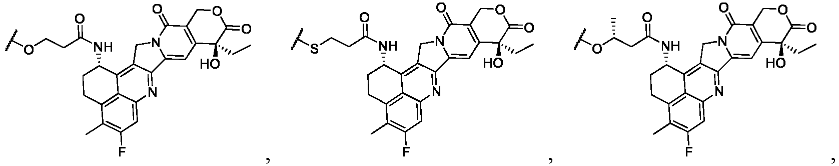

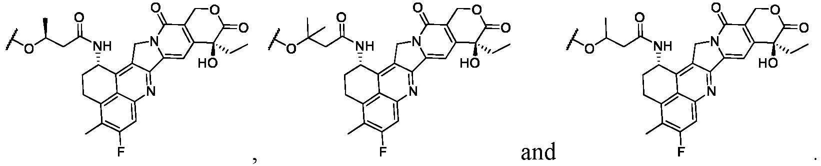

In some embodiments, the present invention provides the anti-B7H4 antibody-drug conjugate, the isomer thereof, the pharmaceutically acceptable salt thereof or the mixture thereof as described herein, wherein the L is -Lₐ-L_{b}-L_{c}-,
the -Lₐ- is 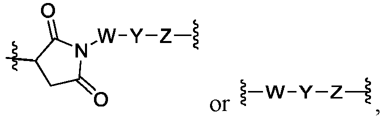
wherein, W is -(C(R^{wa})(R^{wb}))_{wn}-, Y is -(OCH₂CH₂)_{yn}-O_{yp}-, and Z is - (C(R^{za})(R^{zb}))_{zn},
wherein wn is 1, 2, 3 or 6,
0 or 1 methylene unit of W is each independently replaced with -Cyr-, - N(R^{wx})C(O)-, -C(O)N(R^{wx})-, or -C(O)-,
yn is 0, 4 or 8, yp is 0 or 1;
zn is 1, 2 or 3,
1 methylene unit of Z is each independently replaced with -Cyr-, -N(R^{zx})C(O)-, -C(O)N(R^{zx})-, or -C(O)-;
-Cyr- is 3- to 10-membered saturated cycloalkyl, and the -Cyr- is unsubstituted or independently substituted with 1 to 3 substituents R^{cx};
R^{wa}, R^{wb}, R^{za}, R^{zb}, R^{wx}, R^{zx}, and R^{cx} are each independently hydrogen, halogen, or -OR^{r}, or C₁₋₆ alkyl optionally substituted with R^{f},
wherein each R^{r} is independently hydrogen, halogen or C₁₋₆ alkyl;
the -L_{b}- represents a peptide residue composed of 2 to 7 amino acids, and the peptide residue of the -L_{b}- is a peptide residue formed by the amino acids selected from the following group: phenylalanine, glycine, alanine, valine, citrulline, lysine, serine, glutamic acid, and aspartic acid; preferably, -L_{b}- represents a peptide residue composed of 2 to 4 amino acids, and the peptide residue of the -L_{b}- is a peptide residue formed by the amino acids selected from the following group: phenylalanine, glycine, alanine, valine, citrulline and lysine;
the -L_{c}- is 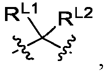
wherein R^{L1} and R^{L2} are each independently selected from the following group: hydrogen, halogen, -OH and C₁₋₆ alkyl.

In some embodiments, the present invention provides the anti-B7H4 antibody-drug conjugate, the isomer thereof, the pharmaceutically acceptable salt thereof or the mixture thereof as described herein, wherein the L is -Lₐ-L_{b}-L_{c}-,
the -Lₐ- is 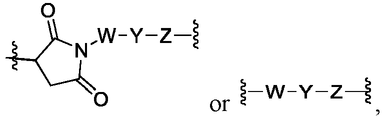
wherein, W is -(C(R^{wa})(R^{wb}))_{wn}-, Y is -(OCH₂CH₂)_{yn}-O_{yp}-, and Z is - (C(R^{za})(R^{zb}))_{zn},
wherein wn is 1, 2, 3 or 6,
0 or 1 methylene unit of W is each independently replaced with -Cyr-, - N(R^{wx})C(O)-, -C(O)N(R^{wx})-, or -C(O)-,
yn is 0, 4 or 8, yp is 0 or 1;
zn is 1, 2 or 3,
1 methylene unit of Z is each independently replaced with -Cyr-, -N(R^{zx})C(O)-, -C(O)N(R^{zx})-, or -C(O)-;
-Cyr- is 3- to 10-membered saturated cycloalkyl, and the -Cyr- is unsubstituted or independently substituted with 1 to 3 substituents R^{cx};
R^{wa}, R^{wb}, R^{za}, R^{zb}, R^{wx}, R^{zx}, and R^{cx} are each independently hydrogen, halogen, or -OR^{r}, or C₁₋₆ alkyl optionally substituted with R^{f},
wherein each R^{r} is independently hydrogen, halogen or C₁₋₆ alkyl;
the -L_{b}- is selected from the following group: 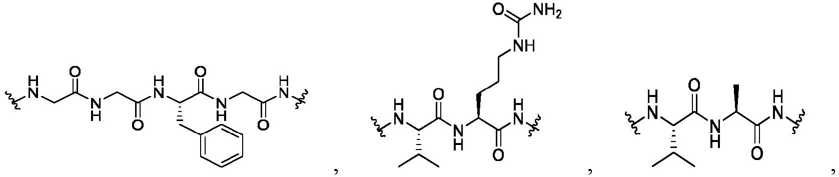 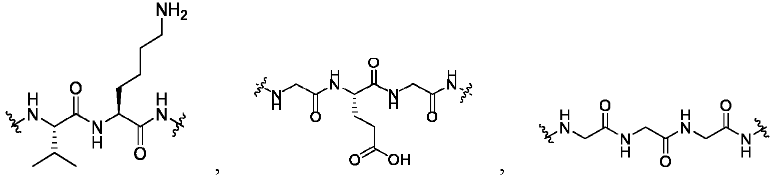 and 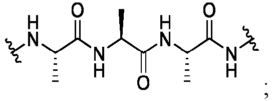
the -L_{c}- is 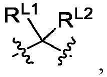
wherein R^{L1} and R^{L2} are each independently selected from the following group: hydrogen, halogen, -OH and C₁₋₆ alkyl.

In some embodiments, the present invention provides the anti-B7H4 antibody-drug conjugate, the isomer thereof, the pharmaceutically acceptable salt thereof or the mixture thereof as described herein, wherein the -Lₐ- is 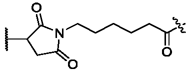 or 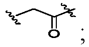 preferably

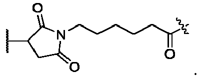

In some embodiments, the present invention provides the anti-B7H4 antibody-drug conjugate, the isomer thereof, the pharmaceutically acceptable salt thereof or the mixture thereof as described herein, wherein the -L_{b}- is 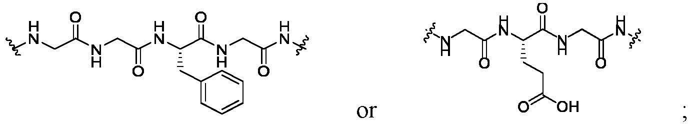 preferably

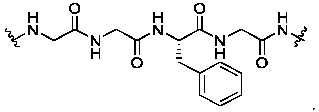

In some embodiments, the present invention provides the anti-B7H4 antibody-drug conjugate, the isomer thereof, the pharmaceutically acceptable salt thereof or the mixture thereof as described herein, wherein the -L_{c}- is

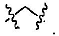

In some embodiments, the present invention provides the anti-B7H4 antibody-drug conjugate, the isomer thereof, the pharmaceutically acceptable salt thereof or the mixture thereof as described herein, wherein the Lₐ end of the linker unit L is linked to Ab, and the L_{c} end thereof is linked to M.

In some embodiments, the present invention provides the anti-B7H4 antibody-drug conjugate, the isomer thereof, the pharmaceutically acceptable salt thereof or the mixture thereof as described herein, wherein the Lₐ end of the linker unit L is linked to Ab, and the L_{c} end thereof is linked to linker unit M.

In some embodiments, the present invention provides the anti-B7H4 antibody-drug conjugate, the isomer thereof, the pharmaceutically acceptable salt thereof or the mixture thereof as described herein, wherein the L is

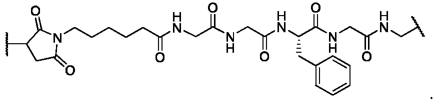

In some embodiments, the present invention provides the anti-B7H4 antibody-drug conjugate as described herein, wherein the linker unit L is

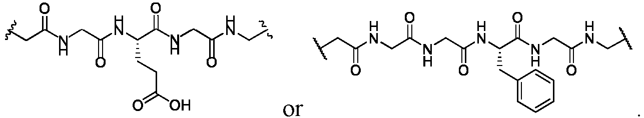

In some embodiments, the present invention provides the anti-B7H4 antibody-drug conjugate as described herein, wherein the structure of the conjugate is shown as formula (II-A): 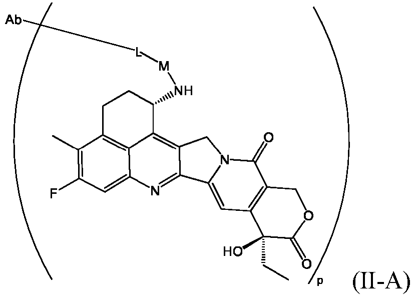 wherein, p represents the average connection number, and p is any integer or decimal from 1 to 10, preferably, any integer or decimal from 3 to 8; Ab, L and M are as defined in any embodiment of the present invention, respectively.

In some embodiments, the present invention provides the anti-B7H4 antibody-drug conjugate, the isomer thereof, the pharmaceutically acceptable salt thereof or the mixture thereof as described herein, wherein the structure of the anti-B7H4 antibody-drug conjugate is shown as formula (II-1) or (II-2): 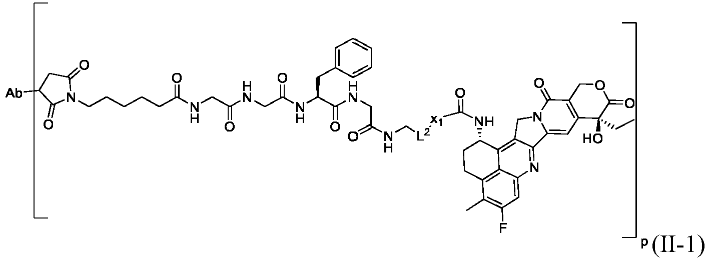 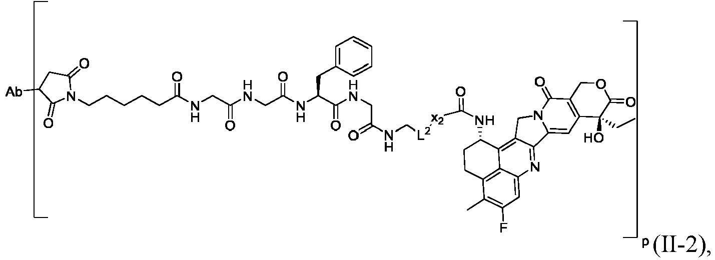 wherein,
p represents the average connection number, and p is an integer or decimal selected from 1 to 10, preferably an integer or decimal from 3 to 8;
Ab is the antibody or the antigen-binding fragment thereof as described in any embodiment herein;
L² is -O- or -S-, preferably -O-;
X₁ is selected from saturated C₃-C₆ cycloalkyl optionally substituted with 1, 2 or 3 R^{2a}, preferably 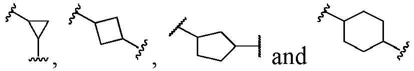 optionally substituted with 1, 2 or 3 R^{2a};
X₂ is selected from -(C(R^{1a})(R^{1b}))ₘ-CH₂-;
m is selected from 1 or 2;
R^{1a} is hydrogen or halogen, or C₁-C₆ alkyl optionally substituted with 1, 2 or 3 R, preferably halogen, or C₁-C₆ alkyl optionally substituted with 1, 2 or 3 R; R^{1b} and R^{2a} can each independently be hydrogen or halogen, or C₁-C₆ alkyl optionally substituted with 1, 2 or 3 R;
each R can independently be hydrogen or halogen.

In some embodiments, the present invention provides the anti-B7H4 antibody-drug conjugate, the isomer thereof, the pharmaceutically acceptable salt thereof or the mixture thereof as described herein, wherein the anti-B7H4 antibody-drug conjugate is selected from the following structural formulas: 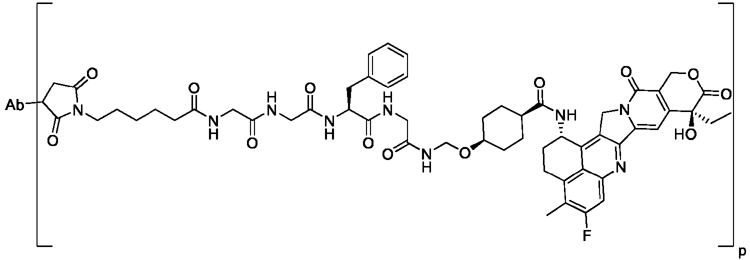 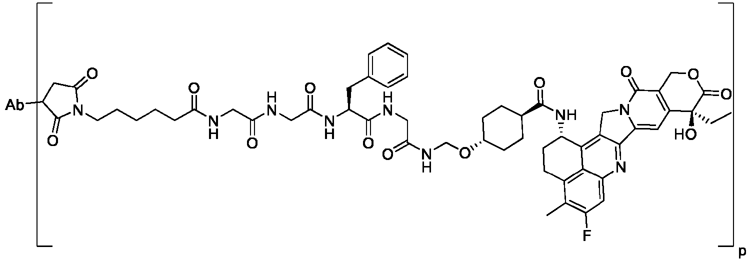 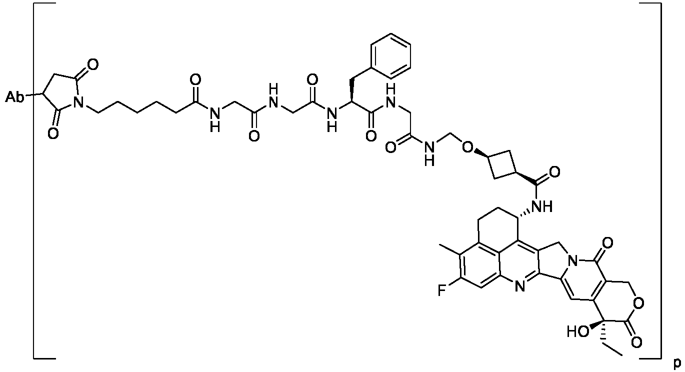 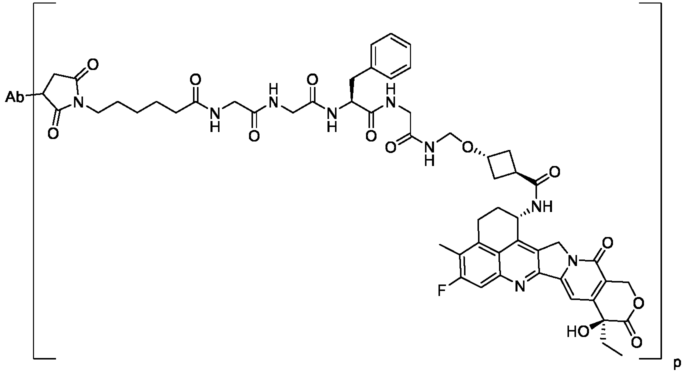 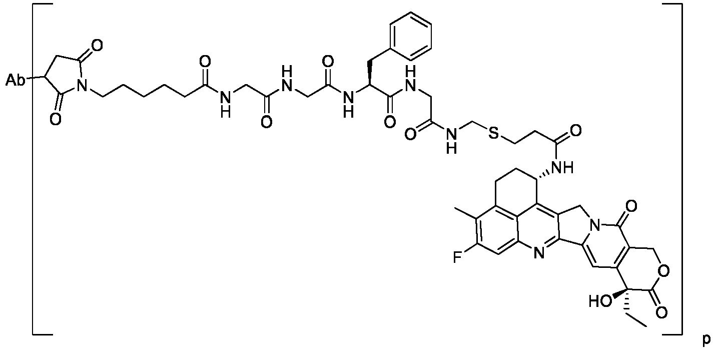 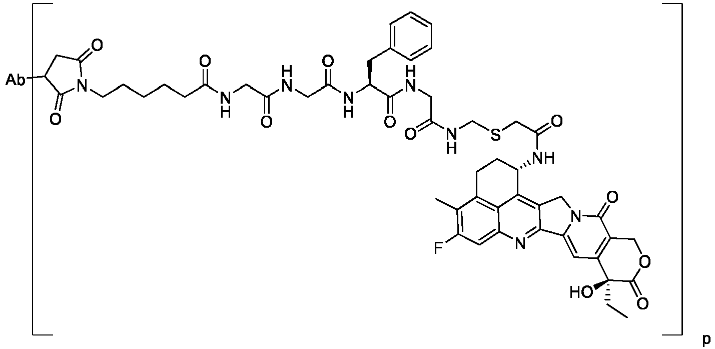 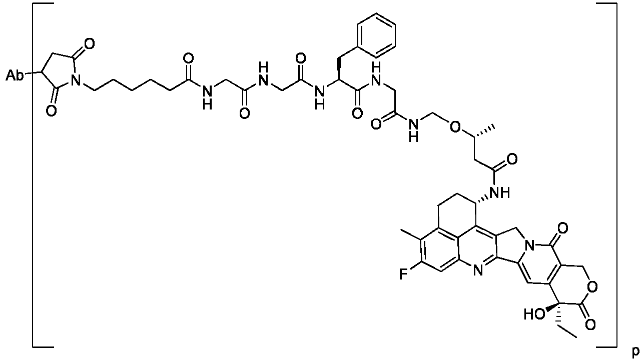 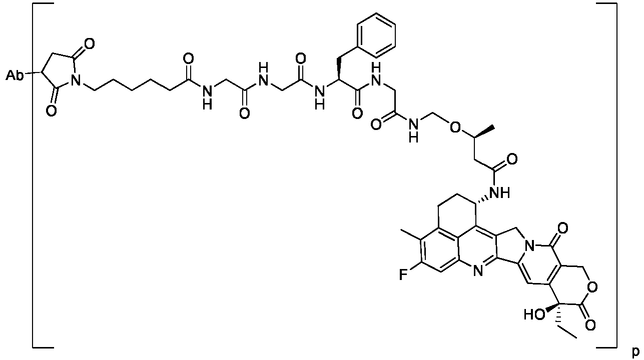 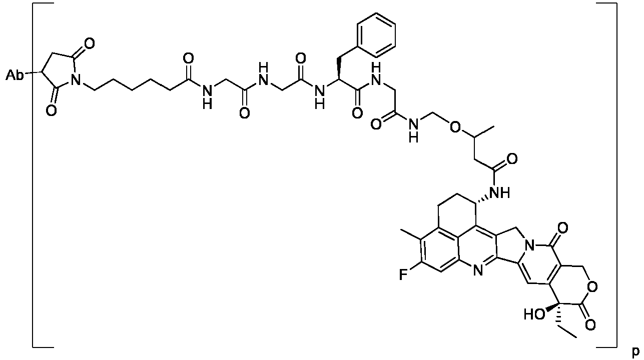 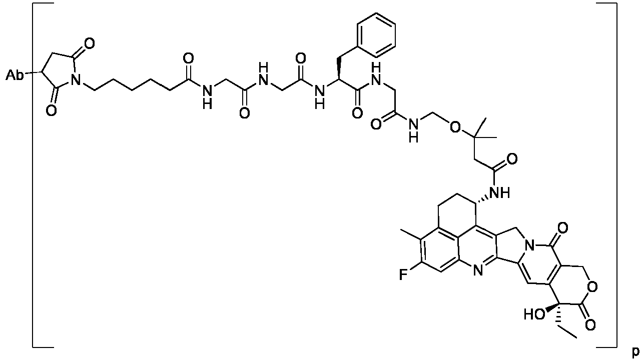 wherein,
p represents the average connection number, and p is an integer or decimal selected from 1 to 10, preferably an integer or decimal from 3 to 8;
Ab is the anti-B7H4 antibody or the antigen-binding fragment thereof as described in any embodiment herein.

In yet another aspect, the present invention provides an anti-B7H4 antibody-drug conjugate, an isomer thereof, a pharmaceutically acceptable salt thereof or a mixture thereof, wherein the anti-B7H4 antibody-drug conjugate is selected from: 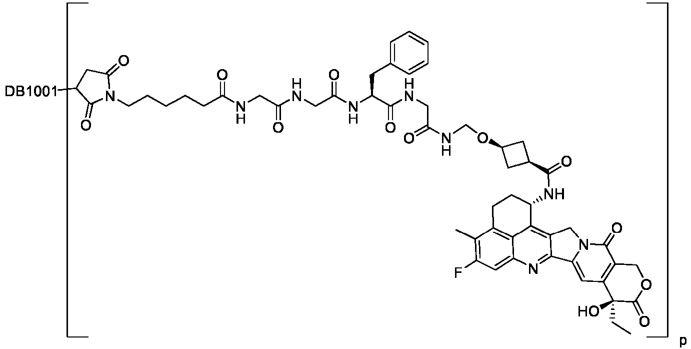 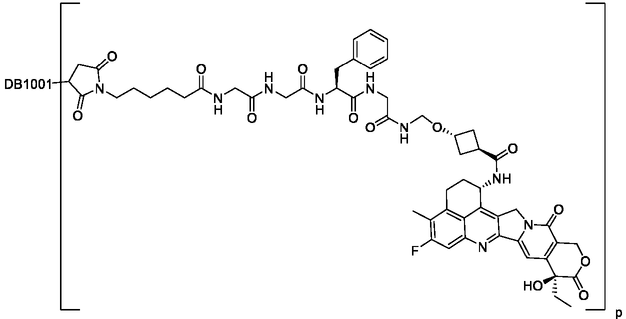 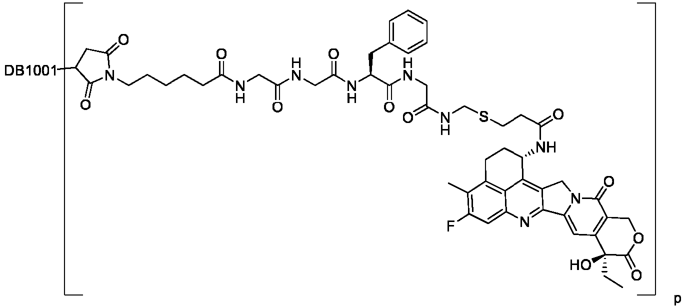 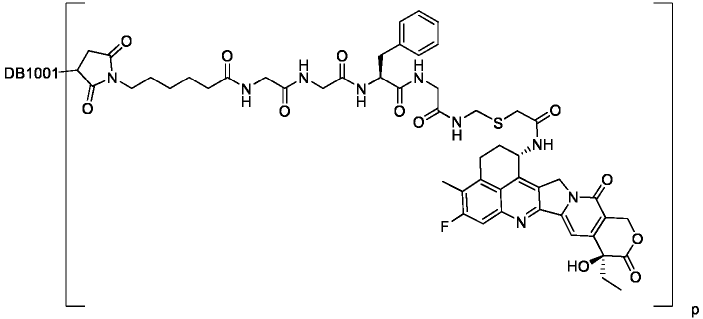 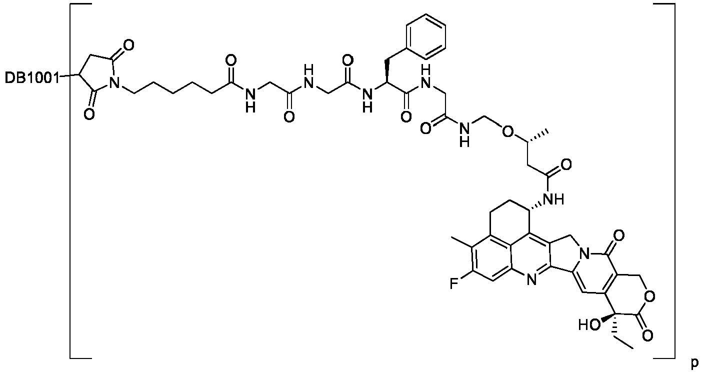 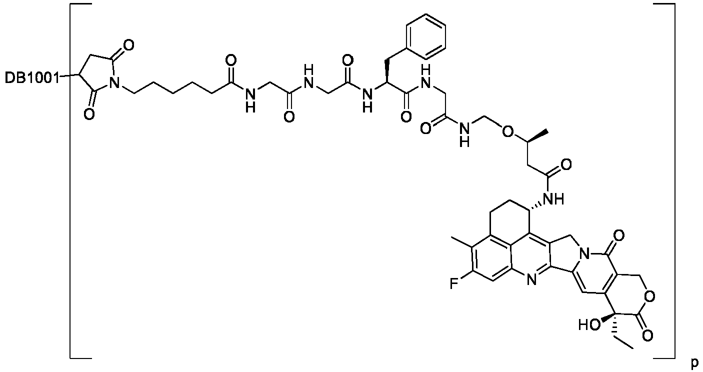 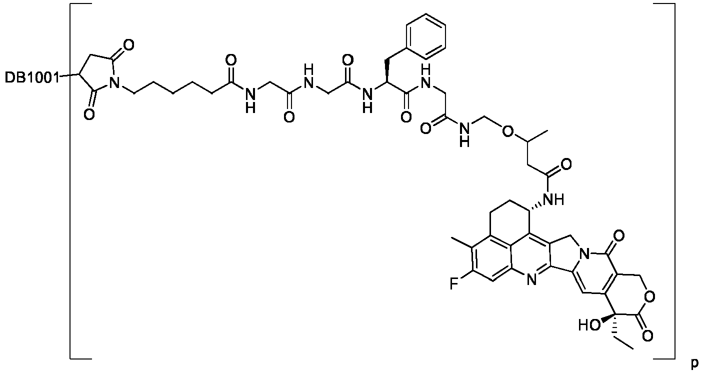 wherein,
p represents the average connection number, and p is an integer or decimal selected from 1 to 10, preferably an integer or decimal from 3 to 8. The amino acid sequence of the DB1001 is as shown in the sequence listing. The average connection number p is 3.89, or p is 5.4.

In some embodiments, the average connection number p of the present invention is selected from an integer or decimal from 1 to 10.

In some embodiments, the average connection number p of the present invention can be an integer or decimal from 2 to 8. For example, the average connection number p can be an integer or decimal from 3 to 8. For example, the average connection number p can be an integer or decimal from 1 to 2, from 2 to 3, from 3 to 4, from 4 to 5, from 5 to 6, from 6 to 7, from 7 to 8, from 8 to 9, or from 9 to 10.

In yet another aspect, the present invention provides a method for preparing an antibody-drug conjugate, comprising the following steps: mixing the antibody dissolved in a buffer and the linker-cytotoxin dissolved in a solvent in the presence of a reducing agent to obtain the antibody-drug conjugate.

In yet another aspect, the present invention provides a pharmaceutical composition comprising the anti-B7H4 antibody-drug conjugate, the isomer thereof, the pharmaceutically acceptable salt thereof or the mixture thereof as described herein, and a pharmaceutically acceptable carrier or excipient.

In yet another aspect, the present invention provides the use of the anti-B7H4 antibody-drug conjugate, the isomer thereof, the pharmaceutically acceptable salt thereof or the mixture thereof or the pharmaceutical composition as described herein in the preparation of a medicament for the treatment and/or prevention of a disease or condition mediated by B7H4, wherein the disease or condition is preferably cancer positive for B7H4 expression.

In yet another aspect, the present invention provides a method for treating and/or preventing a disease or condition mediated by B7H4, the method comprising: administering the anti-B7H4 antibody-drug conjugate, the isomer thereof, the pharmaceutically acceptable salt thereof or the mixture thereof or the pharmaceutical composition as described herein to a subject in need thereof, wherein the disease or condition is preferably cancer with high expression of B7H4.

In yet another aspect, the present invention provides the anti-B7H4 antibody-drug conjugate, the isomer thereof, the pharmaceutically acceptable salt thereof or the mixture thereof or the pharmaceutical composition as described herein for use in the treatment and/or prevention of a disease or condition mediated by B7H4, wherein the disease or condition is preferably cancer positive for B7H4 expression.

In some embodiments, the cancer of the present invention is selected from breast cancer, ovarian cancer and endometrial tumour.

In yet another aspect, the present invention provides a pharmaceutical combination comprising the antibody-drug conjugate or the pharmaceutically acceptable salt thereof as described herein or the pharmaceutical composition as described herein, and one or more additional therapeutic agents.

In yet another aspect, the present invention provides a kit comprising the antibody-drug conjugate as described herein, or the pharmaceutical composition as described herein.

### Definition of Terms

The practice of the present invention may employ, unless otherwise indicated, conventional techniques of molecular biology (including recombinant techniques), microbiology, cell biology, biochemistry, and immunology, which are within the skill of the art.

In order to understand the present invention more easily, certain technical and scientific terms are specifically defined as follows. Unless otherwise explicitly defined elsewhere herein, all technical and scientific terms used herein have the meanings commonly understood by those of ordinary skill in the art to which the present invention belongs. For definitions and terms in this field, professionals can refer to Current Protocols in Molecular Biology (Ausubel). Abbreviations for amino acid residues are the standard 3-letter and/or 1-letter codes used in the art to refer to one of the 20 commonly used L-amino acids. As used herein (including the claims), singular forms include their corresponding plural forms unless the context explicitly dictates otherwise.

The term "about" generally refers to a change within a range of 0.5%-10% above or below a specified value, for example, a change within a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below a specified value.

The term "antibody" refers to any form of antibody that possesses the desired biological activity. Accordingly, it is used in the broadest sense and specifically includes, but is not limited to, monoclonal antibody (including full length monoclonal antibody), polyclonal antibody, multispecific antibody (such as bispecific antibody), humanized antibody, fully human antibody, chimeric antibody and camelized single domain antibody.

The term "monoclonal antibody" refers to an antibody obtained from a substantially homogeneous population of antibody, i.e., the individual antibodies making up the population are identical except for possible naturally occurring mutations that may be present in minor amounts. Monoclonal antibodies are highly specific and are directed against a single antigen epitope. In contrast, conventional (polyclonal) antibody preparations generally include a large number of antibodies against (or specific for) different epitopes. The modifier "monoclonal" indicates the characteristic of an antibody obtained from a substantially homogeneous population of antibody and should not be construed as requiring production of the antibody by any particular method.

The term "full length antibody" refers to an immunoglobulin molecule comprising four peptide chains as it occurs in nature: two heavy (H) chains (about 50-70 kDa in full length) and two light (L) chains (about 25 kDa in full length) connected to each other by disulphide bonds. Each heavy chain consists of a heavy chain variable region (abbreviated herein as VH) and a heavy chain constant region (abbreviated herein as CH). The heavy chain constant region consists of 3 domains (CH1, CH2 and CH3). Each light chain consists of a light chain variable region (abbreviated herein as VL) and a light chain constant region. The light chain constant region consists of one domain CL. The VH and VL regions can be further subdivided into highly variable complementarity determining regions (CDRs) and more conserved regions referred to as framework regions (FRs) spaced by the CDRs. Each VH or VL region consists of three CDRs and four FRs arranged from amino terminal to carboxyl terminal, in the following order: FR1, CDR1, FR2, CDR2, FR3, CDR3, FR4. The variable regions of the heavy and light chains contain binding domains that interact with antigens. The constant region of the antibody can mediate the binding of the immunoglobulin to a host tissue or factor (comprising various cells (e.g., effector cells) of the immune system and the first component of the classical complement system (Clq)).

The term "CDR" refers to a complementarity determining region within the variable sequence of an antibody. There are three CDRs in each variable region of the heavy and light chains, and the CDRs are designated HCDR1, HCDR2 and HCDR3 or LCDR1, LCDR2 and LCDR3 for each heavy and light chain variable region. The precise amino acid sequence boundaries of the variable region CDRs of the antibody of the present invention can be determined using any of a number of well-known schemes, including Chothia based on the three-dimensional structure of an antibody and the topology of a CDR loop (Chothia et al. (1989) Nature 342:877-883; Al-Lazikani et al., "Standard conformations for the canonical structures of immunoglobulins", Journal of Molecular Biology, 273, 927-948 (1997)), Kabat based on antibody sequence variability (Kabat et al., Sequences of Proteins of Immunological Interest, 4th ed., U.S. Department of Health and Human Services, National Institutes of Health (1987)), AbM (University of Bath), Contact (University College London), International ImMunoGeneTics database (IMGT) (1999 Nucleic Acids Research, 27, 209-212), and the North CDR definition based on affinity propagation clustering using a large number of crystal structures. The boundaries of the CDRs of the antibody of the present invention can be determined by a person skilled in the art according to any scheme in the art (e.g., different assignment systems or combinations).

The term "antigen-binding fragment" of an antibody ("parent antibody") includes a fragment or a derivative of an antibody, generally comprising at least a fragment of the antigen binding region or the variable region (for example, one or more CDRs) of the parent antibody, and the fragment or the derivative of the antibody retains at least some of the binding specificity of the parent antibody. Examples of antigen binding fragment include, but are not limited to, Fab, Fab', F(ab')2 and Fv fragments; diabodies; linear antibodies; single-stranded antibody molecules, such as scFv; and nanobodies and multispecific antibodies formed from antibody fragments. When the binding activity of an antigen is expressed on a molar concentration basis, a binding fragment or a derivative generally retains at least 10% of its antigen binding activity. Preferably the binding fragment or the derivative retains at least 20%, 50%, 70%, 80%, 90%, 95% or 100% or more of the antigen binding affinity of the parent antibody. It is also contemplated that an antigen-binding fragment of an antibody may include conservative or non-conservative amino acid substitutions that do not significantly change its biological activity (referred to as "conservative variants" or "functionally conservative variants" of the antibody).

The term "chimeric antibody" is an antibody having a variable domain of a first antibody and a constant domain of a second antibody, wherein the first antibody and the second antibody are from different species. generally, the variable domain is derived from an antibody of a rodent, *etc.* (a "parent antibody"), while the constant domain sequence is derived from a human antibody such that compared with the parental rodent antibody, the obtained chimeric antibody is less likely to induce an adverse immune response in human subjects.

The term "humanized antibody" refers to a form of an antibody that contains sequences from both human and non-human (for example, mouses and rats) antibody. In general, a humanized antibody comprises at least one, usually two, variable domains, wherein all or substantially all of the hypervariable loops correspond to those of a non-human immunoglobulin, and all or substantially all of the framework (FR) regions correspond to those of a human immunoglobulin sequence. A humanized antibody optionally can comprise at least a portion of a human immunoglobulin constant region (Fc).

In the present application, the term "halogen" generally refers to fluorine, chlorine, bromine, or iodine, for example, the term can refer to fluorine or chlorine.

In the present application, the term "alkyl" generally refers to a residue derived from an alkane by removal of a hydrogen atom. Alkyl may be substituted or unsubstituted, or replaced or unreplaced. The term "alkyl" generally refers to a saturated linear or branched aliphatic hydrocarbon group having a residue derived from the parent alkane by removal of hydrogen atoms from the same carbon atom or two different carbon atoms, and it may be a linear or branched group containing 1 to 20 carbon atoms, e.g., 1 to 12 carbon atoms, such as linear or branched alkyl containing 1 to 6 carbon atoms. Non-limiting examples of alkyl include, but are not limited to, methyl, ethyl, n-propyl, isopropyl, butyl, etc. Alkyl may be substituted or unsubstituted, replaced or unreplaced. For example, when it is substituted, substitution with a substituent may be performed at any available linking site, and the substituent may be independently and optionally selected from one or more substituents of alkyl, alkenyl, alkynyl, alkoxy, alkylthio, alkylamino, halogen, sulphhydryl, hydroxyl, nitro, cyano, cycloalkyl, heterocyclyl, aryl, heteroaryl, cycloalkoxy, heterocycloalkoxy, cycloalkylthio, heterocycloalkylthio, and oxo, and it may, e.g., be hydrogen, protium, deuterium, tritium, halogen, -NO₂, -CN, -OH, - SH, -NH₂, -C(O)H, -CO₂H, -C(O)C(O)H, -C(O)CH₂C(O)H, -S(O)H, -S(O)₂H, - C(O)NH₂, -SO₂NH₂, -OC(O)H, -N(H)SO₂H or C₁₋₆ aliphatic group. For example, when substituted with cycloalkyl, it is cycloalkylalkyl.

In the present application, the term "alkylene" generally refers to a saturated linear or branched aliphatic hydrocarbon group having 2 residues derived from the parent alkane by removal of two hydrogen atoms from the same carbon atom or two different carbon atoms, and it may be a linear or branched group containing 1 to 20 carbon atoms; for example, the term "methylene" may refer to a residue derived from a one-carbon atom group by removal of two hydrogen atoms. Methylene may be substituted or unsubstituted, replaced or unreplaced. For example, alkylene contains 1 to 12 carbon atoms, e.g., an alkylene group containing 1 to 6 carbon atoms. Non-limiting examples of alkylene include, but are not limited to, methylene (-CH₂-), 1,1-ethylene (-CH(CH₃)-), 1,2-ethylene (-CH₂CH₂)-), 1,1-propylene (-CH(CH₂CH₃)-), 1,2-propylene (-CH₂CH(CH₃)-), 1,3-propylene (-CH₂CH₂CH₂-), 1,4-butylene (-CH₂CH₂CH₂CH₂-), 1,5-butylene (-CH₂CH₂CH₂CH₂CH₂-), etc. Alkylene may be substituted or unsubstituted, replaced or unreplaced. For example, when it is substituted, substitution with a substituent may be performed at any available linking point, and the substituent may be independently and optionally selected from one or more substituents of alkyl, alkenyl, alkynyl, alkoxy, alkylthio, alkylamino, halogen, sulphhydryl, hydroxyl, nitro, cyano, cycloalkyl, heterocyclyl, aryl, heteroaryl, cycloalkoxy, heterocycloalkoxy, cycloalkylthio, heterocycloalkylthio, and oxo, and it may, e.g., be hydrogen, protium, deuterium, tritium, halogen, -NO₂, -CN, -OH, -SH, -NH₂, -C(O)H, -CO₂H, -C(O)C(O)H, - C(O)CH₂C(O)H, -S(O)H, -S(O)₂H, -C(O)NH₂, -SO₂NH₂, -OC(O)H, -N(H)SO₂H or C₁₋₆ aliphatic group. Methylene or alkylene may be substituted or unsubstituted.

The term "alkoxy" refers to -O-(alkyl) and -O-(unsubstituted cycloalkyl), wherein the alkyl or cycloalkyl is as defined herein. Non-limiting examples of alkoxy include: methoxy, ethoxy, propoxy, butoxy, cyclopropoxy, cyclobutoxy, cyclopentyloxy, and cyclohexyloxy. Alkoxy may be optionally substituted or unsubstituted, and when it is substituted, the substituent is preferably one or more of the following groups independently selected from: alkyl, alkenyl, alkynyl, alkoxy, alkylthio, alkylamino, halogen, sulphhydryl, hydroxyl, nitro, cyano, cycloalkyl, heterocyclyl, aryl, heteroaryl, cycloalkoxy, heterocycloalkoxy, cycloalkylthio and heterocycloalkylthio.

In the present application, the term "alkenyl" generally refers to a linear or branched hydrocarbon group containing one or more double bonds. Exemplary examples of alkenyl include allyl, homoallyl, vinyl, crotyl, butenyl, pentenyl, hexenyl, etc. Exemplary examples of C2-6 linear or branched alkenyl containing more than one double bond include butadienyl, pentadienyl, hexadienyl, and hexatrienyl, as well as branched forms thereof. The positions of the unsaturated bonds (double bonds) may be any positions in the carbon chain. Alkenyl may be substituted or unsubstituted.

In the present application, the term "alkenylene" generally refers to a residue derived from an alkene by removal of two hydrogen atoms from a carbon atom. For example, alkenylene may be acrol, vinylene, butenylene, pentenylene, hexenylene, etc. Alkenylene may be substituted or unsubstituted.

In the present application, the term "alkynyl" generally refers to unsaturated linear or branched hydrocarbon, e.g., ethynyl, 1-propynyl, propargyl, or butynyl. Alkynyl may be substituted or unsubstituted.

In the present application, the term "alkynylene" generally refers to a residue derived from an alkyne by removal of two hydrogen atoms from a carbon atom. For example, alkynylene may be ethynylene, propynylene, propargylene, butynylene, etc. Alkynylene may be substituted or unsubstituted.

In the present application, the term "aryl" generally refers to a residue derived from an aromatic ring by removal of a hydrogen atom. The term "aromatic ring" may refer to a 6- to 14-membered all-carbon monocyclic ring or fused polycyclic ring (i.e., rings which share adjacent pairs of carbon atoms) having a conjugated π-electron system, and it may be 6- to 10-membered, such as benzene and naphthalene. The aromatic ring can be fused to a heteroaryl, heterocyclyl or cycloalkyl ring, wherein the ring connected to the parent structure is the aryl ring. Aryl may be substituted or unsubstituted, and when it is substituted, the substituent may be one or more of the following groups independently selected from the following group: alkyl, alkenyl, alkynyl, alkoxy, alkylthio, alkylamino, halogen, sulphhydryl, hydroxyl, nitro, cyano, cycloalkyl, heterocycloalkyl, aryl, heteroaryl, cycloalkoxy, heterocycloalkoxy, cycloalkylthio, and heterocycloalkylthio. Aryl may be substituted or unsubstituted.

In the present application, the term "heteroaryl" generally refers to a residue derived from a heteroaromatic ring by removal of a hydrogen atom from a carbon atom. The term "heteroaromatic ring" refers to a heteroaromatic system comprising 1 to 4 heteroatoms and 5 to 14 ring atoms, wherein the heteroatoms may be selected from the following group: oxygen, sulphur and nitrogen. Heteroaryl may be 5- to 10-membered and may be 5- or 6-membered, such as furanyl, thienyl, pyridinyl, pyrrolyl, N-alkylpyrrolyl, pyrimidinyl, pyrazinyl, imidazolyl and tetrazolyl. The heteroaryl ring can be fused to an aryl, heterocyclyl or cycloalkyl ring, wherein the ring connected to the parent structure is the heteroaryl ring. Heteroaryl may be optionally substituted or unsubstituted, and when it is substituted, the substituent may be one or more of the following groups independently selected from the following group: alkyl, alkenyl, alkynyl, alkoxy, alkylthio, alkylamino, halogen, sulphhydryl, hydroxyl, nitro, cyano, cycloalkyl, heterocycloalkyl, aryl, heteroaryl, cycloalkoxy, heterocycloalkoxy, cycloalkylthio, and heterocycloalkylthio. Heteroaryl may be substituted or unsubstituted.

In the present application, the term "cycloalkyl" refers to a saturated or partially unsaturated monocyclic or polycyclic hydrocarbon substituent, and the cycloalkyl ring contains 3 to 20 carbon atoms, preferably 3 to 12 carbon atoms, preferably 3 to 10 carbon atoms, preferably 3 to 8 carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc. Examples of polycyclic cycloalkyl include spiro ring, fused ring and bridged ring cycloalkyl. Cycloalkyl may be substituted or unsubstituted. When it is substituted, substitution with a substituent may be performed at any available linking site, and preferably, the substituent is independently and optionally selected from one or more substituents of a hydrogen atom, halogen, alkyl, alkoxy, haloalkyl, hydroxyl, hydroxyalkyl, cyano, amino, nitro, cycloalkyl, heterocyclyl, aryl and heteroaryl.

In the present application, the term "partially unsaturated" generally means that the cyclic structure contains at least one double or triple bond between the ring molecules. The term "partially unsaturated" encompasses cyclic structures having multiple sites of unsaturation, but is not intended to include aromatic or heteroaromatic rings defined herein. The term "unsaturated" means that the moiety has one or more degrees of unsaturation.

In the present application, the term "heterocyclyl" refers to a saturated or partially unsaturated monocyclic or polycyclic hydrocarbon substituent comprising 3 to 20 ring atoms, wherein one or more of the ring atoms are heteroatoms selected from nitrogen, oxygen, or sulphur, and the remaining ring atoms are carbon. Preferably, heterocyclyl contains 3 to 12 ring atoms, 1 to 4 of which are heteroatoms; more preferably, heterocyclyl contains 3 to 8 ring atoms, 1 to 3 of which are heteroatoms; more preferably, heterocyclyl contains 3 to 6 ring atoms, 1 to 3 of which are heteroatoms; most preferably, heterocyclyl contains 5 or 6 ring atoms, 1 to 3 of which are heteroatoms. Non-limiting examples of monocyclic heterocyclyl include pyrrolidinyl, tetrahydropyranyl, piperidinyl, morpholinyl, thiomorpholinyl, homopiperazinyl, etc. Examples of polycyclic heterocyclyl include spiro, fused and bridged heterocyclyl. The heterocyclyl ring can be fused to an aryl, heteroaryl or cycloalkyl ring, and the ring connected to the parent structure is heterocyclyl. Heterocyclyl may be substituted or unsubstituted. When it is substituted, substitution with a substituent may be performed at any available linking site, and preferably, the substituent is independently and optionally selected from one or more of a hydrogen atom, halogen, alkyl, alkoxy, haloalkyl, hydroxyl, hydroxyalkyl, cyano, amino, nitro, cycloalkyl, heterocyclyl, aryl and heteroaryl.

In the present application, the term "ring-forming atom" generally refers to an atom contained in a cyclic structure. For example, a ring-forming atom may be a carbon atom in a benzene ring, or may be a nitrogen atom in a pyridine ring. When a hydrogen atom is linked to a ring-forming atom, the ring-forming atom may be substituted or unsubstituted.

In the present application, the term "each independently" generally means that a variable applies in any case irrespective of the presence or absence of variables having the same or different definitions in the same compound. For example, the variable may refer to the type or number of substituents in the compound, the type of atoms in the compound, and so on. For example, where R occurs twice in a compound and R is defined as "independently carbon or nitrogen", both R can be carbon, both R can be nitrogen, or one R can be carbon and the other R is nitrogen.

In the present application, the term "optional" or "optionally" generally means that the event or circumstance subsequently described may, but not necessarily, occur, and that the description includes instances where the event or circumstance occurs or does not occur. For example, "heterocyclyl group optionally substituted with alkyl" means that alkyl may be, but not necessarily, present, and that the description may include instances where the heterocyclyl group is or is not substituted with alkyl.

In the present application, the term "substituted" generally means that one or more hydrogen atoms in the group, for example, up to 5 (e.g., 1 to 3) hydrogen atoms, are each independently substituted with a corresponding number of substituents. A substituent is only in its possible chemical position, and those skilled in the art will be able to determine (by experiments or theories) possible or impossible substitution without undue efforts. For example, it may be unstable when amino or hydroxyl having a free hydrogen is bound to a carbon atom having an unsaturated (such as olefinic) bond.

In the present application, the term "0 or more (e.g., 0 or 1 or more, 0 or 1, or 0) methylene units are replaced" generally means that when the structure comprises one or more methylene units, the one or more methylene units may not be replaced, or may be replaced by one or more groups that are not methylene (e.g., -NHC(O)-, -C(O)NH-, -C(O)-, -OC(O)-, -C(O)O-, -NH-, -O-, -S-, -SO-, -SO2-, -PH-, -P(=O)H-, -NHSO2-, -SO2NH-, -C(=S)-, -C(=NH)-, -N=N-, -C=N-, -N=C- or -C(=N2)-).

In the present application, the "linking" of group X to group Y may generally be in any orientation, which generally means that when group X is used for linker Y and group Z, two or more linking sites of the group X may be linked arbitrarily to either group Y or group Z.

In the present application, the term "compound" generally refers to a substance having two or more different elements. For example, the compound of the present application may be an organic compound. For example, the compound of the present application may be a compound having a molecular weight of no more than 500 Da, a compound having a molecular weight of no more than 1000 Da, a compound having a molecular weight of no less than 1000 Da, or a compound having a molecular weight of no less than 10000 Da or no less than 100000 Da. In the present application, the compound may also refer to a compound that involves linking by a chemical bond, for example, a compound where one or more molecules having a molecular weight of no more than 1000 Da are linked, by a chemical bond, to a biological macromolecule, wherein the biological macromolecule may be polysaccharide, protein, nucleic acid, polypeptide, and the like. For example, the compound of the present application may include a compound where a protein is linked to one or more molecules having a molecular weight of no more than 1000 Da, may include a compound where a protein is linked to one or more molecules having a molecular weight of no more than 10000 Da, and may include a compound where a protein is linked to one or more molecules having a molecular weight of no more than 100000 Da.

In the present application, the terms such as "alkyl", "alkenyl" and "cycloalkyl" may be preceded by a notation to indicate the number of atoms present in the groups under particular circumstances as in C₁-C₄ alkyl, C₃-C₇ cycloalkoxy, C₁-C₄ alkylcarbonylamino, and the like, as known to those skilled in the art, and the subscript numeral following "C" indicates the number of carbon atoms present in the group. For example, C₃ alkyl refers to an alkyl group having three carbon atoms (e.g., n-propyl, or isopropyl); in C₁₋₁₀, members of the group may contain any number of carbon atoms within the range of 1-10.

One or more hydrogen atoms in the group, for example, up to 5 (e.g., 1 to 3) hydrogen atoms, are each independently substituted with a corresponding number of substituents. A substituent is only in its possible chemical position, and those skilled in the art will be able to determine (by experiments or theories) possible or impossible substitution without undue efforts. For example, it may be unstable when amino or hydroxyl having a free hydrogen is bound to a carbon atom having an unsaturated (such as olefinic) bond.

In the present application, the compound or ligand-drug conjugate of the present application includes tautomers, mesomers, racemates, enantiomers, and/or diastereoisomers thereof. In the present application, the term "diastereoisomer" generally refers to a stereoisomer that has two or more chiral centres and whose molecules are not mirror images of each other. Diastereoisomers may have different physical properties, e.g., melting points, boiling points, spectral properties, and reactivities. In the present application, the terms "tautomer" or "tautomeric form" are used interchangeably and generally refer to structural isomers of different energies that can be converted into each other by crossing a low energy barrier. For example, proton tautomers (also known as prototropic tautomers) include interconversions by proton migration, such as keto-enol isomerization and imine-enamine isomerization. Valence tautomers include interconversions by recombination of some bonding electrons. In the present application, the term "mesomer" generally means that the molecule contains asymmetric atoms but the total optical rotation is zero due to the presence of symmetric factors. The term "racemate" or "racemic mixture" refers to a composition of two enantiomeric substances in equimolar amounts.

In the present application, the term "isomer" of a compound or a ligand-drug conjugate generally includes tautomers, mesomers, racemates, enantiomers and diastereoisomers of the compound.

In the present application, the term "ligand-drug conjugate" generally means that a ligand is linked to a biologically active cytotoxic drug via a stable linking unit. In the present application, the "ligand-drug conjugate" may be an antibody-drug conjugate (ADC), which may mean that a monoclonal antibody or an antibody fragment is linked to a biologically active cytotoxic drug via a stable linking unit.

In the present application, the term "ligand" generally refers to a macromolecular compound capable of recognizing and binding to an antigen or receptor associated with a target cell. The role of ligands may be to present the drug to a target cell population to which the ligand binds, and the ligands include, but are not limited to, protein hormones, lectin, growth factors, antibodies, or other molecules capable of binding to a cell, a receptor and/or an antigen. In the present application, the ligand may be denoted as Pc, and a linking bond is formed between the ligand antigen and the linking unit through a heteroatom in the ligand. The ligand may be an antibody or an antigen-binding fragment (Ab) thereof, wherein the antibody may be selected from a chimeric antibody, a humanized antibody, a fully human antibody, or a murine antibody, and the antibody may be a monoclonal antibody.

The term "cytotoxic drug" generally refers to a toxic drug, and the cytotoxic drug may be a chemical molecule within the tumour cell that is strong enough to disrupt its normal growth. Cytotoxic drugs can kill tumour cells at a sufficiently high concentration. The "cytotoxic drug" may include toxins, such as small molecule toxins or enzymatically active toxins of bacterial, fungal, plant or animal origin, radioisotopes (e.g., At²¹¹, I¹³¹, I¹²⁵, Y⁹⁰, Re¹⁸⁶, Re¹⁸⁸, Sm¹⁵³, Bi²¹², P³² or radioactive isotopes of Lu), toxic drugs, chemotherapeutic drugs, antibiotics and nucleolytic enzymes; for example, the cytotoxic drug may be toxic drugs, including but not limited to camptothecin derivatives, which, for example, may be the camptothecin derivative exatecan (chemical name: (1S,9S)-1-amino-9-ethyl-5-fluoro-2,3-dihydro-9-hydroxy-4-methyl-1H,12H-benzo[de]pyrano[3',4':6,7]imidazo[1,2-b]quinoline-10,13(9H,15H)-dione). In some embodiments of the present invention, a cytotoxic drug is also referred to as cytotoxin.

The term "linker unit" or "linker structure" generally refers to a chemical structural fragment or bond that is linked to a ligand at one end and to a cytotoxic drug at the other end, or that is linked to other linkers before being linked to the cytotoxic drug. The direct or indirect linking of a ligand may mean that the group is directly linked to the ligand via a covalent bond, and may also be linked to the ligand via a linker structure. For example, a chemical structure fragment or bond comprising an acid-labile linker structure (e.g., hydrazone), a protease-sensitive (e.g., peptidase-sensitive) linker structure, a photolabile linker structure, a dimethyl linker structure or a disulphide-containing linker structure may be used as a linker structure.

The term a structure being "optionally linked to other molecular moieties" generally means that the structure is not linked to any other chemical structure, or that the structure is linked (e.g., via a chemical bond or a linker structure) to one or more other chemical structures (e.g., ligands described in the present application) different from the structure.

The term "drug loading" generally refers to the average amount of cytotoxic drug loaded per ligand and may also be expressed as the ratio of cytotoxic drug amount to antibody amount, and the cytotoxic drug loading may range from 0 to 12 (e.g., 1 to 10) cytotoxic drugs per ligand (Ab). In the embodiments of the present application, the drug loading is denoted as Na, and exemplary values may be an average of 1, 2, 3, 4, 5, 6, 7, 8, 9 and 10. The drug loading per ADC molecule after the coupling reaction can be characterized by conventional methods such as UV/visible spectroscopy, mass spectrometry, ELISA assays and HPLC.

In the present application, certain atoms of the compound of the present application may be present in more than one isotopic form. For example, hydrogen may occur as protium (¹H), deuterium (²H), and tritium (³H), and carbon may naturally occur as three different isotopes (¹²C, ¹³C and ¹⁴C). Examples of isotopes that can be incorporated into compound of the present application also include, but are not limited to, ¹⁵N, ¹⁸O, ¹⁷O, ¹⁸F, ³²P, ³³P, ¹²⁹I, ¹³¹I, ¹²³I, ¹²⁴I, ¹²⁵I, or similar isotopes. Thus, the compounds of the present application may be enriched with one or more of these isotopes relative to the natural abundance of these isotopes. Such isotopically enriched compounds can be used for a variety of purposes, as known to those skilled in the art. For example, substitution with heavy isotopes such as deuterium (²H) may offer certain therapeutic advantages, possibly due to higher metabolic stability. For example, the natural abundance of deuterium (²H) is about 0.015%. Accordingly, one out of about 6500 hydrogen atoms in nature is a deuterium atom. Accordingly, the deuterium abundance of one or more sites (as the case may be) in the deuterium-containing compound of the present application is greater than 0.015%. Unless otherwise indicated, the structures described in the present application may also include compounds that differ only in the presence or absence of one or more isotopically enriched atoms. For example, compounds having a structure identical to the structure of the present application except for the substitution of the hydrogen atom with deuterium or tritium or the substitution of the carbon atom with carbon 13 or carbon 14 are within the scope of the present application.

In the present application, the term "pharmaceutical composition" generally refers to a mixture containing one or more of the compounds described in the present application or a physiologically/pharmaceutically acceptable salt or pro-drug thereof, and other chemical components, for example physiologically/pharmaceutically acceptable carriers and excipients. The pharmaceutical composition may promote the administration to an organism, which facilitates the absorption of the active ingredient, thereby exerting biological activities. For the preparation of conventional pharmaceutical compositions, reference can be made to Chinese Pharmacopoeia. The pharmaceutical composition may be in the form of a sterile injectable aqueous or oily suspension for intramuscular and subcutaneous administration. The suspension can be formulated according to a known technique using suitable dispersing agents or wetting agents and suspending agents. The sterile injectable preparation may also be a sterile injection solution or suspension prepared in a parenterally acceptable non-toxic diluent or solvent, e.g., a solution prepared in 1,3-butanediol. In addition, a sterile fixed oil may be conveniently used as a solvent or a suspending medium. For example, any blend fixed oil including synthetic mono- or di-glycerides can be used. In addition, fatty acids such as oleic acid may also be used in the preparation of injections.

In the present application, the term "pharmaceutically acceptable salt" generally refers to a salt of a compound or ligand-drug conjugate of the present application, or a salt of a compound described in the present application. Such salts may be safe and/or effective when used in mammals and may possess the required biological activity, and the antibody-drug conjugate of the present application may form a salt with an acid, and non-limiting examples of pharmaceutically acceptable salts include: hydrochloride, hydrobromide, hydriodide, sulphate, bisulphate, citrate, acetate, succinate, ascorbate, oxalate, nitrate, sorbate, hydrophosphate, dihydrophosphate, salicylate, hydrocitrate, tartrate, maleate, fumarate, formate, benzoate, mesylate, ethanesulphonate, benzenesulphonate and p-toluenesulphonate.

In the present application, the term "pharmaceutically acceptable carrier" generally refers to a carrier or carrier agent that provides therapeutic agents, such as antibodies or polypeptides, genes, and other therapeutic agents. The term refers to any pharmaceutical carrier that does not itself induce the production of antibodies harmful to the individual that receives the composition and can be given without producing undue toxicity. Suitable carriers may be large, slowly metabolized macromolecules, such as proteins, polysaccharides, polylactic acids, polyglycolic acids, poly(amino acid)s, amino acid copolymers, lipid aggregates, and inactivated virus particles. Such carriers are well known to those skilled in the art. Pharmaceutically acceptable carriers in therapeutic compositions may include liquids such as water, saline, glycerol, and ethanol. Auxiliary substances, such as wetting or emulsifying agents, or pH buffering substances, may also be present in these carriers.

In the present application, the terms "treatment" and "treating" generally refer to a method of achieving beneficial or desired effects, including but not limited to therapeutic benefits. Therapeutic benefits include, but are not limited to, eradication, inhibition, reduction, or amelioration of the underlying disorder being treated. In addition, therapeutic benefits are achieved by eradicating, inhibiting, reducing, or ameliorating one or more physiological symptoms associated with the underlying disorder, and thus improvements are observed in the patient, but the patient may still suffer from the underlying disorder.

In the present application, the terms "prevention" and "preventing" generally refer to a method of achieving beneficial or desired effects, including but not limited to prophylactic benefits. For the purpose of prophylactic benefits, a pharmaceutical composition can be administered to a patient at risk of developing a particular disease, or to a patient who reports to have one or more physiological symptoms of a disease, even if the disease has not yet been diagnosed.

In the present application, the term "subject" or "patient" generally refers to a human (i.e., a male or female in any age group, e.g., a paediatric subject (e.g., an infant, a child, or an adolescent) or an adult subject (e.g., a young person, a middle-aged person, or an elderly person)) and/or other primates (e.g., a cynomolgus monkey, or a rhesus monkey); a mammal, including commercially relevant mammals, such as cows, pigs, horses, sheep, goats, cats, and/or dogs; and/or a poultry, including commercially relevant poultries such as chickens, ducks, geese, quail and/or turkeys.

The terms "therapeutically effective amount", "therapeutically effective dose" and "effective amount" refer to an amount of the ligand-drug conjugate of the present invention that is effective in preventing or improving one or more symptoms of a disease or condition or the development of the disease or condition, when administered alone or in combination with other therapeutic drugs to a cell, tissue or subject. The therapeutically effective dose also refers to a dose sufficient to cause an improvement in symptoms, e.g., an amount for treating, curing, preventing or improving a related medical condition or promoting the treatment, cure, prevention or improvement of such a condition. When an active ingredient is administered to an individual alone, a therapeutically effective dose only refers to the amount of the ingredient. In the case of administration in combination, a therapeutically effective dose refers to the combined amount of active ingredients that produces a therapeutic effect, regardless of whether these active ingredients are administered in combination, sequentially or simultaneously. An effective amount of a therapeutic agent will result in an increase in a diagnostic index or parameter by at least 10%, generally at least 20%, preferably at least about 30%, more preferably at least 40%, and most preferably at least 50%.

The term "cancer" is used herein to refer to a group of cells that exhibit abnormally high levels of proliferation and growth. Cancer may be benign (also referred to as benign tumour), premalignant or malignant. Cancer cells may be solid cancer cells or haematological cancer cells. The term "tumour" as used herein refers to one or more cells comprising cancer. The term "tumour growth" is used herein to refer to the proliferation or growth of one or more cells comprising cancer, which results in a corresponding increase in the size or extent of the cancer.

### Anti-B7H4 antibody

The term "B7-H4" or "B7H4" refers to a member of the human B7 protein family, also known as CD276, which is a type I transmembrane protein having four Ig-like extracellular domains. B7-H4 is one of the immune checkpoint proteins expressed on the surface of antigen-presenting cells or cancer cells and has an inhibitory effect on the functional activation of T cells. The term "B7-H4" includes any variant or isotype of B7-H4 naturally expressed by cells. The antibodies of the present invention can cross-react with B7-H4 obtained from non-human species. Alternatively, the antibodies may also be specific for human B7-H4, and may not exhibit cross-reactivity with other species. B7-H4 or any variant or isotype thereof can be isolated from cells or tissues in which they are naturally expressed, or produced by recombinant techniques using techniques commonly used in the art and those described herein. Preferably, the anti-B7-H4 antibodies target human B7-H4 with normal glycosylation pattern.

In the present invention, the term "anti-B7H4 antibody", "anti-B7H4", "B7H4 antibody" or "antibody that binds to B7H4" means that it can bind to B7H4 protein or fragment thereof with sufficient affinity so that the antibody can be used as a diagnostic and/or therapeutic agent targeting B7H4.

In some embodiments, the CDR sequences of the antibodies for use in the drug conjugate, composition, use or method of the present invention include the CDR sequences from the antibody PR008199 described in PCT/CN2021/102952. In some embodiments, the variable region sequences of the antibodies for use in the drug conjugate, composition, or use of the present invention include the variable region sequences from the antibody PR008199 described in PCT/CN2021/102952. In some embodiments, the amino acid sequences of the antibodies for use in the drug conjugate, composition, use or method of the present invention include the full-length amino acid sequences from the antibody PR008199 described in PCT/CN2021/102952.

The anti-B7H4 antibody DB1001 (PR008199) or the antigen-binding fragment thereof of the present invention is prepared with reference to the description in PCT/CN2021/102952. In some embodiments, the CDR sequences of the antibodies for use in the drug conjugate, composition, use or method of the present application comprise HCDR1, HCDR2 and HCDR3 having the amino acid sequences as shown in SEQ ID NO:1, SEQ ID NO:2 and SEQ ID NO:3, respectively, and LCDR1, LCDR2 and LCDR3 having the amino acid sequences as shown in SEQ ID NO:4, SEQ ID NO:5 and SEQ ID NO:6, respectively. In some embodiments, the variable region sequences of the antibodies for use in the drug conjugate, composition or use of the present invention comprise a heavy chain variable region with an amino acid sequence as shown in SEQ ID NO:7, and a light chain variable region with an amino acid sequence as shown in SEQ ID NO:8. In some embodiments, the antibody for use in the drug conjugate, composition, use or method of the present invention comprises a heavy chain with an amino acid sequence as shown in SEQ ID NO: 9, and a light chain with an amino acid sequence as shown in SEQ ID NO: 10. In some embodiments, the antibody for use in the drug conjugate, composition, use or method of the present invention is the anti-B7H4 antibody DB1001.

The variable region amino acid sequences of the anti-B7H4 antibody DB1001 are as follows, and the CDR regions are determined in accordance with the IMGT numbering rules.
DB1001 heavy chain variable region: 
QVQLVESGGGVVQPGRSLRLSCAASGFTFRSFGMHWVRQAPGKGLE
WVAVISYDASNEYYADSVKGRFIISRDNSKDTLYLQMNSLRAGDTAVYYC
AKGGALRWYFAYWGQGTLVTVSS (SEQ ID NO:7)
   HCDR1: **SFGMH** (SEQ ID NO:1)
   HCDR2: **VISYDASNEYYADSVKG** (SEQ ID NO:2)
   HCDR3: **GGALRWYFAY** (SEQ ID NO:3)
DB1001 light chain variable region: 
EIVMTQSPATLSVSPGERATLSCRASQSISSNLGWYQQKPGQAPRLLIY
GASTRATGIPARFSGSGSGTEFTLTISSLQSEDFAVYYCQQYRSWPPLTFGG
GTKVEIK (SEQ ID NO:8)
   LCDR1: **RASOSISSNLG** (SEQ ID NO:4)
   LCDR2: **GASTRAT** (SEQ ID NO:5)
   LCDR3: **QQYRSWPPLT** (SEQ ID NO:6)
DB1001 heavy chain amino acid sequence 
QVQLVESGGGVVQPGRSLRLSCAASGFTFRSFGMHWVRQAPGKGLE
WVAVISYDASNEYYADSVKGRFIISRDNSKDTLYLQMNSLRAGDTAVYYC
AKGGALRWYFAYWGQGTLVTVSSASTKGPSVFPLAPSSKSTSGGTAALGC
LVKDYFPEPVTVSWNSGALTSGVHTFPAVLQSSGLYSLSSVVTVPSSSLGT
QTYICNVNHKPSNTKVDKKVEPKSCDKTHTCPPCPAPELLGGPSVFLFPPK
PKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPRE
PQVYTLPPSREEMTKNQVSLTCLVKGFYPSDIAVEWESNGQPENNYKTTPP
VLDSDGSFFLYSKLTVDKSRWQQGNVFSCSVMHEALHNHYTQKSLSLSPG
K (SEQ ID NO:9)
DB1001 light chain amino acid sequence

EIVMTQSPATLSVSPGERATLSCRASQSISSNLGWYQQKPGQAPRLLIY
GASTRATGIPA
RFSGSGSGTEFTLTISSLQSEDFAVYYCQQYRSWPPLTFGGGTKVEIKRTVA
APSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGE
C (SEQ ID NO:10)

The antibody of the present invention can be produced using any suitable method for producing antibodies. Any suitable form of B7H4 can be used as an immunogen (antigen) for antibody production. By way of example and not limitation, any B7H4 variant or fragment thereof can be used as an immunogen. In some embodiments, hybridoma cells producing murine monoclonal anti-human B7H4 antibody can be produced by methods well-known in the art. The antibody derived from rodents (for example, mice) may cause unwanted antibody immunogenicity when used *in vivo* as a therapeutic drug, and repeated use leads to the immune response of the human body against therapeutic antibodies, and such immune response at least leads to the loss of therapeutic efficacy, and in severe cases leads to potentially fatal allergic reactions. An approach to reduce the immunogenicity of rodent antibodies includes the generation of chimeric antibodies, in which mouse variable regions are fused to human constant regions (Liu et al. (1987) Proc. Natl. Acad. Sci. USA 84:3439-43). However, retention of intact rodent variable regions in the chimeric antibodies may still cause deleterious immunogenicity in patients. Grafting of complementarity determining region (CDR) loops of rodent variable domains onto human frameworks (i.e. humanization) has been used to further minimize rodent sequences (Jones et al. (1986) Nature 321:522; Verhoeyen et al. (1988) Science 239:1534).

In some embodiments, the chimeric or humanized antibody of the present invention can be prepared based on the sequence of the prepared murine monoclonal hybridoma antibody. DNA coding heavy and light chain immunoglobulins can be obtained from murine hybridomas of interest and engineered to contain non-murine (for example, human) immunoglobulin sequences using standard molecular biology techniques.

In some embodiments, the chimeric B7H4 antibody of the present invention can be prepared by effectively linking the variable regions of immunoglobulin heavy chain and light chain from hybridoma with the constant region of human IgG (see, for example, U.S. Pat. No. 4,816,567 belonging to Cabilly et al.) by methods known in the art to obtain a chimeric heavy chain and a chimeric light chain. In some embodiments, the constant region comprised in the chimeric antibody of the present invention can be selected from any human IgG subtype, such as IgG1, IgG2, IgG3, IgG4, preferably IgG4.

In some embodiments, the chimeric B7H4 antibody of the present invention can be obtained by transfecting expression cells with an expression plasmid of the chimeric light chain and the chimeric heavy chain in a manner of "mix and match", and the above binding assay and other conventional binding assays (for example, ELISA) can be used to determine the B7H4 binding of such "mixed and matched" antibody.

For the humanized antibody of the present invention, the murine CDR regions can be inserted into the human germline framework regions using methods known in the art. See U.S. Patent No. 5,225,539 to Winter et al. and U.S. Patent No. 5,530,101; 5,585,089; 5,693,762 and 6,180,370 to Queen et al.

In some embodiments, amino acid changes include amino acid deletions, insertions or substitutions. In some embodiments, the anti-B7H4 antibodies or the antigen-binding fragments thereof of the present invention include those antibodies that have amino acid sequences have been mutated by amino acid deletion, insertion or substitution, but still have at least about 90%, 91%, 92%, 93%, 94%, 95%, 96%, 97%, 98%, 99% or 100% identity to the above antibodies (especially in the CDR regions depicted in the above sequences). In some embodiments, the antibody of the present invention has no more than 1, 2, 3, 4 or 5 amino acid mutations in the CDR region by amino acid deletion, insertion or substitution when compared to the CDR region depicted in the specific sequence.

In some embodiments, one or more amino acid modifications can be introduced into the Fc region of the antibody provided herein to generate a Fc region variant. The Fc region variant may comprise human Fc region sequences (for example, human IgG1, IgG2, IgG3 or IgG4 Fc regions) comprising amino acid modifications (for example, substitutions) at one or more amino acid positions.

In some embodiments, it may be desirable to generate a cysteine-engineered antibody, such as "thioMAb," in which one or more residues of the antibody are substituted with cysteine residues.

In some embodiments, the antibody provided herein can be further modified to contain other non-protein moieties known and readily available in the art. Moieties suitable for antibody derivatization include, but are not limited to, water soluble polymers. Non-limiting examples of water soluble polymers include, but are not limited to, polyethylene glycol (PEG), ethylene glycol/propylene glycol copolymer, carboxymethylcellulose, dextran, polyvinyl alcohol, polyvinylpyrrolidone, poly-1,3-dialkyl, poly-1,3,6-trialkyl, ethylene/maleic anhydride copolymer, polyamino acid (homopolymer or random copolymer), and dextran or poly(n-vinyl pyrrolidone) polyethylene glycol, propylene glycol homopolymer, polypropylene oxide/ethylene oxide copolymer, polyoxyethylated polyols (such as glycerol), polyvinyl alcohol, and mixtures thereof.

### Drug conjugate

The present application provides an anti-B7H4 antibody-drug conjugate, an isomer thereof, a pharmaceutically acceptable salt thereof or a mixture thereof, which can have one or more effects selected from the following group: (1) having inhibitory activity against tumour cell proliferation *in vitro*; (2) having a targeting inhibition property; (3) having plasma stability; (4) having a tumour inhibition effect *in vivo*; (5) having a bystander killing effect; (6) having a capacity in inhibiting transport via a transporter; (7) having an *in vivo* tumour targeting capability; and (8) having better safety *in vivo.*

The present application provides an anti-B7H4 antibody-drug conjugate, an isomer thereof, a pharmaceutically acceptable salt thereof or a mixture thereof, wherein the structure of the anti-B7H4 antibody-drug conjugate is shown as formula (I-1): 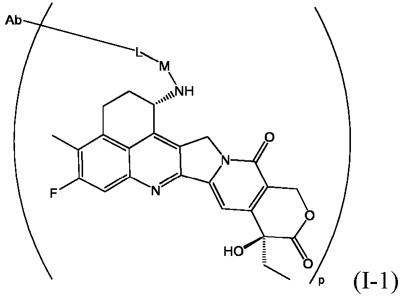 wherein,
M is -L²-L¹-C(O)-;
L² is -O- or -S-,
L¹ is -(C(R^{1a})(R^{1b}))ₘ-CH₂-, saturated C₃-C₆ cycloalkyl or 3- to 6-membered saturated heterocyclyl, wherein the saturated C₃-C₆ cycloalkyl and 3- to 6-membered saturated heterocyclyl are each independently and optionally substituted with one or more R^{2a};
m is selected from 1, 2, 3 or 4;
each R^{1a} is independently selected from hydrogen, halogen, hydroxyl, amino and C₁-C₆ alkyl, wherein the C₁-C₆ alkyl is optionally substituted with one or more R; preferably, R^{1a} is halogen, or C₁-C₆ alkyl optionally substituted with 1, 2 or 3 R;
R^{1b} and R^{2a} are each independently selected from hydrogen, halogen, hydroxyl, amino and C₁-C₆ alkyl, wherein the C₁-C₆ alkyl is optionally substituted with one or more R;
each R is independently hydrogen or halogen;
L is a linker unit;
p represents the average connection number, and p is an integer or decimal selected from 1 to 10, preferably an integer or decimal from 3 to 8;
Ab is an anti-B7H4 antibody or an antigen-binding fragment thereof.

In some embodiments, the anti-B7H4 antibody or the antigen-binding fragment thereof comprises a heavy chain variable region and a light chain variable region, wherein the heavy chain variable region comprises HCDR1, HCDR2 and HCDR3 having the amino acid sequences as shown in SEQ ID NO: 1, SEQ ID NO:2 and SEQ ID NO:3, respectively, and the light chain variable region comprises LCDR1, LCDR2 and LCDR3 having the amino acid sequences as shown in SEQ ID NO:4, SEQ ID NO:5 and SEQ ID NO:6, respectively; preferably, the anti-B7H4 antibody or the antigen-binding fragment thereof of the present invention comprises: a heavy chain variable region with an amino acid sequence as shown in SEQ ID NO:7, and a light chain variable region with an amino acid sequence as shown in SEQ ID NO:8; more preferably, the anti-B7H4 antibody or the antigen-binding fragment thereof of the present invention comprises: a heavy chain with an amino acid sequence as shown in SEQ ID NO: 9, and a light chain with an amino acid sequence as shown in SEQ ID NO: 10.

In some embodiments, in formulas (I-1), (II-1) and (II-2), L¹ is -(C(R^{1a})(R^{1b}))ₘ-CH₂-, R^{1a} is selected from: halogen, hydroxyl, and amino, and C₁-C₆ alkyl optionally substituted with R, R^{1b} is selected from: hydrogen, halogen, hydroxyl, and amino, and C₁-C₆ alkyl optionally substituted with R, m is selected from 1, 2, 3 or 4, and each R can independently be hydrogen or halogen; preferably, R^{1a} is selected from: halogen and C₁-C₆ alkyl, R^{1b} is selected from: hydrogen, halogen and C₁-C₆ alkyl, and m is selected from 1 or 2; preferably, R^{1a} is -CH₃; R^{1b} is selected from: hydrogen and -CH₃, and m is selected from 1 or 2; and preferably, L¹ is selected from

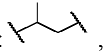

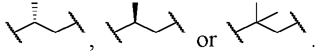

In some embodiments, in formulas (I-1), (II-1) and (II-2), L¹ is saturated C₃-C₆ cycloalkyl or 3- to 6-membered saturated heterocyclyl, wherein the saturated C₃-C₆ cycloalkyl and 3- to 6-membered saturated heterocyclyl are each independently and optionally substituted with one or more R^{2a}, and each R^{2a} is independently selected from: hydrogen, halogen, hydroxyl, amino and C₁-C₆ alkyl; preferably, L¹ is saturated C₃-C₆ cycloalkyl optionally substituted with one or more R^{2a}, and each R^{2a} is independently selected from: hydrogen, halogen and C₁-C₆ alkyl; preferably, L¹ is saturated C₃-C₆ cycloalkyl; and preferably, L¹ is selected from

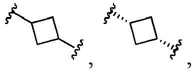

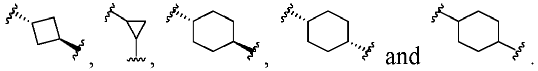

In some embodiments, in formulas (I-1), (II-1) and (II-2), L¹ is selected from

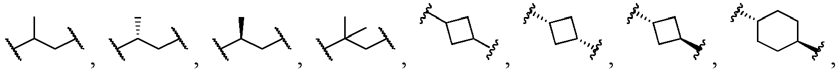

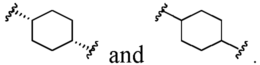

In some embodiments, in formulas (I-1), (II-1) and (II-2), M is -L²-L¹-C(O)-;
L² is -O- or -S-, preferably -O-;
L¹ is -(C(R^{1a})(R^{1b}))ₘ-CH₂- or saturated C₃-C₆ cycloalkyl, wherein the saturated C₃-C₆ cycloalkyl is optionally substituted with one or more R^{2a}; preferably, L¹ is 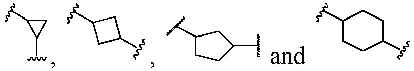 optionally substituted with 1, 2 or 3 R^{2a};
m is selected from 1 or 2;
each R^{1a} is independently selected from halogen and C₁-C₆ alkyl, wherein the C₁-C₆ alkyl is optionally substituted with one or more R;
R^{1b} and R^{2a} are each independently selected from hydrogen, halogen and C₁-C₆ alkyl, wherein the C₁-C₆ alkyl is optionally substituted with one or more R; each R is independently hydrogen or halogen.

In some preferred embodiments, in formulas (I-1), (II-1) and (II-2), M is selected from:

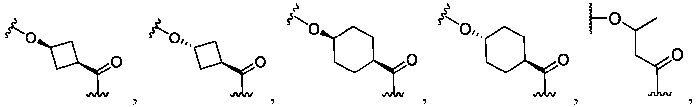

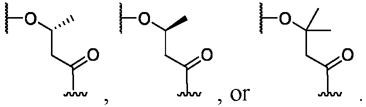

In some most preferred embodiments, the present invention provides the anti-B7H4 antibody-drug conjugate, the isomer thereof, the pharmaceutically acceptable salt thereof or the mixture thereof as described herein, wherein the L is

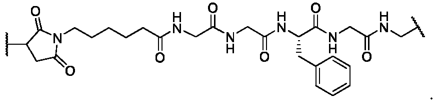

In some embodiments, the present invention provides the anti-B7H4 antibody-drug conjugate, the isomer thereof, the pharmaceutically acceptable salt thereof or the mixture thereof as described herein, wherein the structure of the anti-B7H4 antibody-drug conjugate is shown as formula (II-1) or (II-2): 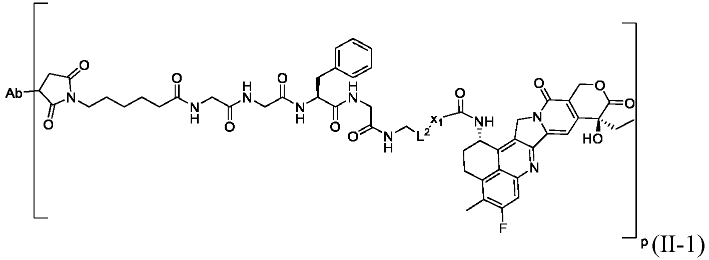 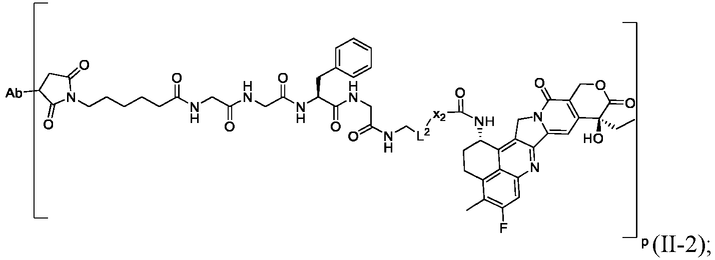 wherein,
L² is -O- or -S-; preferably, L² is -O-;
X₁ is selected from saturated C₃-C₆ cycloalkyl optionally substituted with 1, 2 or 3 R^{2a}; preferably, X₁ is 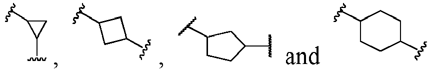 optionally substituted with 1, 2 or 3 R^{2a};
X₂ is selected from -(C(R^{1a})(R^{1b}))ₘ-CH₂-;
m is selected from 1 or 2;
R^{1a} is halogen, or C₁-C₆ alkyl optionally substituted with 1, 2 or 3 R;
R^{1b} and R^{2a} can each independently be hydrogen or halogen, or C₁-C₆ alkyl optionally substituted with 1, 2 or 3 R;
each R can independently be hydrogen or halogen;
p represents the average connection number, and n is an integer or decimal selected from 1 to 10, preferably an integer or decimal from 3 to 8;
Ab is an anti-B7H4 antibody or an antigen-binding fragment thereof comprising a heavy chain variable region and a light chain variable region, wherein the heavy chain variable region comprises HCDR1, HCDR2 and HCDR3 having the amino acid sequences as shown in SEQ ID NO: 1, SEQ ID NO:2 and SEQ ID NO:3, respectively, and the light chain variable region comprises LCDR1, LCDR2 and LCDR3 having the amino acid sequences as shown in SEQ ID NO:4, SEQ ID NO:5 and SEQ ID NO:6, respectively; preferably, the anti-B7H4 antibody or the antigen-binding fragment thereof of the present invention comprises: a heavy chain variable region with an amino acid sequence as shown in SEQ ID NO:7, and a light chain variable region with an amino acid sequence as shown in SEQ ID NO:8; more preferably, the anti-B7H4 antibody or the antigen-binding fragment thereof of the present invention comprises: a heavy chain with an amino acid sequence as shown in SEQ ID NO: 9, and a light chain with an amino acid sequence as shown in SEQ ID NO: 10.

In some embodiments, the average connection number p of the present invention can be an integer or decimal from 2 to 8. For example, the average connection number p can be an integer or decimal from 3 to 8. For example, the average connection number p can be an integer or decimal from 1 to 2, from 2 to 3, from 3 to 4, from 4 to 5, from 5 to 6, from 6 to 7, from 7 to 8, from 8 to 9, or from 9 to 10.

### Pharmaceutical composition and pharmaceutical preparation

In yet another aspect, the present invention provides a pharmaceutical composition comprising the anti-B7H4 antibody-drug conjugate, the isomer thereof, the pharmaceutically acceptable salt thereof or the mixture thereof as described herein, and a pharmaceutically acceptable carrier or excipient.

It should be understood that the anti-B7H4 antibody-drug conjugate, or the isomer thereof, the pharmaceutically acceptable salt thereof or the mixture thereof, or the pharmaceutical composition thereof provided in the present invention can be administered in combination with suitable carriers, excipients and other agents in the preparation, so as to provide improved transfer, delivery, tolerance and the like.

The term "pharmaceutical composition" refers to a preparation that allows the active ingredients contained therein to be present in a biologically effective form and does not contain additional ingredients that would be unacceptably toxic to the subject to which the preparation is administered.

The anti-B7H4 antibody-drug conjugate or the pharmaceutically acceptable salt thereof of the present invention having the desired purity can be mixed with one or more optional pharmaceutical adjuvant materials (Remington's Pharmaceutical Sciences, 16th edition, Osol, A. ed. (1980)) to prepare the pharmaceutical preparation comprising the anti-B7H4 antibody described herein, preferably in the form of an aqueous solution or a lyophilized preparation.

The pharmaceutical composition or preparation of the present invention may also comprise one or more other active ingredients as required for the particular indication being treated, preferably those active ingredients with complementary activities that do not adversely affect each other. In some embodiments, other active ingredients are chemotherapeutic agents, immune checkpoint inhibitors, growth inhibitors, antibiotics, or various known anti-tumour or anti-cancer agents, and the active ingredients are present in a suitable combination in an amount effective for the intended use. In some embodiments, the pharmaceutical composition of the present invention further comprises a composition of a polynucleotide coding the anti-B7H4 antibody.

In yet another aspect, the present invention provides a pharmaceutical combination comprising the antibody-drug conjugate or the pharmaceutically acceptable salt thereof as described herein or the pharmaceutical composition as described herein, and one or more additional therapeutic agents.

In yet another aspect, the present invention provides a kit comprising the antibody-drug conjugate, or the isomer thereof, the pharmaceutically acceptable salt thereof or the mixture thereof as described herein or the pharmaceutical composition as described herein, preferably further comprising a drug delivery device.

### Medical use

In yet another aspect, the present invention provides the use of the antibody-drug conjugate, the isomer thereof, the pharmaceutically acceptable salt thereof or the mixture thereof as described herein or the pharmaceutical composition as described herein in the preparation of a medicament for the treatment and/or prevention of a disease or condition mediated by B7H4, wherein the disease or condition is preferably cancer positive for B7H4 expression.

In yet another aspect, the present invention provides the antibody-drug conjugate, the isomer thereof, the pharmaceutically acceptable salt thereof or the mixture thereof as described herein or the pharmaceutical composition as described herein for use in the treatment and/or prevention of a disease or condition mediated by B7H4, wherein the disease or condition is preferably cancer positive for B7H4 expression.

In yet another aspect, the present invention provides a method for treating and/or preventing a disease or condition mediated by B7H4, the method comprising: administering the antibody-drug conjugate, the isomer thereof, the pharmaceutically acceptable salt thereof or the mixture thereof as described herein or the pharmaceutical composition as described herein to a subject in need thereof, wherein the disease or condition is preferably cancer with high expression of B7H4.

In some embodiments, the cancer is selected from breast cancer, ovarian cancer and endometrial tumour.

In some embodiments, modes of administration of the present invention include, but are not limited to, oral, intravenous, subcutaneous, intramuscular, intraarterial, intraarticular (for example, in arthritic joints), via inhalation, aerosol delivery, or intratumoural administration and the like.

In some embodiments, the present invention provides administration in combination with a therapeutically effective amount of one or more therapies (for example, treatment modalities and/or other therapeutic agents) to a subject. In some embodiments, the therapies include surgery and/or radiation therapy.

In some embodiments, the method or use provided in the present invention further comprises administering one or more therapies (for example, treatment modalities and/or other therapeutic agents) to an individual. The antibody-drug conjugate or pharmaceutically acceptable salt thereof of the present invention can be used alone or in combination with other therapeutic agents in the therapy. For example, it can be co-administered with at least one additional therapeutic agent.

The positive effects of the present invention are as follows:
In the anti-B7H4 antibody-drug conjugate of the present invention, the anti-B7H4 antibody or the antigen-binding fragment thereof is linked to a biologically active cytotoxic drug via a linker unit; when the antibody-drug conjugate is transferred to the tumour cells, the linker is cleaved to release the cytotoxic drug H-M-D. For example, when the antibody-drug conjugate DB1001-X1 of the present invention is transferred to the cells, small molecule compound P-II-3 is released; and when the antibody-drug conjugate DB1001-X2 of the present invention is transferred to the cells, small molecule compound P-III-30 is released. The cytotoxic drug of the present invention has significantly enhanced inhibitory activities against the proliferation of NCI-N87 cells, JIMT-1 cells, Colo205 cells and MDA-MB-231 cells and can exhibit excellent anti-tumour effects.

The MDA-MB-468 cells positive for B7H4 expression exhibit relatively good endocytosis for the anti-B7H4 antibody-drug conjugate of the present invention, which is superior to that for the reference ADC-1.

The antibody-drug conjugate of the present application has significant inhibitory activities on the proliferation of MDA-MB-468 cells, colorectal cancer cell HT29, human breast cancer cell MX-1, and endometrial carcinoma cell RL95-2 positive for B7H4 expression.

The anti-B7H4 antibody-drug conjugate of the present invention can block the inhibition of B7H4-positive cells on T cells derived from different donors, and is superior to the reference ADC-1.

The anti-B7H4 antibody-drug conjugate of the present invention has good stability in human, rat and monkey plasma *in vitro.* In addition, due to the low plasma concentration and short half-life of the cytotoxic drug P-III-30, P-III-30 has a low systemic exposure, thereby providing a relatively high level of safety.

The anti-B7H4 antibody-drug conjugate of the present invention exhibits significant anti-tumour activities in MDA-MB-468 tumour-bearing mice and mice bearing other tumours (breast cancer cell MX-1, breast cancer cell MCF-7, endometrial carcinoma RL95-2, and ovarian cancer cell OVCAR-3, respectively).

Therefore, the present invention has good application prospects in diseases positive for B7H4 expression (such as cancer).

### Brief Description of the Drawings

FIG. 1: pharmacodynamic evaluation of the antibody-drug conjugates in MDA-MB-468 tumour-bearing mice.
FIG. 2: species cross-reactivity of the antibody-drug conjugate in human, cynomolgus monkey, and mice B7H4-transfected HEK293T cells.
FIG. 3: binding specificity of the antibody-drug conjugate in cells over-expressing human B7 family proteins.
FIG. 4: integrated area of fluorescence signal generated at different time points during the endocytosis of the antibody-drug conjugate by MDA-MB-468 cells.
FIG. 5: endocytosis efficiency of MDA-MB-468 cells for the antibody-drug conjugate.
FIG. 6: inhibition rate of the antibody-drug conjugate on the *in vitro* proliferation of HT29-B7H4.
FIG. 7: efficiency of the antibody-drug conjugate, monoclonal antibody, and payload (cytotoxic drug) in inhibiting tumour cell MX-1 proliferation *in vitro* (3D cell culture).
FIG. 8: efficiency of the antibody-drug conjugate, monoclonal antibody, and payload (cytotoxic drug) in inhibiting tumour cell RL95-2 proliferation *in vitro* (3D cell culture).
FIG. 9: efficiency of the antibody-drug conjugate, monoclonal antibody, and payload (cytotoxic drug) in inhibiting tumour cell JIMT-1 proliferation *in vitro* (3D cell culture).
FIG. 10: PBMC-derived T cell activation by the antibody-drug conjugate.
FIG. 11: PBMC-derived T cell activation by the antibody-drug conjugate.
FIG. 12: bystander killing effect of the antibody-drug conjugate on HT29-Luc cells co-incubated with HT29-B7H4.
FIG. 13: payload (cytotoxic drug) release rate of the antibody-drug conjugate after 21 days of *in vitro* incubation in human, monkey and rat plasma.
FIG. 14: pharmacodynamic evaluation of the antibody-drug conjugate in tumour-bearing mice (breast cancer MX-1).
FIG. 15: pharmacodynamic evaluation of the antibody-drug conjugate in tumour-bearing mice (endometrial carcinoma RL95-2).
FIG. 16: pharmacodynamic evaluation of the antibody-drug conjugate in tumour-bearing mice (ovarian cancer OVCAR-3).

### Detailed Description of Embodiments

### Sample detection

### 1. ADC DAR analysis - HIC-HPLC (hydrophobic interaction chromatography)

High performance liquid chromatograph: Waters e2965 high performance liquid chromatography system.
Chromatographic column: MabPac^{™} HIC-Butyl 5 µm 4.6 × 100 mm (manufacturer: Thermo);
mobile phase A: 1.5 M (NH4)₂SO₄ + 50 mM K₂HPO₄ (pH 7.0);
Mobile phase B: 50 mM K₂HPO₄ (pH 7.0)/isopropanol (75 : 25 V/V);
elution is carried out according to the following elution procedure,

| Time | Mobile phase B |
|---|---|
| 0-2 min | 0%-10% |
| 2-22 min | 10%-65% |
| 22-24 min | 65%-100% |
| 24-26 min | 100%-0% |
| 26-30 min | 0% |

Detection conditions: the flow rate of the mobile phase is set to be 1 ml/min, the detection wavelength is 280 nm, and the column temperature is 30°C.

### 2. SEC purity analysis - SEC-HPLC (size exclusion chromatography)

High performance liquid chromatograph: 1260 Agilent liquid chromotograph.
Chromatographic column: Waters Xbridge BEH200 SEC (7.8 × 300 mm, 3.5 µm)
Mobile phase: 50 mM NaH₂PO₄ + 200 mM arginine (pH 6.80) +10% isopropanol

| Time | Mobile phase |
|---|---|
| 0-30 min | 100% |

Detection conditions: the flow rate of the mobile phase is set to be 0.5 ml/min, the detection wavelength is 280 nm, and the column temperature is 30°C.

The present invention includes all combinations of the particular embodiments described. Further embodiments and the full scope of applicability of the present invention will become apparent from the detailed description provided hereinafter. However, it should be understood that the detailed description and specific examples, while indicating preferred embodiments of the present invention, are given by way of illustration only, since various changes and modifications within the spirit and scope of the present invention will become apparent to those skilled in the art from this detailed description. All publications, patents, and patent applications cited herein, including quotations, are incorporated herein by reference in their entirety for all purposes.

### EXAMPLES

The following examples are provided to demonstrate and further explain some preferred embodiments and aspects of the present invention and should not be construed as limiting the scope thereof.

### Example 1. Anti-B7H4 antibody

The antibody of the present invention is prepared with reference to PCT/CN2021/102952, wherein the variable region amino acid sequences of the anti-B7H4 antibody DB1001 (PR008199) are as follows, and the CDR regions are determined in accordance with the Kabat numbering rules.
DB1001 heavy chain variable region: 
QVQLVESGGGVVQPGRSLRLSCAASGFTFRSFGMHWVRQAPGKGLE
WVAVISYDASNEYYADSVKGRFIISRDNSKDTLYLQMNSLRAGDTAVYYC
AKGGALRWYFAYWGQGTLVTVSS (SEQ ID NO:7)
   HCDR1: **SFGMH** (SEQ ID NO:1)
   HCDR2: **VISYDASNEYYADSVKG** (SEQ ID NO:2)
   HCDR3: **GGALRWYFAY** (SEQ ID NO:3)
DB1001 light chain variable region: 
EIVMTQSPATLSVSPGERATLSCRASQSISSNLGWYQQKPGQAPRLLIY
GASTRATGIPARFSGSGSGTEFTLTISSLQSEDFAVYYCQQYRSWPPLTFGG
GTKVEIK (SEQ ID NO:8)
   LCDR1: **RASOSISSNLG** (SEQ ID NO:4)
   LCDR2: **GASTRAT** (SEQ ID NO:5)
   LCDR3: **QQYRSWPPLT** (SEQ ID NO:6)

After primer design based on the above-mentioned sequences, the VH/VK gene fragments are constructed by PCR to obtain the variable region. The antibody variable region is then homologously recombined with the constant region gene fragment to construct the complete antibody sequence of DB 1001. After transfection of CHO cells, antibody DB1001 is obtained through conventional expression and purification methods.
DB1001 heavy chain amino acid sequence 
QVQLVESGGGVVQPGRSLRLSCAASGFTFRSFGMHWVRQAPGKGLE
WVAVISYDASNEYYADSVKGRFIISRDNSKDTLYLQMNSLRAGDTAVYYC
AKGGALRWYFAYWGQGTLVTVSSASTKGPSVFPLAPSSKSTSGGTAALGC
LVKDYFPEPVTVSWNSGALTSGVHTFPAVLQSSGLYSLSSVVTVPSSSLGT
 
QTYICNVNHKPSNTKVDKKVEPKSCDKTHTCPPCPAPELLGGPSVFLFPPK
PKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPRE
PQVYTLPPSREEMTKNQVSLTCLVKGFYPSDIAVEWESNGQPENNYKTTPP
VLDSDGSFFLYSKLTVDKSRWQQGNVFSCSVMHEALHNHYTQKSLSLSPG
K (SEQ ID NO:9)
DB1001 light chain amino acid sequence

EIVMTQSPATLSVSPGERATLSCRASQSISSNLGWYQQKPGQAPRLLIY
GASTRATGIPA
RFSGSGSGTEFTLTISSLQSEDFAVYYCQQYRSWPPLTFGGGTKVEIKRTVA
APSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGE
C (SEQ ID NO:10)

### Example 2. Preparation of anti-B7H4 antibody-drug conjugate (ADC)

### 2.1. Preparation of cytotoxic drug (payload)

### Preparative example 1

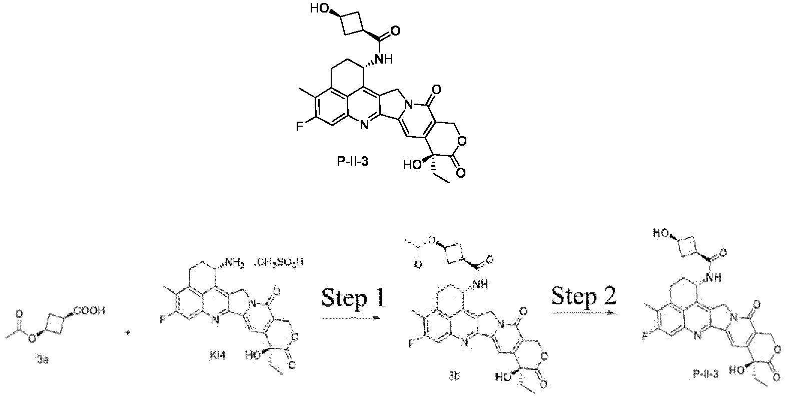

### Step 1:

Under nitrogen protection, to a solution of KI4 (900 mg, 1.69 mmol), HATU (691 mg, 1.88 mmol), and 3a (320 mg, 2.00 mmol) in DMF (18 mL) at 0°C was added DIEA (500 mg, 3.87 mmol). The mixture was stirred at 25°C for 3 hours. TLC (EA) showed that the raw materials were completely reacted. The reaction liquid was added dropwise to 320 mL of deionized water and then filtered to obtain a grey solid (850 mg, yield: 87%).

### Step 2:

To 3b (100 mg, 0.174 mmol) in MeOH/DCM (1/1, 3 mL) was added NaHCO3 (solid, 42 mg, 0.50 mmol) and the mixture was stirred at 25°C for 3 hours. TLC (EA) showed that the reaction was completed. The reaction liquid was filtered and then spun to dryness at low temperature. The residue was slurried with aq. HCl (0.5 M, 10 mL), filtered, subjected to prep-HPLC (0.1% TFA) and lyophilized to obtain a grey solid (15 mg, yield: 16%).

MS m/z (ESI): 534 [M+1];
H-NMR (400 MHz, DMSO-D): 8.45(d, 1H), 7.81 (d, 1H), 7.32 (s, 1H), 6.52 (m, 1H), 5.58-5.56 (m, 1 H), 5.44 (s, 2H), 5.14 (dd, 2H), 3.96 (m, 1H), 3.48 (m, 1H), 3.19 (m, 2H), 2.53-2.28 (m, 3H), 2.48 (s, 3H), 2.20-2.00(m, 4H), 1.95-1.80 (m, 2H), 0.89 (t, 3H).

### Preparative example 2

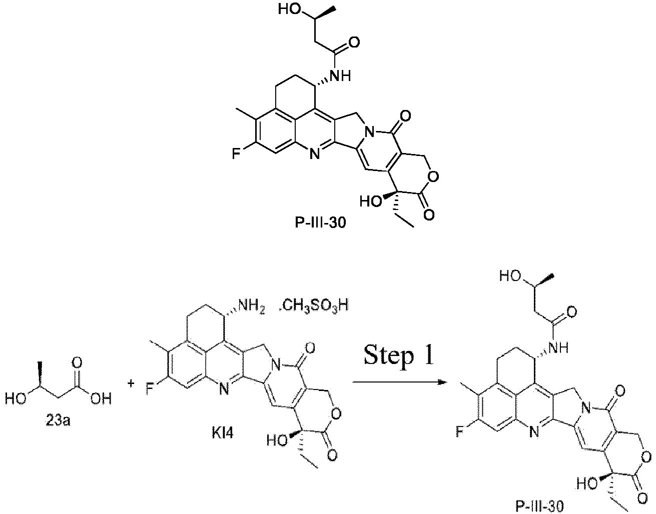

### Step 1

Under nitrogen protection, DIEA (60.6 mg, 0.47 mmol) was added dropwise to a solution of KI4 (100 mg, 0.19 mmol), HATU (85.7 mg, 0.23 mmol), and 23a (21.5 mg, 0.21 mmol) in DMF (2 mL). After the addition, the mixture was reacted at 0°C for 2 hours. LCMS showed that the raw materials were completely reacted. The reaction liquid was added dropwise to 20 mL of water and the mixture was stirred to precipitate a solid, which was filtered to obtain a grey solid P-III-30 (60.2 mg, yield: 61%). MS-ESI: m/z 522.2 [M+H]+.

1H NMR (400 MHz, DMSO-d6) δ 8.42 (d, J = 8.7 Hz, 1H), 7.79 (d, J = 11.0 Hz, 1H), 7.30 (s, 1H), 6.53 (s, 1H), 5.62 - 5.53 (m, 1H), 5.42 (s, 2H), 5.30 - 5.16 (m, 2H), 4.63 (d, J = 4.6 Hz, 1H), 4.09 - 3.99 (m, 1H), 3.22 - 3.11 (m, 2H), 2.40 (s, 3H), 2.28 (dd, J = 13.7, 7.2 Hz, 1H), 2.22 - 2.08 (m, 3H), 1.94 - 1.78 (m, 2H), 1.08 (d, J = 6.1 Hz, 3H), 0.87 (t, J = 7.3 Hz, 3H).

### Preparative example 3

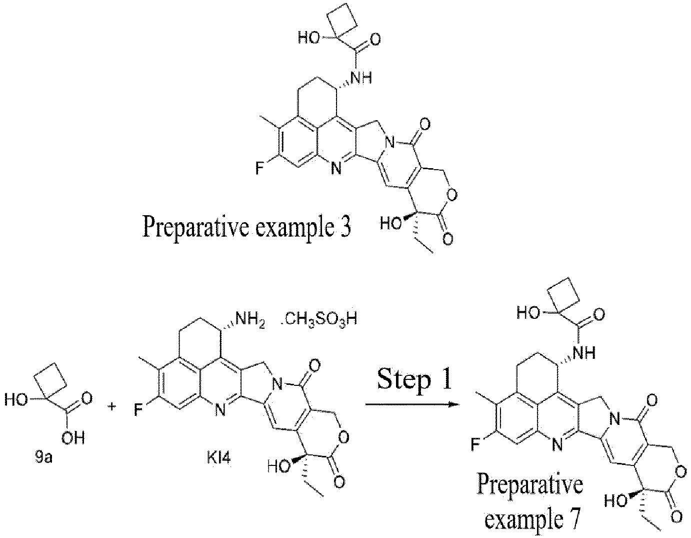

### Step 1

KI4 (50 mg, 0.094 mmol), 25a (11 mg, 0.094 mmol), and HATU (39 mg, 0.103 mmol) were taken and added to DMF (3 mL). After nitrogen replacement, DIEA (30 mg, 0.235 mmol) was added and the mixture was reacted at 25°C for 1.5 hours. LCMS showed that the reaction was completed. The reaction liquid was added dropwise to water under stirring (50 mL). After the addition, the mixture was left to stand for 5 minutes and then filtered. The filter cake was lyophilized to obtain grey solid 25 (20 mg, yield: 40%).

MS-ESI: m/z 534.3 [M+H]+;
1H NMR (400 MHz, DMSO-d6) δ 8.26 (d, J = 9.1 Hz, 1H), 7.72 (d, J = 10.9 Hz, 1H), 7.28 (s, 1H), 6.51 (s, 1H), 6.12 (s, 1H), 5.59 - 5.50 (m, 1H), 5.40 (s, 2H), 5.15 (d, J = 18.8 Hz, 1H), 4.98 (d, J= 19.0 Hz, 1H), 3.28 - 3.16 (m, 1H), 3.15 - 3.02 (m, 1H), 2.74 - 2.52 (m, 2H), 2.36 (s, 3H), 2.24 - 2.04 (m, 4H), 1.92 - 1.79 (m, 4H), 0.86 (t, J = 7.3 Hz, 3H).

### Reference example 1

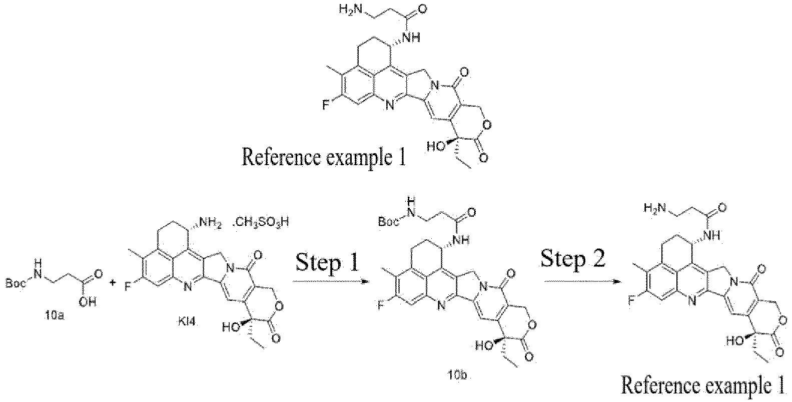

### Step 1

KI4 (50 mg, 0.094 mmol), 26a (18 mg, 0.094 mmol), and HATU (39 mg, 0.103 mmol) were taken and added to DMF (3 mL). After nitrogen replacement, DIEA (48 mg, 0.376 mmol) was added and the mixture was reacted at 25°C for 1.5 hours. LCMS showed that the reaction was completed. The reaction liquid was added dropwise to water under stirring (50 mL). After the addition, the mixture was left to stand for 5 minutes and then filtered. The filter cake was lyophilized to obtain grey solid 26b (30 mg, yield: 52%). MS-ESI: m/z 607.4 [M+H]+.

### Step 2

26b (30 mg, 0.049 mmol) was taken and dissolved in DCM (2 mL). After N2 replacement, the mixture was cooled to 0°C, and TFA (0.5 mL) was added. After the resulting mixture was reacted at 0°C for 1.5 hours, LCMS showed that the reaction was completed. The reaction liquid was spun to dryness at low temperature, and extracted with DCM once. Then acetonitrile and water were added for lyophilization to obtain a yellow solid (20 mg, yield: 80%).

MS-ESI: m/z 507.1 [M+H]+;
1H NMR (400 MHz, DMSO-d6) δ 8.67 (d, J = 8.6 Hz, 1H), 7.86 - 7.66 (m, 4H), 7.32 (s, 1H), 6.56 (brs, 1H), 5.63 - 5.54 (m, 1H), 5.43 (s, 2H), 5.29 (d, J = 18.9 Hz, 1H), 5.22 (d, J = 18.9 Hz, 1H), 3.23 - 3.15 (m, 2H), 3.13 - 3.03 (m, 2H), 2.57 - 2.51 (m, 2H), 2.43 - 2.38 (m, 3H), 2.27 - 2.08 (m, 2H), 1.94 - 1.78 (m, 2H), 0.87 (t, J = 7.3 Hz, 3H).

### 2.2. Preparation of linker-cytotoxin (linker-payload)

### Linker-cytotoxin X1

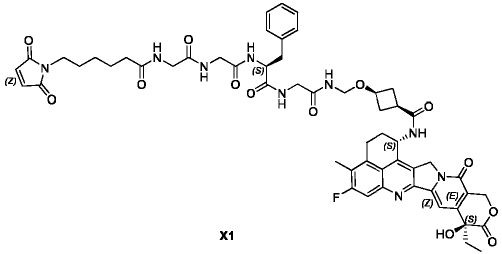

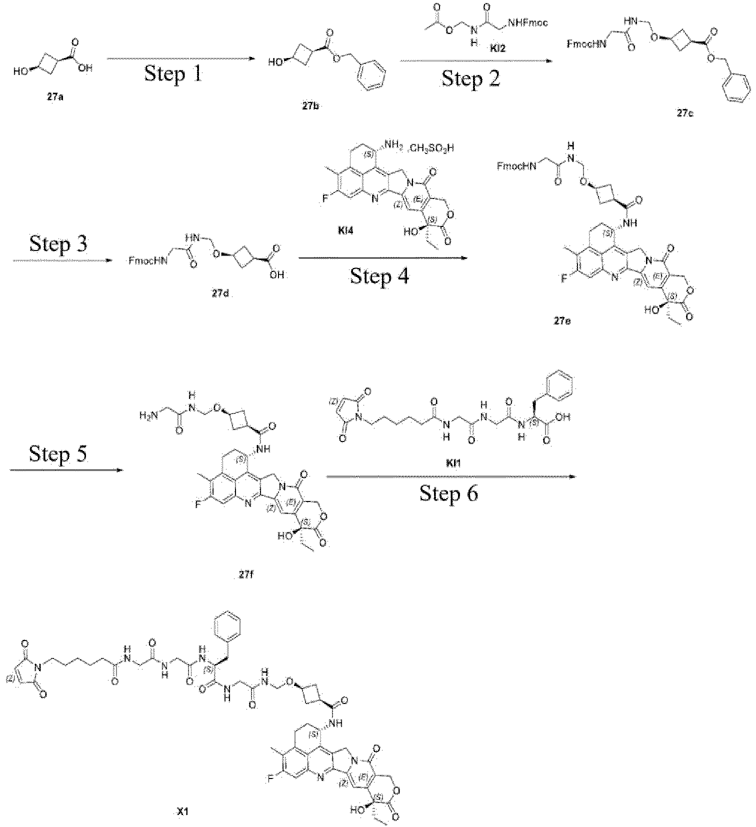

### Step 1

Under nitrogen protection, to a solution of 27a (5.00 g, 43.0 mmol) and NaHCO3 (10.9 g, 129 mmol) in DMF (50 mL) was added dropwise benzyl bromide (11.0 g, 64.6 mmol), and the mixture was reacted at 25°C for 17 hours. TLC (PE/EA = 2/1) showed that the reaction was completed. The reaction liquid was added to water (500 mL), and the resulting mixture was extracted with EA (250 mL) twice. After liquid separation, the organic phase was washed with saturated sodium chloride aqueous solution (500 mL), dried over anhydrous Na2SO4, concentrated and purified by column chromatography (PE : EA = 3 : 2) to obtain a colourless liquid (5.1 g, yield: 57.1%).

### Step 2

Under nitrogen protection, a solution of 27b (4.50 g, 21.8 mmol) in THF (10 mL) was added dropwise to a solution of KI2 (4.00 g, 10.9 mmol) and TsOH (800 mg, 4.65 mmol) in THF (30 mL) at 0°C, and the resulting mixture was reacted at 25°C for 2 hours. TLC (PE/EA = 1/2) showed that the reaction was completed. The reaction liquid was added to water (200 mL), and the resulting mixture was extracted with EA (200 mL) twice. After liquid separation, the organic phase was dried over anhydrous Na2SO4, concentrated and purified by column chromatography (PE/EA=3/2) to obtain a white solid (1.56 g, yield: 26%).

### Step 3

Under hydrogen atmosphere, Pd/C (80 mg) was added to a mixed solution of 27c (800mg, 1.55mmol) in EtOH (8 mL) and EA (8 mL) at 0°C, and the mixture was stirred at 0°C for 2.5 hours. LCMS showed that the reaction was completed. The reaction liquid was filtered over celite, and the filter cake was washed with EA (200 mL). After concentration, the residue was dissolved with THF (20 mL) and spun to dryness to obtain a white solid (600 mg, yield: 91%).

### Step 4

Under nitrogen protection, DIEA (152 mg, 1.18 mmol) was added to a solution of 27d (220 mg, 0.515 mmol), HY-13631A (250 mg, 0.47 mmol) and HATU (214 mg, 0.56 mmol) in DMF (6 mL) at 0°C, and the resulting mixture was reacted at 0°C for 2 hours. LCMS showed that the reaction was completed. The reaction liquid was added to citric acid aqueous solution (pH = 4) (150 mL). The resulting mixture was filtered, and the filter cake was washed with water (175 mL), filtered to dryness and then dried with an oil pump to obtain a brown solid (260 mg, yield: 66%).

### Step 5

Under nitrogen protection, diethylamine (8 mL) was added dropwise to a solution of 27e (260 mg, 0.309 mmol) in DCM (30 mL) at 0°C, and the mixture was reacted at 0°C for 3 hours. LCMS showed that the reaction was completed. The reaction liquid was added to a petroleum ether solution (600 mL) at 0°C, and a solid was precipitated and left to stand. When the solid was adsorbed onto the bottom of the flask, the solution was poured out and the solid was dried with an oil pump to obtain a brown solid (90 mg, yield: 47.1%).

### Step 6

Under nitrogen protection, HATU (74 mg, 0.19 mmol) was added to a solution of 27f(90 mg, 0.13 mmol), KI-1 (92 mg, 0.19 mmol) and DIEA (50 mg, 0.39 mmol) in DMF (2.5 mL) at 0°C, and the resulting mixture was reacted at 0°C for 2 hours. LCMS showed that the reaction was substantially completed. At 0°C, the reaction liquid was added to citric acid aqueous solution (PH=4, 30 mL), and a flocculent solid was precipitated, filtered, and purified by a preparative plate (DCM/MecOH=10/1) to obtain a light yellow solid X1 (9.2 mg, yield: 6%).
MS m/z (ESI): 1074 [M+1]
H-NMR (400 MHz, MeOD): 7.65 (d, 1H), 7.62 (s, 1H), 7.30-7.21(m, 5H), 6.79 (s, 2H), 5.69-5.65 (m, 1 H), 5.57 (d, 1H), 5.43-5.10 (m, 3H), 4.70 (d, 2H), 4.48-4.39 (m, 2H), 4.10-4.05 (m, 1H), 4.01-3.75 (m, 5H),3.46 (t, 2H), 3.22-3.15 (m, 2H), 3.07-3.00 (m, 1H), 2.75 (m, 1H), 2.62 (m, 1H), 2.45 (s, 3H), 2.37-2.20 (m, 6H), 2.10-2.02 (m, 2H), 2.00-1.92 (m, 2H) 1.68-1.57 (m, 6H), 1.01 (t, 3H)

### Linker-cytotoxin X2

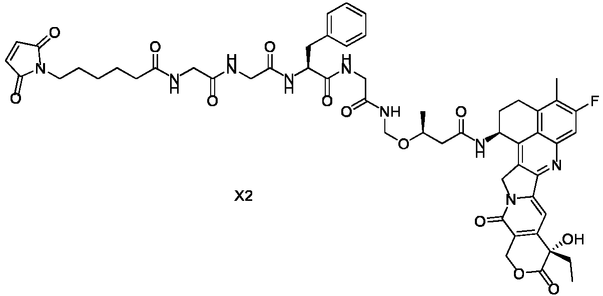

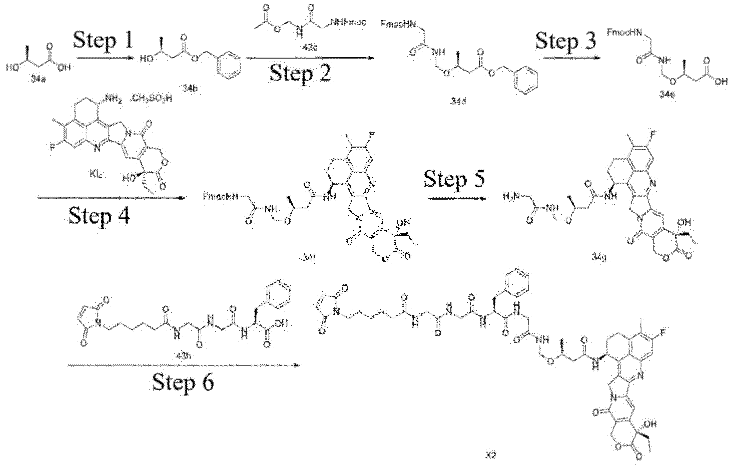

### Step 1

34a (5 g, 48.0 mmol) and K2CO3 (19.9 g, 144.0 mmol) were dissolved in DMF (20 mL), benzyl bromide (12.3 g, 72.0 mmol) was then added dropwise, and the resulting mixture was reacted at 25°C for 17 hours. Upon complete reaction of the raw materials monitored by TLC (PE/EA = 3/1), the reaction liquid was added to water (200 mL), and the mixture was extracted with EA (250 mL). After liquid separation, the organic phase was washed with saturated NaCl, dried over anhydrous Na2SO4, concentrated and purified by column chromatography (PE:EA = 2:1) to obtain a colourless liquid 34b (8.7 g, yield: 93%). MS-ESI: m/z 195.1 [M+H]+.

### Step 2

34c (7.3 g, 19.8 mmol) and TsOH (1.46 g, 8.5 mmol) were taken and dissolved in THF (20 mL). Under nitrogen protection, the mixture was cooled to 0°C, and a solution of 43b (7.7 g, 39.6 mmol) in THF (10 mL) was added dropwise. After the addition, the mixture was reacted at 0°C for 2 hours. TLC (PE/EA = 2/1) showed that most of the raw materials were reacted. The reaction liquid was poured into water (100 mL), and the resulting mixture was extracted with DCM (100 mL). After liquid separation, the organic phase was washed with saturated NaCl, dried over anhydrous Na2SO4, and purified by column chromatography (PE/EA = 1/1) to obtain a colourless viscous substance 34d (3.9 g, yield: 39%). MS-ESI: m/z 503.3 [M+H]+.

### Step 3

Under hydrogen atmosphere, Pd/C (1 g, 10 wt.%) was added to a mixed solution of 34d (1.9 g, 3.78 mmol) in EtOH (100 mL) and EA (100 mL) at 0°C, and the mixture was reacted at 0°C for 3 hours. TLC (PE/EA = 2/1) showed that the reaction was completed. The reaction liquid was filtered over celite, and the filter cake was washed with EA/EtOH (1:1, 100 mL×3). The filtrate was concentrated, and the residue was dissolved with THF (50 mL×3) and spun to dryness. The steps were repeated three times to obtain a grey solid 34e (1 g, yield: 64%). MS-ESI: m/z 435.2 [M+Na]+.

### Step 4

Under nitrogen protection, DIEA (303 mg, 2.35 mmol) was added dropwise to a solution of 34e (426 mg, 1.03 mmol), KI4 (500 mg, 0.94 mmol) and HATU (429 mg, 1.13 mmol) in DMF (20 mL) at 0°C. After the addition, the mixture was reacted at 0°C for 2 hours. LCMS showed that the reaction was completed. The reaction liquid was added dropwise to water (300 mL) and stirred. The mixture was then left to stand for 5 minutes and filtered. After the filter cake was dissolved with a solution of DCM/MeOH (10:1, 100 mL), the mixture was spun to dryness and the residue was stirred for column chromatography (EA:MeOH = 30:1) to obtain a yellow solid 34f (600 mg, yield: 77%). MS-ESI: m/z 830.3 [M+H]+.

### Step 5

Under nitrogen protection, diethylamine (5 mL) was added dropwise to a solution of 34f (150 mg, 0.18 mmol) in DCM (5 mL) at 0°C, and the mixture was reacted at 0°C for 2 hours. LCMS showed that the reaction was completed. A petroleum ether solution (100 mL×6) was added to the reaction liquid, and a solid was precipitated and left to stand. After precipitation, the solution was poured out and the solid was dried with an oil pump to obtain a white powder 34g (120 mg, LCMS showed that the product content was 70%, yield: 76%). MS-ESI: m/z 608.3 [M+H]+.

### Step 6

Under nitrogen protection, a solution of HATU (45 mg, 0.118 mmol) in DMF (1 mL) was added to a solution of 34g (60 mg, 0.099 mmol), 43h (51 mg, 0.108 mmol), and DIEA (32 mg, 0.25 mmol) in DMF (1 mL) at 0°C, and the mixture was reacted at 0°C for 2 hours. LCMS showed that the raw materials were completely reacted. The reaction liquid was directly purified by reversed phase column chromatography (eluents: (MeCN/MeOH = 1/1) : H2O = 60%: 40%) to obtain a yellow solid X2 (14.8 mg, yield: 14%).

MS-ESI: m/z 1062.4 [M+H]+.

1H NMR (400 MHz, Methanol-d4) δ 7.69 - 7.61 (m, 2H), 7.22 - 7.16 (m, 2H), 7.16 - 7.09 (m, 3H), 6.76 (s, 2H), 5.70 - 5.64 (m, 1H), 5.60 (d, J = 16.4 Hz, 1H), 5.40 - 5.31 (m, 2H), 5.26 (d, J = 19.0 Hz, 1H), 4.65 - 4.50 (m, 7H), 4.25 - 4.16 (m, 1H), 3.87 (d, J = 16.7 Hz, 1H), 3.83 - 3.76 (m, 3H), 3.72 (d, J = 17.0 Hz, 2H), 3.44 (t, J = 7.1 Hz, 2H), 3.25 - 3.17 (m, 2H), 3.10 - 3.02 (m, 1H), 2.92 - 2.83 (m, 1H), 2.45 - 2.39 (m, 5H), 2.32 - 2.20 (m, 5H), 1.97 - 1.89 (m, 2H), 1.63 - 1.50 (m, 4H), 1.34 - 1.20 (m, 6H), 0.99 (t, J = 7.3 Hz, 3H).

### Linker-cytotoxin X3

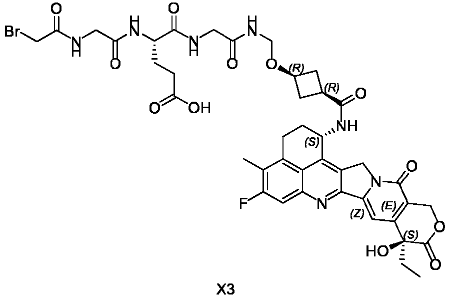

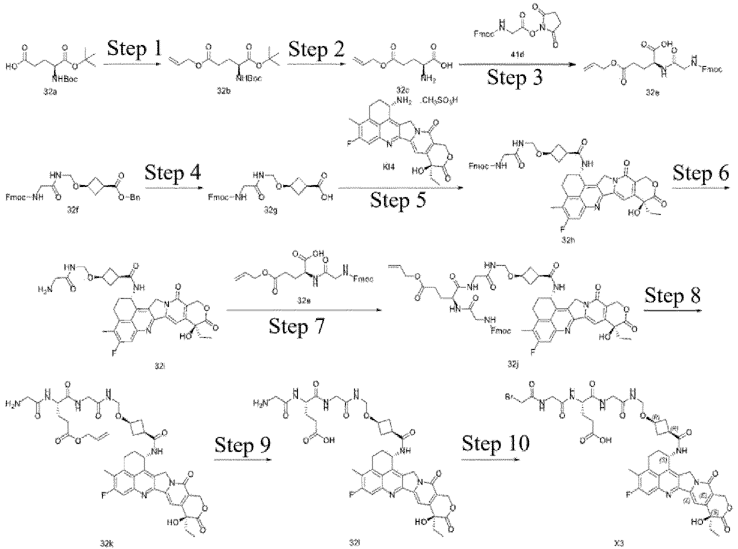

### Step 1

To 32a (2.00 g, 6.6 mmol) and K2CO3 (1.82 g, 13.2 mmol) in MeCN (20 mL) was added bromopropene (960 mg, 7.92 mmol), and the mixture was stirred at 20°C for 5 hours. TLC (PE/EA = 1/2) showed that the reaction was completed. The reaction liquid was poured into water (100 mL). The mixture was adjusted to pH 5, extracted with EA (100 mL) three times, dried over anhydrous sodium sulphate, spun to dryness and purified by column chromatography (PE/EA = 2/1) to obtain a white solid 32b (1.83 g, yield: 81%).

### Step 2

To 32b (1.38 g, 4.02 mmol) in DCM (10 mL) was added TFA (10 mL) and the mixture was stirred at 25°C for 17 hours. TLC (PE/EA = 1/3) showed that the reaction was completed. The reaction liquid was spun to dryness to obtain a yellow viscous substance 32c (0.91 g, yield: not calculated).

### Step 3

To 32c (910 mg, 4.87 mmol) and NaHCO3 (613 mg, 7.3 mmol) in DME/H2O (20 mL/10 mL) was added 41d (1.92 g, 4.87 mmol), and the mixture was stirred at 25°C for 3 hours. TLC (DCM/MeOH = 1/1) showed that the reaction was completed. The reaction liquid was poured into water (100 mL), and the mixture was adjusted to pH 5 with aq. HCl (1 N), extracted with EA (150 mL) twice, dried over anhydrous sodium sulphate, spun to dryness and purified by column chromatography (DCM/MeOH = 20/1) to obtain a white solid 32e (1.53 g, yield: 67%). MS-ESI: m/z 467.4 [M+H]+.

### Step 4

To 32f (3 g, 5.83 mmol) in MeOH (50 mL) was added Pd/C (600 mg), and the mixture was stirred under a hydrogen balloon at 25°C for 5 hours. TLC (EA) showed that the reaction was completed. The reaction liquid was filtered and spun to dryness to obtain a white solid 32g (1.9 g, yield: 77%).

### Step 5

To 32g (789 mg, 1.86 mmol), KI4 (900 mg, 1.69 mmol), and triethylamine (342 mg, 3.38 mmol) in DMF (10 mL) was added HATU (707 mg, 1.86 mmol), and the mixture was stirred at 0°C for 3.5 hours. TLC (EA) showed that the reaction was completed. The reaction liquid was poured into H2O (80 mL), extracted with EA (100 mL) twice, dried over anhydrous sodium sulphate, spun to dryness and purified by column chromatography (EA) to obtain a white solid 32h (1.186 g, yield: 83%). MS-ESI: m/z 842.3 [M+H]+.

### Step 6

32h (1.186 g, 1.41 mmol) in DCM/diethylamine (20 mL, 20/1) was stirred at 25°C for 17 hours. TLC (DCM/MeOH = 10/1) showed that the reaction was completed. The reaction liquid was poured into petroleum ether (200 mL) and filtered to obtain a white solid 32i (768 mg, yield: 88%). MS-ESI: m/z 620.3 [M+H]+.

### Step 7

To 32i (676 mg, 1.09 mmol), 32e (508 mg, 1.09 mmol), and DIEA (423 mg, 3.27 mmol) in DMF (10 mL) was added HATU (414 mg, 1.09 mmol), and the mixture was stirred at 20°C for 17 hours. TLC (PE/EA = 1/5) showed that the reaction was completed. The reaction liquid was poured into water (30 mL) and filtered, and the filter cake was purified by column chromatography (DCM/MeOH = 50/1) to obtain a white solid 32j (511 mg, yield: 44%). MS-ESI: m/z 1068.3 [M+H]+.

### Step 8

### A solution of 32j (482 mg, 0.451 mmol) in diethylamine /DCM (10 mL, 1/5) was stirred at 10°C for 17 hours. TLC (EA) showed that the reaction was completed. The reaction liquid was poured into PE (300 mL) and filtered to obtain a white solid 32k (301 mg, yield: not calculated).

### Step 9

To 32k (301 mg, 0.356 mmol) and Pd(PPh3)4 (82 mg, 0.071 mmol) in THF (5 mL) was added morpholine (93 mg, 1.07 mmol), and the mixture was stirred at 25°C for 5 hours. LCMS showed that the reaction was completed. The reaction liquid was subjected to a preparative method to obtain a white solid 32l (108 mg, yield: 38%). MS-ESI: m/z 806.3 [M+H]+.

### Step 10

To 32l (108 mg, 0.134 mmol) and triethylamine (41 mg, 0.402 mmol) in THF (2 mL) and DMF (2 mL) was added bromoacetyl bromide (27 mg, 0.134 mmol), and the mixture was stirred at 0°C for 1 hour. TLC (DCM/MeOH = 10/1) showed that the reaction was completed. The reaction liquid was directly subjected to a preparative method to obtain a white solid X3 (15 mg, yield: 12%).

MS-ESI: m/z 926.3 [M+H]+.

1H NMR (400 MHz, DMSO-d6) δ 12.11 (s, 1H), 8.54 - 8.42 (m, 3H), 8.27 - 8.16 (m, 2H), 7.78 (d, J = 11.0 Hz, 1H), 7.30 (s, 1H), 6.53 (s, 1H), 5.61 - 5.51 (m, 1H), 5.42 (s, 2H), 5.20 - 5.05 (m, 2H), 4.56 - 4.42 (m, 2H), 4.32 - 4.22 (m, 1H), 3.96 - 3.87 (m, 3H), 3.79 (d, J = 5.6 Hz, 2H), 3.70 (d, J = 5.9 Hz, 2H), 3.25 - 3.08 (m, 2H), 2.61 - 2.53 (m, 2H), 2.45 - 2.36 (m, 4H), 2.36 - 2.22 (m, 3H), 2.20 - 2.03 (m, 4H), 1.99 - 1.68 (m, 4H), 0.87 (t, J = 7.3 Hz, 3H).

### Linker-cytotoxin X4

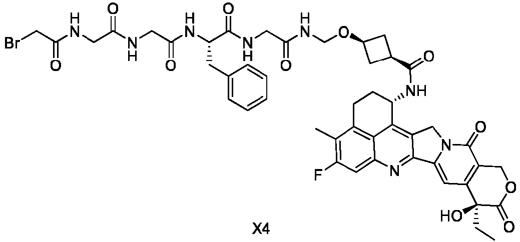

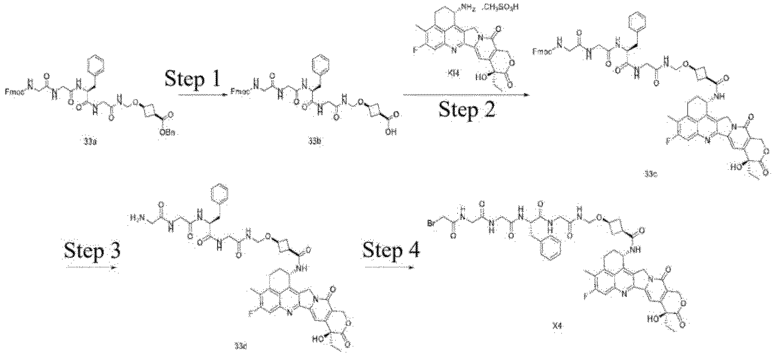

### Step 1

To 33a (2.00 g, 2.58 mmol) in MeOH (20 mL) was added Pd/C (400 mg, 10 wt.%) and the mixture was stirred at 20°C for 5 hours. TLC (EA) showed that the reaction was completed. The reaction liquid was filtered and spun to dryness to obtain a white solid 33b (1.3 g, yield: 74%).

### Step 2

To 33b (0.55 g, 0.802 mmol), KI4 (427 mg, 0.802 mmol), and DIPEA (310 mg, 2.40 mmol) in DMF (5 mL) was added HATU (305 mg, 0.802 mmol), and the mixture was stirred at 0°C for 2 hours. TLC (DCM/MeOH = 1/10) showed that the reaction was completed. The reaction liquid was poured into water (40 mL) and filtered to obtain a crude, which was purified by column chromatography (DCM/MeOH = 20/1) to obtain a yellow solid 33c (360 mg, yield: 41%).

### Step 3

To 33c (360 mg, 0.326 mmol) in DCM (10 mL) was added diethylamine (2 mL). The mixture was stirred at 25°C for 17 hours. TLC (DCM/MeOH = 5/1) showed that the reaction was completed. The reaction liquid was poured into PE (100 mL) and filtered to obtain a white solid 33d (205 mg, yield: 71%). MS-ESI: m/z 881.3 [M+H]+.

### Step 4

To 33d (205 mg, 0.233 mmol) and triethylamine (118 mg, 1.17 mmol) in DMF (1 mL) and water (1 mL) was added a solution of bromoacetyl bromide (94 mg, 0.446 mmol) in THF (2 mL), and the mixture was stirred at 0°C for 1 hour. The reaction liquid was directly subjected to a preparative method to obtain a white solid X4 (15 mg, yield: 6%).

MS-ESI: m/z 1001.2 [M+H]+.

1H NMR (400 MHz, DMSO-d6) δ 8.57 - 8.50 (m, 1H), 8.50 - 8.43 (m, 2H), 8.35 - 8.29 (m, 1H), 8.19 - 8.12 (m, 2H), 7.80 (d, J = 10.8 Hz, 1H), 7.27 - 7.14 (m, 7H), 6.53 (s, 1H), 5.59 - 5.51 (m, 1H), 5.44 - 5.39 (m, 2H), 5.20 - 5.07 (m, 2H), 4.56 - 4.44 (m, 3H), 3.92 (s, 3H), 3.80 - 3.68 (m, 5H), 3.41 (s, 1H), 3.21 - 3.12 (m, 2H), 2.83 - 2.74 (m, 1H), 2.58 - 2.55 (m, 3H), 2.39 (s, 4H), 2.18 - 2.03 (m, 4H), 1.93 - 1.78 (m, 2H), 0.87 (t, J = 7.3 Hz, 3H).

### 2.3. Preparation of anti-B7H4 antibody-drug conjugate

### Preparation of antibody-drug conjugate (ADC) DB1001-X1

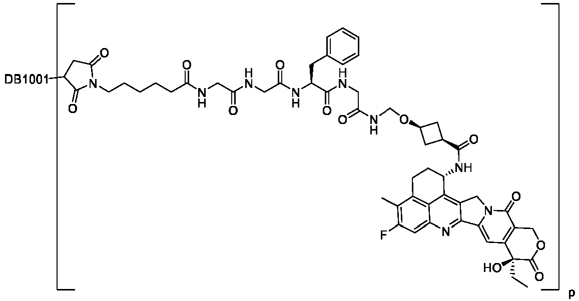

Reducing agent 2 mg/ml aqueous TCEP solution (Tris-2-carboxyethyl-phosphine, manufacturer: Thermo) and protective agent 100 mmol/L aqueous EDTA solution (disodium ethylenediamine tetraacetic acid, manufacturer: Sigma) were formulated separately with ultrapure water.

160 mg of 17.99 mg/ml DB1001 (PR08199) monoclonal antibody was taken and placed in a 600 ml centrifugal bottle. A 30 mM His-HAc, pH 5.5 buffer was added to dilute the antibody to a concentration of 10 mg/ml, and a 100 mM aqueous EDTA solution, accounting for 5% of the total volume of the reaction liquid, was added. After the mixture was shaken and mixed uniformly, 2 mg/ml aqueous TCEP solution was then added at a molar ratio of TCEP to the antibody of 2.3:1 for antibody reduction. The resulting mixture was then shaken and mixed uniformly, and reacted at 37°C for 2 h in a refrigerated thermomixer. The aforementioned Linker-payload DMA solution was added according to the molar ratio of the final concentration of the drug to that of the antibody of 12:1, DMA, accounting for 10% of the total volume of the reaction solution, was added, and the resulting mixture was shaken and mixed uniformly, and then reacted at 4°C for 1 h in the refrigerated thermomixer. An ultrafiltration tube (MWCO 30 KD, manufacturer: Millipore) was used for the replacement of the sample preservation buffer. Ultrafiltration was performed 3 times with 30 mM His-HAc, pH 5.5 buffer containing 10% DMSO and then 6 times with 30 mM DMSO-free His-HAc, pH 5.5 buffer to obtain 125.6 mg of the antibody-drug conjugate DB1001-X1 (concentration: 12.854 mg/mL, yield: 78.5%).

The antibody-drug conjugate DB1001-X1 had a drug-to-antibody ratio (DAR) of 3.89 and an SEC purity of 100% as detected by HIC.

### Preparation of antibody-drug conjugate (ADC) DB1001-X2

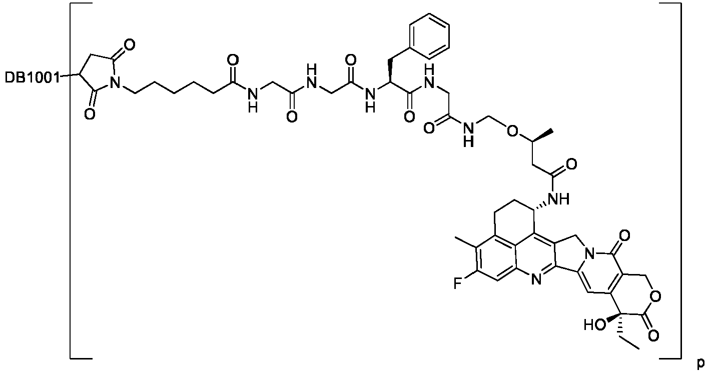

Reducing agent 2 mg/ml aqueous TCEP solution (Tris-2-carboxyethyl-phosphine, manufacturer: Thermo) and protective agent 100 mmol/L aqueous EDTA solution (disodium ethylenediamine tetraacetic acid, manufacturer: Sigma) were formulated separately with ultrapure water.

Linker-payload X2 was dissolved in dry DMA (N,N-dimethylacetaamide, manufacturer: Sinopharm) to formulate a 10 mg/mL linker-payload DMA solution.

160 mg of 17.99 mg/ml DB1001 (PR08199) monoclonal antibody was taken and placed in a 600 ml centrifugal bottle. A 30 mM His-HAc, pH 5.5 buffer was added to dilute the antibody to a concentration of 10 mg/ml, and a 100 mM aqueous EDTA solution, accounting for 5% of the total volume of the reaction liquid, was added. After the mixture was shaken and mixed uniformly, 2 mg/ml aqueous TCEP solution was then added at a molar ratio of TCEP to the antibody of 2.3:1 for antibody reduction. The resulting mixture was then shaken and mixed uniformly, and reacted at 37°C for 2 h in a refrigerated thermomixer. The aforementioned Linker-payload DMA solution was added according to the molar ratio of the final concentration of the drug to that of the antibody of 12 : 1, DMA, accounting for 10% of the total volume of the reaction solution, was added, and the resulting mixture was shaken and mixed uniformly, and then reacted at 4°C for 1 h in the refrigerated thermomixer. An ultrafiltration tube (MWCO 30 KD, manufacturer: Millipore) was used for the replacement of the sample preservation buffer. Ultrafiltration was performed 3 times with 30 mM His-HAc, pH 5.5 buffer containing 10% DMSO and then 6 times with 30 mM DMSO-free His-HAc, pH 5.5 buffer to obtain 128.3 mg of the antibody-drug conjugate DB1001-X2 (concentration: 10.522 mg/mL, yield: 80.18%).

The antibody-drug conjugate DB1001-X2 had a drug-to-antibody ratio (DAR) of 5.4 and an SEC purity of 98.1% as detected by HIC.

### Preparation of reference ADC-1

Reference ADC-1 was prepared with reference to compound 34 (hu2F7-exatecan) in WO 2020244657 A1.

### Example 3. In vitro proliferation inhibition test of small molecule compound (cytotoxic drug)

### 3.1. In vitro proliferation inhibition test of small molecule compound (cytotoxic drug) against tumour cells

### Test objective

To detect the inhibitory activity of the drug compound against the proliferation of NCI-N87, JIMT-1 and MBA-MB-231 tumour cells *in vitro.* Cells were treated with different concentrations of the compound *in vitro,* after 6 days of culture, the proliferation of the cells was detected using CTG (CellTiter-Glo^{®} Luminescent Cell Viability Assay, Promega, Cat. No.: G7558) reagent, and the *in vitro* activity of the compound was evaluated according to the IC₅₀ values.
1. Cell culture: NCI-N87/JIMT-1/MBA-MB-231 cells were cultured with 10% FBS RPMI-1640 medium.
2. Preparation of cells: the NCI-N87/JIMT-1/MBA-MB-231 cells in the logarithmic growth phase were taken and washed once with PBS, and 2-3 ml of trypsin was then added for digestion for 2-3 min. After the cells were completely digested, 10-15 ml of cell culture medium was added to elute the digested cells, the cells were centrifuged at 1000 rpm for 5 min, the supernatant was discarded, and then 10-20 ml of cell culture medium was added to resuspend the cells to prepare a single cell suspension.
3. Cell plating: the NCI-N87/JIMT-1/MBA-MB-231 single cell suspensions were mixed uniformly, and respectively adjusted to a live cell density of 6×10⁴ cells/ml with a cell culture medium, and the density-adjusted cell suspensions were mixed uniformly, and added to 96-well cell culture plates at 50 ul/well. The culture plates were cultured in an incubator for 18 hours (37°C, 5% CO₂).
4. Preparation of compound: the compound was dissolved in DMSO to formulate a stock solution with an initial concentration of 10 mM.
   A total of 8 concentrations of the small molecule compound were involved: 300, 100, 30, 10, 3, 1, 0.3, and 0.1 nM, respectively.
5. Sample loading operation: different concentrations of the formulated samples to be tested were added to culture plates, with two replicate wells for each sample. The culture plates were incubated in an incubator for 6 days (37°C, 5% CO₂).
6. Color development operation: the 96-well cell culture plates were taken out, 50 ul of CTG reagent was added to each well, and the plates were incubated for 10 minutes at room temperature.
7. Plate reading operation: the 96-well cell culture plates were taken out, and placed in a microplate reader, and the chemiluminescence was determined using the microplate reader.

Data analysis: the data was processed and analysed using Microsoft Excel and Graphpad Prism 5.

**Table 1 IC₅₀ values of small molecule compound of the present application for inhibition of tumour cell proliferation in vitro**

| Compound No. | NCI-N87 | JIMT-1 | MDA-MB-231 |
|---|---|---|---|
| | IC₅₀ (nM) | IC₅₀ (nM) | IC₅₀ (nM) |
| Preparative example 3 | - | 23.1 | - |
| P-II-3 | 9.558 | 5.0 | 9.4 |

Conclusion: according to the results in Table 1, the toxin of the drug conjugate of the present application has significantly enhanced inhibitory activity against the proliferation of NCI-N87 cells, JIMT-1 cells and MDA-MB-231 cells.

### 3.2. In vitro proliferation inhibition test of small molecule compound (cytotoxic drug) against tumour cells

### Test objective

To detect the inhibitory activity of the drug compound against the proliferation of NCI-N87 and Colo205 tumour cells *in vitro.* Cells were treated with different concentrations of the compound *in vitro,* after 6 days of culture, the proliferation of the cells was detected using CTG (CellTiter-Glo^{®} Luminescent Cell Viability Assay, Promega, Cat. No.: G7558) reagent, and the *in vitro* activity of the compound was evaluated according to the IC₅₀ values.
1. Cell culture: NCI-N87/Colo205 cells were cultured with 10% FBS RPMI-1640 medium.
2. Preparation of cells: the NCI-N87/Colo205 cells in the logarithmic growth phase were taken and washed once with PBS, and 2-3 ml of trypsin was then added for digestion for 2-3 min. After the cells were completely digested, 10-15 ml of cell culture medium was added to elute the digested cells, the cells were centrifuged at 1000 rpm for 5 min, the supernatant was discarded, and then 10-20 ml of cell culture medium was added to resuspend the cells to prepare a single cell suspension.
3. Cell plating: the NCI-N87/Colo205 single cell suspensions were mixed uniformly, and respectively adjusted to a live cell density of 6 × 10⁴ cells/ml with a cell culture medium, and the density-adjusted cell suspensions were mixed uniformly, and added to 96-well cell culture plates at 50 ul/well. The culture plates were cultured in an incubator for 18 hours (37°C, 5% CO₂).
4. Preparation of compound: the compound was dissolved in DMSO to formulate a stock solution with an initial concentration of 10 mM.
   A total of 8 concentrations of the small molecule compound were involved: 300, 100, 30, 10, 3, 1, 0.3, and 0.1 nM, respectively.
5. Sample loading operation: different concentrations of the formulated samples to be tested were added to culture plates, with two replicate wells for each sample. The culture plates were incubated in an incubator for 6 days (37°C, 5% CO₂).
6. Colour development operation: the 96-well cell culture plates were taken out, 50 ul of CTG reagent was added to each well, and the plates were incubated for 10 minutes at room temperature.
7. Plate reading operation: the 96-well cell culture plates were taken out, and placed in a microplate reader, and the chemiluminescence was determined using the microplate reader.

Data analysis: the data was processed and analysed using Microsoft Excel and Graphpad Prism 5.

**Table 2 IC₅₀ values of small molecule compound of the present application for inhibition of NCI-N87 and Colo205 cell proliferation in vitro**

| Compound No. | Abs-IC₅₀ (nM) | |
|---|---|---|
| | P-III-30 | Reference example 1 |
| Colo205 | 102.9 | >1000 |
| NCI-N87 | 6.5 | >300 |

Conclusion: according to the results in Table 2, the toxin of the drug conjugate of the present application has significant inhibitory activity on the proliferation of NCI-N87 and Colo205 cells and is significantly superior to Reference example 1.

### Example 4. Endocytotic activity for antibody-drug conjugate

### Test objective

To detect the endocytosis of the anti-B7H4 antibody-drug conjugate of the present application by B7H4-expressing MDA-MB-468 cells. The cells were co-incubated with the antibody-drug conjugate (ADC) at a fixed concentration and the endocytosis indicator pHrodo, and the internalization ability of the antibody-drug conjugate was evaluated by observing the fluorescent signals generated at different time points by pHrodo, which entered the cells with the antibody-drug conjugate.

### Experimental method

1. Cell culture: MDA-MB-468 cells were cultured with 10% FBS Leibovitz's L-15 medium.
2. Preparation of cells: the MDA-MB-468 cells in the logarithmic growth phase were taken and washed once with PBS, and then digested for 2-3 min. After the cells were completely digested, 10-15 ml of cell culture medium was added to elute the digested cells, the cells were centrifuged at 1000 rpm for 5 min, and the supernatant was discarded. A cell culture medium was added to resuspend the cells to prepare a single cell suspension, and the single cell suspension was adjusted to a live cell density of 3×10⁵ cells/ml.
3. Cell plating: the single cell suspension was added to 96-well cell culture plates at 50 µL/well. The culture plates were cultured in an incubator for 48 hours (37°C, 5% CO₂).
4. Sample loading operation: the antibody-drug conjugate to be tested was co-incubated with Fab-pHrodo to form a complex with a concentration of 120 nM, and the complex was subjected to 5-fold gradient dilution to adjust the concentration, and added to the cells at 50 ul/well. A total of 8 concentrations were involved, with two replicate wells for each concentration.
5. Cell culture: the culture plates were incubated in an incubator for 48 hours (37°C).
6. Plate reading operation: at the corresponding time points, the 96-well cell culture plates were taken out, the cells were digested, and then the cell count and fluorescence values were read by FACS.

**Table 3 Fluorescence signals generated at different time points during the endocytosis of the antibody-drug conjugate of the present application by MDA-MB-468 cells**

| Sample concentration | Fluorescence value (DB 1001-X2) | | Fluorescence value (DB1001-X1) | |
|---|---|---|---|---|
| 30 nM | 343 | 327 | 334 | 374 |
| 6 nM | 219 | 235 | 282 | 219 |
| 1.2 nM | 119 | 130 | 143 | 139 |
| 0.24 nM | 103 | 105 | 122 | 121 |
| 0.048 nM | 97.8 | 104 | 106 | 114 |
| 0.0096 nM | 97.1 | 98.2 | 104 | 107 |
| 0.00192 nM | 96.6 | 97.4 | 109 | 102 |
| 0.000384 nM | 98.2 | 98.1 | 103 | 96.6 |

Conclusion: according to the results in Table 3, MDA-MB-468 cells with high expression of B7H4 exhibit endocytosis for the antibody-drug conjugate of the present application.

### Example 5: In vitro proliferation inhibition test of antibody-drug conjugate against tumour cells

To assess the cell proliferation inhibitory effect using the CellTiter-Glo^{®} Luminescent Cell Viability Assay (i.e., CTG) after anti-B7H4 ADCs DB1001-X1 and DB1001-X2 were incubated with MDA-MB-468 cells positive for B7H4 expression and MDA-MB-231 cells negative for B7H4 expression for 7 days.

The cells in the logarithmic growth phase were collected, and plated at a density of 2000 cells/well, and the cell plates were cultured overnight in an incubator with 5% CO₂ at 37°C. On the second day of the experiment, the DB1001-X1 and the DB1001-X2 were subjected to 5-fold dilution with a complete medium to obtain 9 concentration gradients (starting from the highest concentration of 300 nM) of the drugs, and then the drugs were added to the cell culture plates at 50 µL/well. The complete medium was used as a blank control; 2 replicate wells were set up; and the plates were incubated in the incubator at 37°C for another 7 days. After the incubation was completed, the cell culture plates were taken out and equilibrated to room temperature, and 50 µL of CTG detection reagent (Promega, Cat#: G7573) was added to each well. Subsequently, the plates were shaken and mixed uniformly, and left to stand in the dark for 10 minutes, and then the signal values were read using a microplate reader. Using GraphPad Prism software, a sigmoidal dose-response curve was plotted by using a non-linear regression model, and the IC₅₀ value was calculated. Cell viability calculation formula=(Lum_{drug to be tested}-Lum_{blank control})/(Lum_{solvent blank control}-Lum_{blank control}) × 100%.

The experimental results are as shown in the following table:

**Table 4: In vitro proliferation inhibitory activity of antibody-drug conjugates**

| Cell | Drug | IC₅₀ (nM) |
|---|---|---|
| MDA-MB-468 | DB1001-X1 | 30.3 |
| | DB1001-X2 | 50.7 |
| MDA-MB-321 | DB1001-X1 | >200 |
| | DB1001-X2 | >200 |

Experimental conclusion: according to the results in Table 4, the antibody-drug conjugates DB1001-X1 and DB1001-X2 of the present application have significantly enhanced inhibitory activity on the proliferation of MDA-MB-468 cells positive for B7H4 expression.

### Example 6: Pharmacodynamic evaluation of antibody-drug conjugates in tumour-bearing mice (MDA-MB-468 cells)

To investigate the inhibitory effect of DB1001-X1 and DB1001-X2 on the tumours formed *in vivo,* wherein the anti-tumour effects of DB1001-X1 and DB1001-X2 were assessed after transplanted tumours were established in mice with MDA-MB-468 cells positive for B7H4 expression.
1. Test drugs and materials
   Blank control group (control group): Physiological saline
   DB1001-X1 (treatment group): 3 mg/kg
   DB1001-X1 (treatment group): 10 mg/kg
   DB1001-X2 (treatment group): 3 mg/kg
   DB1001-X2 (treatment group): 10 mg/kg
2. Formulation method: all samples were diluted and formulated with physiological saline.
3. Experimental animals: 6-8-week-old female CB-17 SCID mice, purchased from Beijing Vital River Laboratory Animal Technology Co., Ltd.
4. Experimental method:
   6-8-week-old female NOD/SCID mice were inoculated subcutaneously on the right back with 10 × 10⁶ MDA-MB-468 cells. When the tumour grew to about 172 mm³, the tumour-bearing mice were randomly grouped using StudyDirectorTM. On the same day (day 0), the mice were injected intravenously (i.v.) with DB1001-X1 or DB1001-X2 at doses of 3 mg/kg and 10 mg/kg, respectively, for a total of 1 injection. The tumour volumes and body weights were measured twice a week, and the data were recorded.

There were 5 mice in each vehicle control group or treatment group. The tumour inhibition rate was calculated by measuring the tumour volume.

The tumour volume calculation formula is: V = 0.5 *a* × *b²*, where *a* and *b* represent the long and short diameters of the tumour, respectively. The tumour inhibition effect of the compound was evaluated by T/C (%). T/C (%) is an indicator reflecting tumour growth inhibition in percentage, with T and C representing the mean tumour volume on a certain day in the administration group and the control group, respectively. The tumour growth inhibition rate was calculated using the following formula: TGI (%) = [1-(Tᵢ-T₀)/ (Vᵢ-V₀)] × 100, where Tᵢ is the mean tumour volume on a certain day in the administration group, T₀ is the mean tumour volume at the beginning of administration in the same administration group; Vᵢ is the mean tumour volume on a certain day (the same day as Tᵢ) in the vehicle control group, and V₀ is the mean tumour volume at the beginning of administration in the vehicle control group. Significance analysis between the test article groups and the Vehicle group was performed by one-way ANOVA.

**Table 5 Tumour volume ¹(mm³) in each group at different time points**

| **Group** | **1** | **2** | **3** | **4** | **5** |
|---|---|---|---|---|---|
| **Days** | Blank control group | DB1001-X2, 3 mg/kg | DB1001-X2, 10 mg/kg | DB1001-X1, 3 mg/kg | DB1001-X1, 10 mg/kg |
| **0²** | 171 ± 11 | 172 ± 11 | 170 ± 14 | 172 ± 12 | 172 ± 16 |
| **4** | 196 ± 12 | 179 ± 10 | 173 ± 14 | 181 ± 11 | 177 ± 17 |
| **7** | 229 ± 15 | 143 ± 8 | 93 ± 11 | 174 ± 9 | 140 ± 14 |
| **11** | 267 ± 15 | 67 ± 3 | 38 ± 1 | 123 ± 8 | 63 ± 4 |
| **14** | 315 ± 22 | 56 ± 3 | 32 ± 1 | 111 ± 8 | 55 ± 4 |
| **18** | 356 ± 27 | 48 ± 2 | 25 ± 1 | 123 ± 8 | 46 ± 4 |
| **21** | 400 ± 34 | 55 ± 3 | 20 ± 0 | 163 ± 6 | 42 ± 7 |

| | | | | | |
|---|---|---|---|---|---|
| Note: 1. Tumour volume is expressed as mean ± standard error; 2. Days after the beginning of administration. | | | | | |

**Table 6 Evaluation of tumour inhibition efficacy of test articles in subcutaneous xenograft tumour models of MDA-MB-468 cell**

| (Calculated based on tumour volume at day 21 after administration) | | | | |
|---|---|---|---|---|
| Group | Tumour volume (mm3) 1 | T/C (%) ² | TGI (%) ² | p value ³ |
| Blank control group, | 400 ± 34 | -- | -- | -- |
| DB1001-X2, 3 mg/kg | 55 ± 3 | 13.78 | 151.33 | 0.002 |
| DB1001-X2, 10 mg/kg | 20 ± 0 | 4.97 | 165.84 | 0.002 |
| DB1001-X1, 3 mg/kg | 163 ± 6 | 40.69 | 103.95 | 0.008 |
| DB1001-X1, 10 mg/kg | 42 ± 7 | 10.62 | 156.50 | 0.002 |

| | | | | |
|---|---|---|---|---|
| 1. Tumour volume is expressed as mean ± standard error; 2. Tumour growth inhibition is reflected by T/C (T/C (%) = T₂₁/ V₂₁ ×100) and TGI (TGI (%) = [1-(T₂₁-T₀)/ (V₂₁-V₀)] × 100); 3. The *p* value is calculated based on the tumour volume (*p* < 0.05 indicates statistical difference, p < 0.01 indicates significant difference). | | | | |

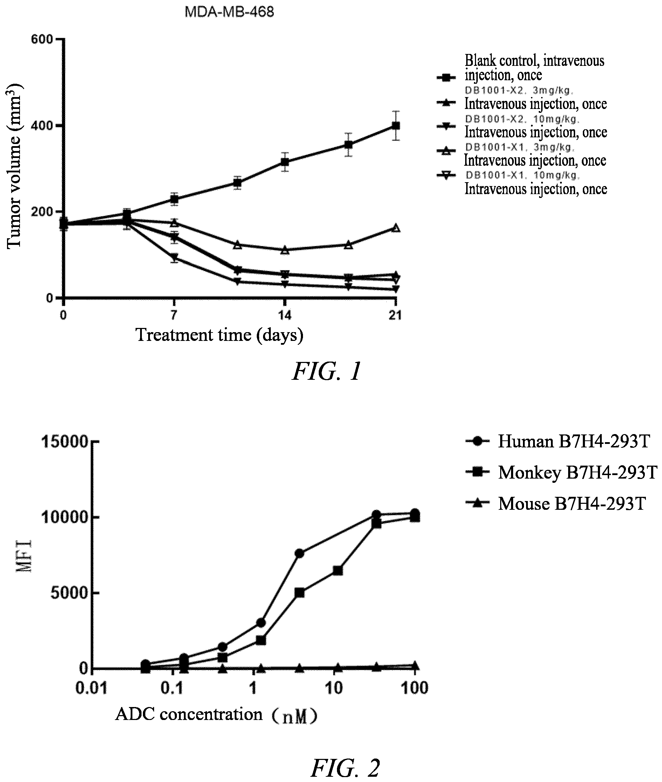

The experimental results are as shown in FIG. 1, Table 5 and Table 6. The antibody-drug conjugates DB1001-X1 and DB1001-X2 exhibit significantly enhanced dose-dependent tumour inhibitory activity after a single dose.

### Example 7. Species cross-reactivity of antibody-drug conjugate

### Test objective

To detect the species cross-reactivity of the antibody-drug conjugate DB1001-X2 in HEK293T cells transfected with human, cynomolgus monkey and mouse B7H4.

### Experimental method

1. All cell strains were cultured in a complete medium at 37°C and 5% CO₂.
2. Cells in the logarithmic growth phase were harvested and the cell viability was detected by trypan blue exclusion to ensure that the cell viability was 90% or greater. The cells were centrifuged at 1000 r/min for 5 min, and then the supernatant was discarded. The cells were washed once with PBS, and resuspended with FACS Buffer to prepare a single cell suspension, and the single cell suspension was adjusted to a cell density of 5 × 10⁶ cells/mL;
3. 50 µL of each cell suspension was added to 96-well plates to achieve the highest concentration of the test article working solution of 100 nM. The test article working solution was sequentially diluted 3-fold for a total of 8 concentrations; and the mixture was mixed uniformly, and then the cells were incubated at 4°C for 40 min.
4. The cells were washed 3 times with FACS Buffer (400 µL for each time), centrifuged at a speed of 1000 r/min for 5 min, and finally resuspended with 100 µL of FACS Buffer;
5. 2 µL of PE-labelled secondary antibody (PE anti-human IgG Fc Antibody) was added, and mixed uniformly, and the cells were incubated at 4°C for 40 min in the dark;
6. The cells were washed 3 times with FACS Buffer (400 µL for each time), centrifuged at a speed of 1000 r/min for 5 min, and finally resuspended with 250 µL of FACS Buffer;
7. The fluorescence values were detected by a flow cytometer.

**Table 7 Affinity of DB1001-X2 for human, monkey and mouse B7H4**

| Cell strain | DB1001-X2 | |
|---|---|---|
| | EC₅₀ (nM) | MFI.Max |
| Human B7H4-293T | 2.18 | 10312 |
| Monkey B7H4-293T | 5.36 | 10869 |
| Mouse B7H4-293T | Not binding | Not binding |

As shown in Table 7 and FIG. 2, DB1001-X2 has similar affinity for human and cynomolgus monkey B7-H4, but does not bind to mouse B7-H4.

In the following examples, Isotype ADC refers to an antibody-drug conjugate, in which the antibody is a negative control antibody and the linker-cytotoxin is X2, and was prepared with reference to DB 1001-X2.

### Example 8. Targeting specificity of antibody-drug conjugate

### Test objective

To detect the binding specificity of the antibody-drug conjugate DB 1001-X2 to cells overexpressing human B7 family proteins by flow cytometry.

### Experimental method

1. HEK293T monoclonal cell strains stably expressing human PD-L1 (B7-H1), PD-L2 (B7-DC), ICOSLG (B7-H2), CD276 (B7-H3), B7H4, VISTA (B7-H5), CD80 (B7-1) and CD86 (B7-2), and a CHO-K1 monoclonal cell strain stably expressing human B7-H7 (HHLA2) were established;
2. All cell strains were cultured in a complete medium at 37°C and 5% CO₂;
3. Cells in the logarithmic growth phase were harvested and the cell viability was detected by trypan blue exclusion to ensure that the cell viability was 90% or greater. The cells were centrifuged at 1000 r/min for 5 min, and then the supernatant was discarded. The cells were washed once with PBS, and resuspended with FACS Buffer to prepare a single cell suspension, and the single cell suspension was adjusted to a cell density of 5×10⁶ cells/mL;
4. 50 µL of each cell suspension was added to 96-well plates to achieve the concentration of the test article working solution of 100 nM or 300 nM. A total of one concentration was involved; and the mixture was mixed uniformly, and then the cells were incubated at 4°C for 40 min;
5. The cells were washed 3 times with FACS Buffer (400 µL for each time), centrifuged at a speed of 1000 r/min for 5 min, and finally resuspended with 100 µL of FACS Buffer;
6. 2 µL of PE-labeled secondary antibody (PE anti-human IgG Fc Antibody) was added, and mixed uniformly, and the cells were incubated at 4°C for 40 min in the dark;
7. The cells were washed 3 times with FACS Buffer (400 µL for each time), centrifuged at a speed of 1000 r/min for 5 min, and finally resuspended with 250 µL of FACS Buffer;
8. The fluorescence values were detected by a flow cytometer.

### Experimental results

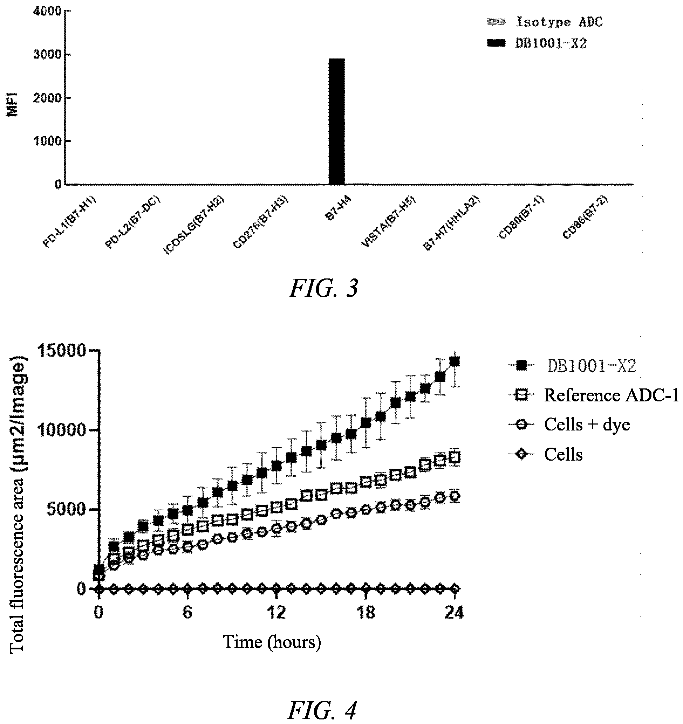

As shown in FIG. 3, DB1001-X2 bound to human B7-H4, and the binding to other human B7 family proteins B7-H1, B7-DC, B7-H2, B7-H3, B7-H5, B7-1, B7-2 and B7-H7 was not detected.

### Experimental conclusion

DB1001-X2 can specifically bind to human B7-H4 protein and has no cross-reactivity to other B7 family proteins.

### Example 9: Endocytotic activity for antibody-drug conjugate

### Test objective

To detect the endocytosis efficiency of B7H4-expressing MDA-MB-468 cells for the anti-B7H4 antibody-drug conjugate of the present application in comparison with other anti-B7H4 antibody-drug conjugates. The cells were co-incubated with the antibody-drug conjugate at a fixed concentration and an endocytosis indicator Incucyte^{®} Fabfluor-pH, and the internalization ability of the antibody-drug conjugate was evaluated by continuously observing the changes in the fluorescent signals of live cells for 24 hours.

### Experimental method

1. Cell culture: MDA-MB-468 cells were cultured with 10% FBS Leibovitz's L-15 medium.
2. Preparation of cells: the MDA-MB-468 cells in the logarithmic growth phase were taken and washed once with PBS, and then digested for 2-3 min. After the cells were completely digested, 10-15 ml of cell culture medium was added to elute the digested cells, the cells were centrifuged at 1000 rpm for 5 min, and the supernatant was discarded. A cell culture medium was added to resuspend the cells to prepare a single cell suspension, and the single cell suspension was adjusted to a live cell density of 1 × 10⁵ cells/ml.
3. Cell plating: the single cell suspension was added to 96-well cell culture plates at 50 µL/well. The culture plates were cultured overnight in an incubator (37°C, 5% CO₂).
4. Labelling of antibody-drug conjugate to be tested: the test article stock solution was diluted to 240 nM 4×working solution (final concentration of 60 nM), Inducyte^{®} Fabfluor-pH stock solution was diluted to 720 nM 4×working solution (final concentration of 180 nM), and the two diluted working solutions were mixed thoroughly and incubated at 37°C for 15 minutes in the dark.
5. Capture and analysis of images: the labelled test article working solution was transferred to the corresponding wells of experimental plates, the experimental plates were transferred to Incucyte live-cell analysis device, the scanning and photographing program was set up, and the images were acquired using Incucyte live-cell analysis system. Quantitation was performed at 1-hour intervals for 24 hours. The analysis results are expressed as: total fluorescence area (µm²/image).

### Experimental results

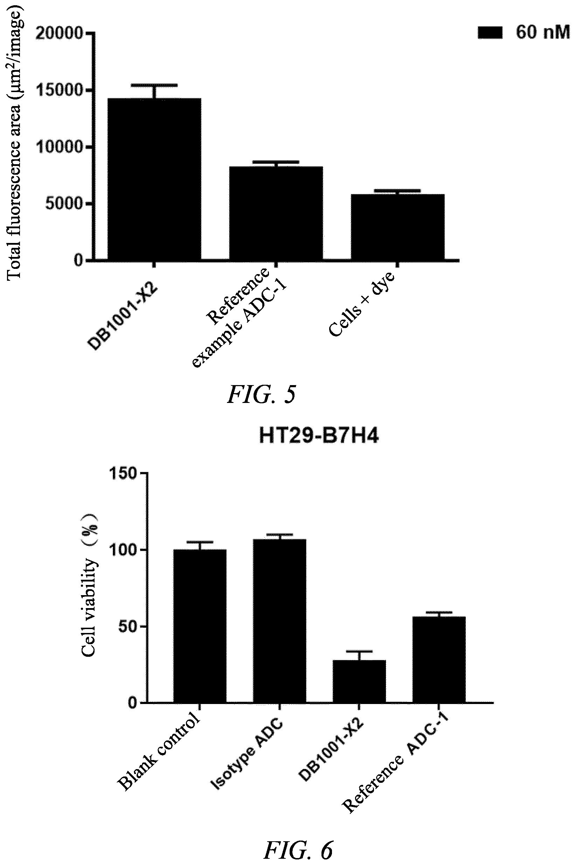

The integrated areas of the fluorescence signals generated at different time points during the endocytosis of the antibody-drug conjugate DB1001-X2 of the present application and the control antibody-drug conjugate reference ADC-1 by MDA-MB-468 cells are as shown in FIG. 4. FIG. 5 shows the comparison of the endocytosis efficiency of MDA-MB-468 cells for the antibody-drug conjugate DB1001-X2 of the present application and the control antibody-drug conjugate.

**Table 8 Antibody-drug conjugate endocytosis of MDA-MB-468**

| | Total fluorescence area (µm²/image) | Fold over blank control |
|---|---|---|
| DB1001-X2 | 14318.1 | 2.4 |
| Reference ADC-1 | 8300.4 | 1.4 |
| Blank control (cells+dye) | 5856.4 | 1.0 |

### Experimental conclusion

According to the results in Table 8, FIG. 4 and FIG. 5, B7H4-expressing MDA-MB-468 cells exhibit endocytosis for the antibody-drug conjugate of the present application, which is superior to that for the reference ADC-1.

### Example 10: In vitro proliferation inhibition test of antibody-drug conjugate against tumour cells (2D cell culture)

### Test objective

To detect, using the CellTiter-Glo^{®} Luminescent Cell Viability Assay (i.e., CTG), the inhibitory effect of the anti-B7H4 antibody-drug conjugate of the present application on the *in vitro* proliferation of B7H4-overexpressing human colorectal cancer cells HT29 in comparison with other anti-B7H4 antibody-drug conjugates.

### Experimental method

The cells in the logarithmic growth phase were collected, and plated at a density of 15000 cells/well, and the cell plates were cultured overnight in an incubator with 5% CO₂ at 37°C. On the second day of the experiment, the test article was diluted with a complete medium to obtain a drug with a final concentration of 10 nM, the drug was then added to cell culture plates at 50 µL/well, the complete medium was used as a blank control, and 3 replicate wells were set up; and the plates were incubated in the incubator at 37°C for another 7 days. After the incubation was completed, the cell culture plates were taken out and equilibrated to room temperature, and 50 µL of CTG detection reagent (Promega, Cat#: G7573) was added to each well. Subsequently, the plates were shaken and mixed uniformly, and left to stand in the dark for 10 minutes, and then the signal values were read using a microplate reader. GraphPad Prism software was used. Cell viability calculation formula = (Lum_{drug to be tested}-Lum_{blank control})/(Lum_{solvent blank control}-Lum_{blank control}) × 100%.

### Experimental results

**Table 9 Inhibitory effect of the antibody conjugate on the in vitro proliferation of HT29-B7H4**

| | Cell viability (%) |
|---|---|
| Blank control | 100.0 |
| Isotype ADC | 106.9 |
| DB1001-X2 | 28.0 |
| Reference ADC-1 | 56.6 |

### Experimental conclusion

According to the experimental results in Table 9 and FIG. 6, the antibody-drug conjugate DB1001-X2 of the present application has significant inhibitory activity against the proliferation of B7H4-overexpressing human colorectal cancer cells HT29, and the activity is superior to that of the reference ADC-1.

### Example 11: In vitro proliferation inhibition test of antibody-drug conjugate against tumour cells (3D cell culture)

### Test objective

To assess the proliferation inhibitory effects of the anti-B7H4 ADC DB1001-X2 and anti-B7H4 monoclonal antibody DB 1001 on human breast cancer cells MX-1 with high expression of B7H4, endometrial carcinoma cells RL95-2 with low expression of B7H4, and breast cancer cells JIMT-1 negative for B7H4 expression under 3D culture conditions using CellTiter-Glo^{®} Luminescent Cell Viability Assay (i.e., CTG).

### Experimental method

The cells in the logarithmic growth phase were collected and a cell culture medium was added to resuspend the cells to prepare a single cell suspension. The single cell suspension was adjusted to a live cell density of 1 × 10⁵ cells/ml, and 3.5 mL of the cell suspension was mixed uniformly with 6.5 mL of 1% methylcellulose, avoiding bubbles as much as possible. 90 µL of the cell suspension was added to each well of 96-well plates, and the plates were cultured overnight in an incubator with 5% CO₂ at 37°C. On the second day of the experiment, the test article was diluted with a complete medium to obtain a 10× solution, 10 µL of the test article solution (the highest final concentration of 100 nM, 9 concentrations, 3-fold dilution) was added to each well, the complete medium was used as a blank control, and 3 replicate wells were set up; and the plates were incubated in the incubator at 37°C for another 6 days. After the incubation was completed, the cell culture plates were taken out and equilibrated to room temperature, and 100 µL of CTG detection reagent (Promega, Cat#: G7573) was added to each well. The plates were shaken and mixed uniformly, then left to stand in the dark for 10 minutes, and then placed at room temperature for 30 minutes to stabilize the luminescence signal. Luminescence was read using EnVision. Using GraphPad Prism software, a sigmoidal dose-response curve was plotted by using a non-linear regression model, and the IC₅₀ value was calculated. Cell viability calculation formula = (Lum_{drug to be tested}-Lum_{blank control})/(Lum_{solvent blank control}-Lum_{blank} control) × 100%.

Isotype ADC: an antibody-drug conjugate, in which the antibody is a negative control antibody and the linker-cytotoxin is X2, prepared with reference to DB 1001-X2.

### Experimental results

**Table 10: Proliferation inhibitory activity of antibody-drug conjugate on tumour cell strains**

| Cell strain | DB1001-X2 | | DB1001 | | Isotype ADC | | P-III-30 | |
|---|---|---|---|---|---|---|---|---|
| | IC₅₀ (nM) | Max inh. (%) | IC₅₀ (nM) | Max inh. (%) | IC₅₀ (nM) | Max inh. (%) | IC₅₀ (nM) | Max inh. (%) |
| MX-1 | 52.396 | 55.47% | >100 | 5.78% | >100 | 7.69% | 1.768 | 84.47% |
| RL95-2 | 69.436 | 60.44% | >100 | 0.65% | >100 | 41.56% | 0.843 | 95.07% |
| JIMT-1 | >100 | 2.53% | >100 | 1.25% | >100 | -4.04% | 24.270 | 70.98% |

### Experimental conclusion

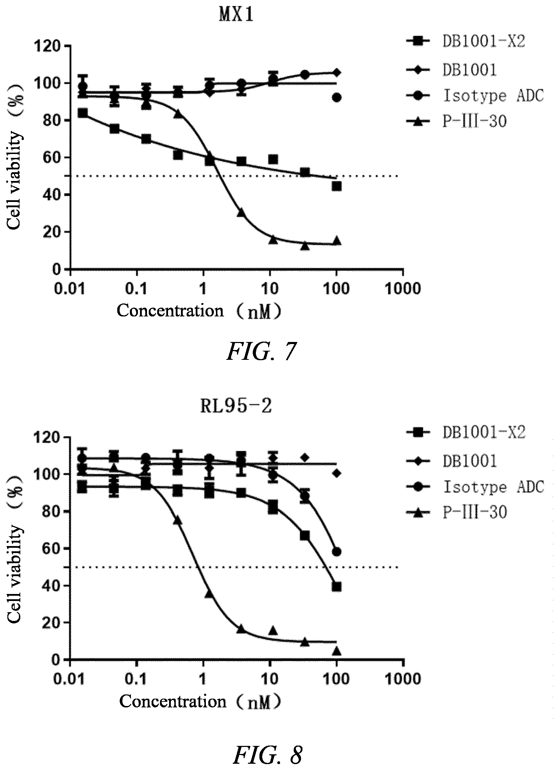

As shown in Table 10 and FIG. 7, FIG. 8 and FIG. 9, DB1001-X2 has strong killing effects on both tumour cell strains with high and low expression of B7H4 *in vitro,* and has no killing effect on cells that do not express B7H4, indicating that the killing effect is dependent on B7H4 expression.

### Example 12: T cell activation of antibody-drug conjugate

### Test objective

To detect the ability of the anti-B7H4 antibody-drug conjugate of the present application to block the inhibition of B7H4 on T cells and to activate T cells for IFN-gamma production in comparison with other anti-B7H4 antibody-drug conjugates.
1. Experimental method: 293T-OS8-human B7H4 cells (KYinno Biotechnology Co., Ltd.) in the logarithmic growth phase were collected, and centrifuged at 300 g for 5 minutes.
2. The cells were resuspended to 1×10⁵ cells/ml, and plated at a density of 1×10⁴ cells/100 µL/well, and the plates were cultured overnight.
3. PBMCs were isolated from healthy donor 1 (Shanghai Milestone, Cat. No.: PB100C-W, Batch No. A10Z983077) and healthy donor 2 PBMC (Milestone, Cat. No.: PB050C-W, Batch No. P122010104C) using Miltenyi T cell isolation kit (Miltenyi, CaT#: 130-096-535) to obtain human primary T cells.
4. The test article to be tested was formulated at 2×final concentration. The final concentration was 0.02 nM.
5. The media were removed from the experimental wells, and then the human primary T cells from the donor 1 and donor 2 were added to the experimental wells containing 293T-OS8-human B7H4 cells at a density of 2×10⁵ cells/100 µL/well.
6. Then the antibody to be tested was added at 100 µL/well, and two replicates were set up.
7. After 3 days of culture, the supernatant was collected, and the concentration of IFN-gamma was detected by ELISA.

Isotype ADC: an antibody-drug conjugate, in which the antibody is a negative control antibody and the linker-cytotoxin is X2, prepared with reference to DB1001-X2.

### Experimental results

**Table 11 Activation of T cells to secrete IFN-γ by antibody-drug conjugate**

| | Donor 1 IFN-γ (pg/ml) | Donor 2 IFN-γ (pg/ml) | Mean IFN-γ (pg/ml) |
|---|---|---|---|
| Blank control | 1914.7 | 2407.6 | 2161.1 |
| Isotype ADC | 2030.4 | 2111.3 | 2070.8 |
| DB1001-X2 | 6491.1 | 5914.4 | 6202.8 |
| Reference ADC-1 | 4164.0 | 3645.6 | 3904.8 |

### Experimental conclusion

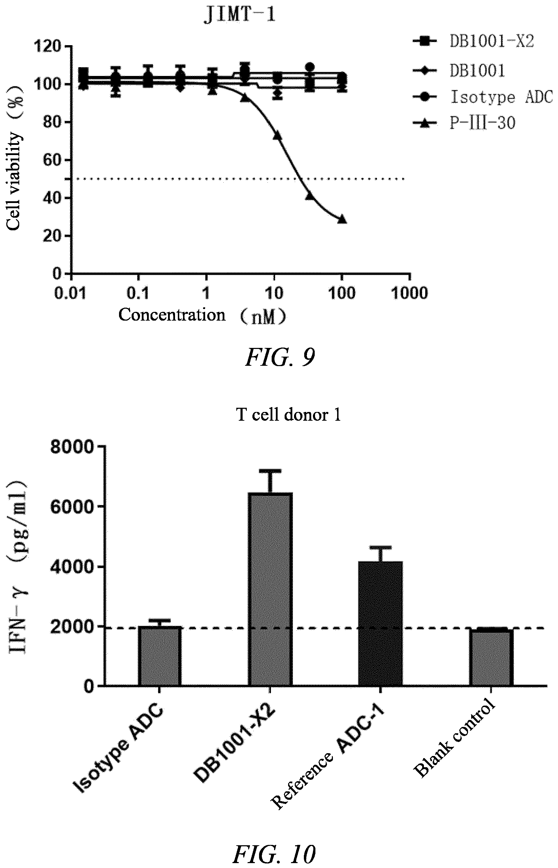

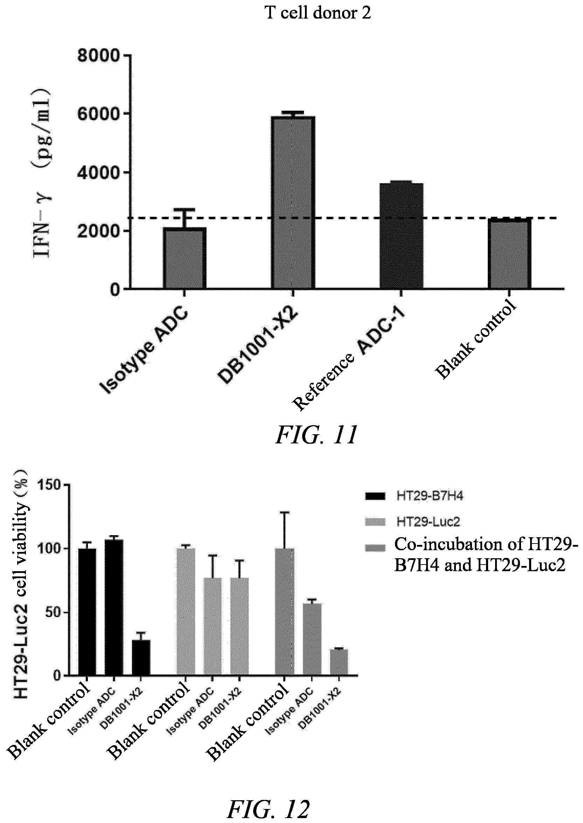

As shown in Table 11, FIG. 10 and FIG. 11, DB1001-X2 can block the inhibition of B7H4-positive cells on T cells of different donor origins, and the blocking effect is significantly superior to that of the reference ADC-1.

### Example 13: Bystander killing effect of antibody-drug conjugate

### Test objective

To detect the bystander effect of the anti-B7H4 antibody-drug conjugate of the present application in comparison with other anti-B7H4 antibody-drug conjugates, the bystander effect being that the small molecule drug released after endocytosis diffuses from B7H4-positive cells to nearby cells negative for B7H4 expression and achieves cell killing on the cells.

Unlabelled B7H4-overexpressing HT29 cells (HT29-B7H4) and luciferase-transfected HT29 cells (HT29-Luc2) that do not express B7H4 were mixed according to a ratio, and then co-incubated with the test article for a period of time. A luciferase substrate was added, and the luciferase content was detected based on the value of luciferin (produced from the substrate under the catalysis of the luciferase produced by live cells). That is, the luciferase content reflects the number of live cells.

### Experimental method

1. The cells in the logarithmic growth phase were collected, and 1640 medium containing 2% serum was added to resuspend the cells and adjust the number of the HT29-B7H4 cells and the HT29-Luc2 cells. The cell suspensions were added to 96-well plates at 45 ul/well; and the plates were cultured overnight in an incubator with 5% CO₂ at 37°C, wherein the ratio of the cells was 3:1, i.e., 11250 HT29-B7H4 cells/well and 3750 HT29-Luc2 cells/well, respectively.
2. The test article solution was formulated at 10×concentration, and 10 µL of the drug solution was added to the cells in each well of 96-well cell plates to achieve a final test article concentration of 10 nM, with 3 replicate wells. The luminescence value at day 0 was detected.
3. The cells in the 96-well plates to which the drug was added were cultured at 37°C and 5% CO₂ for another 7 days.
4. After the incubation was completed, the cell culture plates were taken out and equilibrated to room temperature, 50 µL of CTG detection reagent (Promega, Cat#: G7573) was added to each well where HT29-B7H4 was separately incubated. The plates were shaken and mixed uniformly, and left to stand in the dark for 10 minutes, and then the signal values were read using a microplate reader.
5. An equal volume of Bright-Glo solution was added to each well where separate incubation and co-incubation of HT29-Luc2 were performed, and the cells were lysed by shaking on an orbital shaker for 15 minutes. Then the luminescence values were read by a multifunctional microplate reader.
5. Data analysis: GraphPad Prism software was used. Cell viability calculation formula=(Lum_{drug to be tested}-Lum_{blank control})/(Lum_{solvent blank control}-Lum_{blank control})×100%. Experimental results

**Table 12 Bystander killing effect of antibody-drug conjugate on B7H4-negative HT29-Luc2**

| | HT29-B7H4 cell viability (%) Separate incubation | HT29-Luc2 cell viability (%) Separate incubation | HT29-Luc2 cell viability (%) Co-incubation |
|---|---|---|---|
| Blank control | 100.0 | 100.0 | 100.0 |
| Isotype ADC | 106.9 | 76.9 | 56.7 |
| DB1001-X2 | 28.0 | 76.8 | 20.3 |

Experimental conclusion: as shown in Table 12 and FIG. 12, DB1001-X2 has good bystander killing effects on B7H4-negative cells co-incubated with B7H4-positive cells.

### Example 14: Stability of antibody-drug conjugate in plasma of human, rat and monkey in vitro

### Test objective

To assess the plasma stability of the antibody-drug conjugate after 21 days of *in vitro* incubation in human, rat and monkey plasma, wherein the samples were collected at different time points, and the amount of the small molecule toxin (cytotoxic drug) released in the plasma was measured by liquid chromatography and mass spectrometry.

### Experimental method

DB1001-X2 was diluted with the serum of human, rat or cynomolgus monkey to a final concentration of 150 µg/mL and incubated at 37°C for 21 consecutive days. The intermediate sampling time points were: T0, 2 hours, 8 hours, 1 day, 4 days, 7 days, 14 days, and 21 days. The plasma samples were precipitated with acetonitrile according to a volume ratio of 1: 1, and centrifuged to obtain the supernatant, and then the supernatant was analysed by LC-MS/MS (liquid phase: Thermo Vanquish; triple quadrupole mass spectrometry: Thermo TSQ Quantis). The antibody-drug conjugate release rate was calculated based on the concentration of the cytotoxic drug P-III-30.

### Experimental results

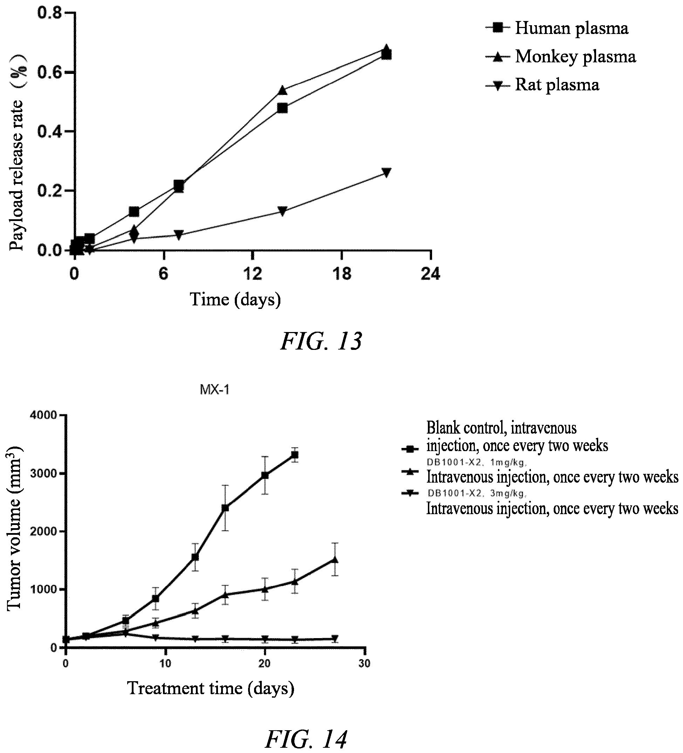

As shown in Table 13 and FIG. 13, after 21 days of incubation, 150 µg/mL DB1001-X2 has a payload (cytotoxic drug) P-III-30 release rate of less than 1% in human, rat and monkey plasma.

**Table 13 Cytotoxic drug P-III-30 release rate in human, rat and monkey plasma after 21 days**

| | Human plasma | Cynomolgus monkey plasma | Rat plasma |
|---|---|---|---|
| P-III-30 release rate (%) | 0.66 | 0.68 | 0.26 |

### Experimental conclusion

DB1001-X2 has good stability in human, rat and monkey plasma *in vitro.*

### Example 15: Pharmacokinetics of antibody-drug conjugate

### Test objective

DB1001-X2 was intravenously administered to cynomolgus monkeys, once every 3 weeks for a total of 2 administrations. To investigate the pharmacokinetic characteristics to provide a reference for subsequent studies.

### Experimental method

4 cynomolgus monkeys (two males and two females, weighed 2.2 to 3.6 kg at the time of grouping) were divided into a total of 2 groups: low-dose DB1001-X2 group and high-dose DB1001-X2 group, and the groups were administered at doses of 30 and 80 mg/kg, respectively, once every 3 weeks for a total of 2 doses. The administration volume was 5 mL/kg.

Sampling time: TK blood samples were collected before the first dose and at 5 min, 0.5 h, 2 h, 4 h, 8 h, 24 h, 48 h, 72 h, 120 h, 168 h, 336 h and 504 h after the first dose; and TK blood samples were collected at 5 min, 0.5 h, 2 h, 4 h, 8 h, 24 h and 48 h after the last dose and before dissection. Sampling method: about 0.5 mL of whole blood was collected from forelimb vein or other appropriate veins.

Treatment of blood samples: after collection, the blood was placed in a labelled blood collection tube containing EDTA•K2 as an anticoagulant. The sample tube was gently inverted several times to ensure uniform mixing, and then immediately placed in wet ice for storage. The sample was centrifuged at 3800 rpm for 10 min at 4°C within 1 h after collection, the plasma was pipetted and sub-packaged in two tubes (100 µL/tube), and the tubes were temporarily stored in dry ice and transferred to an ultra-low temperature refrigerator (-60°C and below) within 4 h. The whole blood collection time and plasma collection time were recorded in the experimental record.

After all samples were collected, they were stored under dry ice conditions and transported to the detection department.

Sample analysis: the blood drug levels of the total antibodies and the ADC drug in the serum were analysed using established ELISA. The blood drug level of the cytotoxic drug P-III-30 in the plasma was analysed by existing LC-MS/MS.

The experimental results are as shown in Table 14.

**Table 14 Pharmacokinetics of antibody-drug conjugate in cynomolgus monkey plasma**

| **Analytes** | **Doses Mpk** | **T_{1/2} (h)** | **Tₘₐₓ (h)** | **Cₘₐₓ (ug/mL)** | **AUC₀₋ₜ (h.ng/mL)** |
|---|---|---|---|---|---|
| Total Abs | 30 | 157.5 | 0.083 | 794377 | 51521254 |
| | 80 | 232 | 0.083 | 2088878 | 174105639 |
| DB1001-X2 | 30 | 164.9 | 0.083 | 828689 | 51863287 |
| | 80 | 218.5 | 0.292 | 2242292 | 183899767 |
| P-III-30 | 30 | 155 | 2 | 4.87 | 236 |
| | 80 | 150.5 | 2 | 12.9 | 875 |

### Experimental conclusion

According to the results in Table 14, no difference in pharmacokinetics is observed between DB1001-X2 and total antibodies, indicating that DB 1001-X2 has good plasma stability *in vivo.* Meanwhile, in cynomolgus monkeys administered at doses of 30 mg/kg and 80 mg/kg, low plasma concentrations of P-III-30 are detected, indicating that due to the stability of DB 1001-X2 and the short systemic half-life of P-III-30, P-III-30 has a low systemic exposure, thereby providing a relatively high level of safety.

### Example 16: Pharmacodynamic evaluation of antibody-drug conjugate in tumour-bearing mice (human breast cancer cells MX-1 with high expression of B7H4)

To investigate the inhibitory effect of DB1001-X2 on the tumours formed *in vivo,* wherein the anti-tumour effects of DB1001-X2 were assessed after transplanted tumours were established in mice with human breast cancer cells MX-1 positive for high B7H4 expression.
1. Test drugs and materials
   Blank control group (control group): Physiological saline
   DB1001-X2 (treatment group): 1 mg/kg
   DB1001-X2 (treatment group): 3 mg/kg
2. Formulation method: all samples were diluted and formulated with physiological saline.
3. Experimental animals: 6-8-week-old female BALB/c Nude mice, purchased from Jicui Gempharmatech Co., Ltd.
4. Experimental method:
   6-8-week-old female BALB/c Nude mice were inoculated subcutaneously on the right back with 5 × 10⁶ MX-1 cells. When the tumour grew to about 160.82 mm³, the tumour-bearing mice were randomly grouped using StudyDirectorTM. On the same day (day 0), the mice were injected intravenously (i.v.) with the test article, once every two weeks for a total of 2 injections (doses: DB1001-X2: 1 mg/kg and 3 mg/kg, respectively, and ISO-ADC: 3 mg/kg). On day 27 after grouping, the experiment reached the endpoint, the tumour volumes and body weights were measured twice a week, and the data were recorded.

There were 5 mice in each control group or treatment group. The tumour inhibition rate was calculated by measuring the tumour volume.

The tumour volume calculation formula is: V = 0.5 *a* × *b²*, where *a* and *b* represent the long and short diameters of the tumour, respectively. The tumour inhibition effect of the compound was evaluated by T/C (%). T/C (%) is an indicator reflecting tumour growth inhibition in percentage, with T and C representing the mean tumour volume on a certain day in the administration group and the control group, respectively. The tumour growth inhibition rate was calculated using the following formula: TGI (%) = [1-(Tᵢ-T₀)/ (Vᵢ-V₀)] × 100, where Tᵢ is the mean tumour volume on a certain day in the administration group, T₀ is the mean tumour volume at the beginning of administration in the same administration group; Vᵢ is the mean tumour volume on a certain day (the same day as Tᵢ) in the vehicle control group, and V₀ is the mean tumour volume at the beginning of administration in the vehicle control group.

Comparisons between two groups of samples were made using an independent samples T-Test, and the data were analysed using SPSS, wherein P < 0.05 indicated significant difference. The data was plotted using GraphPad Prism software.

**Table 15. Evaluation of tumour inhibition efficacy of test articles in subcutaneous xenograft tumour models of MX-1 cell**

| (Calculated based on tumour volume at day 27 after administration) | | | | |
|---|---|---|---|---|
| Group | Tumour volume (mm3) 1 | T/C (%) ² | TGI (%) ² | p value ³ |
| Blank control group, | 3319.54 ± 119.76 | - | - | - |
| DB1001-X2, 1 mg/kg | 1521.41 ± 281.89 | 45.83 | 54.17 | 0.0000800 |
| DB1001-X2, 3 mg/kg | 154.12 ± 60.47 | 4.64 | 95.36 | 1.44e-8 |

| | | | | |
|---|---|---|---|---|
| 1. Tumour volume is expressed as mean ± standard error; 2. Tumour growth inhibition is reflected by T/C (T/C (%) = T₂₇/ V₂₇ ×100) and TGI (TGI (%) = [1-(T₂₇-T₀)/ (V₂₇-V₀)] ×100); 3. The *p* value is calculated based on the tumour volume (*p <* 0.05 indicates statistical difference, p < 0.01 indicates significant difference). | | | | |

The experimental results are as shown in FIG. 14 and Table 15. The antibody-drug conjugate DB1001-X2 exhibits significant dose-dependent tumour inhibitory activity after administration.

### Example 17: Pharmacodynamic evaluation of antibody-drug conjugate in tumour-bearing mice (human B7H4-positive breast cancer cells MCF-7)

To compare DB1001-X2 with similar competing products in terms of the inhibitory effect on the tumours formed *in vivo,* wherein the anti-tumour effects of DB1001-X2 were assessed after transplanted tumours were established in mice with human breast cancer cells MCF-7 positive for B7H4 expression.
1. Test drugs and materials
   Blank control group (control group): Physiological saline
   DB1001-X2 (treatment group): 3 mg/kg
2. Formulation method: all samples were diluted and formulated with physiological saline.
3. Experimental animals: 6-7-week-old female NCG mice, from Jiangsu Jicui Gempharmatech Co., Ltd.
4. Experimental method:
   MCF-7 cells (inoculation passage N+12) in the logarithmic growth phase were collected, the medium was removed, and the cells were washed twice with PBS and then inoculated (cell viability before and after tumour model establishment: 96.03% and 94.81%), wherein the inoculation amount was 1×10⁷ cells/75 µL/mouse (a matrigel was added at a ratio of 1:1). On day 19 after inoculation (when the mean tumour volume reached 107.44 mm³), 50 mice were selected and randomly divided into 10 groups based on the tumour volume, with 5 mice in each group. The day of grouping was defined as D0, and dosing was initiated on the day of grouping (D0). The remaining mice after grouping were subjected to euthanasia treatment. The mice were injected with the test article at a dose of 3 mg/kg for a total of 1 injection. The tumour volumes and body weights were measured twice a week, and the data were recorded.

There were 5 mice in each vehicle control group or treatment group. On day 25 after grouping, the experiment reached the endpoint and the tumour inhibition rate was calculated by measuring the tumour volume.

The tumour volume calculation formula is: V = 0.5 *a* × *b²*, where *a* and *b* represent the long and short diameters of the tumour, respectively. The tumour inhibition effect of the compound was evaluated by T/C (%). T/C (%) is an indicator reflecting tumour growth inhibition in percentage, with T and C representing the mean tumour volume on a certain day in the administration group and the control group, respectively. The tumour growth inhibition rate was calculated using the following formula: TGI (%) = [1-(Tᵢ-T₀)/ (Vᵢ-V₀)] × 100, where Tᵢ is the mean tumour volume on a certain day in the administration group, T₀ is the mean tumour volume at the beginning of administration in the same administration group; Vᵢ is the mean tumour volume on a certain day (the same day as Tᵢ) in the vehicle control group, and V₀ is the mean tumour volume at the beginning of administration in the vehicle control group.

Comparisons between two groups of samples were made using an independent samples T-Test, and the data were analysed using SPSS, wherein P < 0.05 indicated significant difference. The data was plotted using GraphPad Prism software.

Experimental results: The antibody-drug conjugate DB1001-X2 of the present invention exhibits significant dose-dependent tumour inhibitory activity after a single dose.

### Example 18: Pharmacodynamic evaluation of antibody-drug conjugate in tumour-bearing mice (human endometrial carcinoma cells RL95-2 with low expression of B7H4)

To investigate the inhibitory effect of DB1001-X2 on the tumours formed *in vivo,* wherein the anti-tumour effects of DB1001-X2 were assessed after transplanted tumours were established in mice with human endometrial carcinoma cells RL95-2 positive for low B7H4 expression.
1. Test drugs and materials
   Blank control group (control group): phosphate buffered saline (DPBS)
   DB1001-X2 (treatment group): 1 mg/kg
   DB1001-X2 (treatment group): 3 mg/kg
   DB1001-X2 (treatment group): 10 mg/kg
2. Formulation method: all samples were diluted and formulated with phosphate buffered saline (DPBS).
3. Experimental animals: 6-8-week-old female BALB/c Nude mice, purchased from Zhejiang Vital River Laboratory Animal Technology Co., Ltd.
4. Experimental method:
   RL95-2 cells in the logarithmic growth phase were collected, and each mouse was inoculated subcutaneously on the right nape with 5×106 RL95-2 cells, wherein the inoculation volume was 0.2 mL, and the cell suspension was prepared with PBS plus matrigel (volume ratio: 1:1). *In vivo* efficacy experiment: On day 20 after cell inoculation (when the mean tumour volume reached 134 mm³), the mice were randomly grouped (5 mice/group) based on the tumour volume and administered with the drug. The day of grouping was defined as D0, and dosing was initiated on the day of grouping (D0). The mice were injected with the test article once every two weeks at a dose of 3 mg/kg for a total of 2 injections. On day 28 after grouping, the experiment reached the endpoint, the tumour volumes and body weights were measured twice a week, and the data were recorded.

The tumour volume calculation formula is: V = 0.5 *a* × *b²*, where *a* and *b* represent the long and short diameters of the tumour, respectively. The tumour inhibition effect of the compound was evaluated by T/C (%). T/C (%) is an indicator reflecting tumour growth inhibition in percentage, with T and C representing the mean tumour volume on a certain day in the administration group and the control group, respectively. The tumour growth inhibition rate was calculated using the following formula: TGI (%) = [1-(Tᵢ-T₀)/ (Vᵢ-V₀)] × 100, where Tᵢ is the mean tumour volume on a certain day in the administration group, T₀ is the mean tumour volume at the beginning of administration in the same administration group; Vᵢ is the mean tumour volume on a certain day (the same day as Tᵢ) in the vehicle control group, and V₀ is the mean tumour volume at the beginning of administration in the vehicle control group.

Statistical analysis: On the basis of the data from day 28 after grouping, statistical analysis was performed using GraphPad Prism software to assess the differences between groups. Comparisons between three or more groups were analysed using one-way ANOVA (Dunnett's multiple comparison test and Tukey's multiple comparison test). p < 0.05 was considered significant difference.

**Table 16. Evaluation of tumour inhibition efficacy of test articles in subcutaneous xenograft tumour models of RL95-2 cell**

| (Calculated based on tumour volume at day 28 after administration) | | | | |
|---|---|---|---|---|
| Group | Tumour volume (mm³) ¹ | T/C (%) ² | TGI (%) ² | p value ³ |
| Blank control group, | 1050 ± 197 | | | |
| DB1001-X2, 1 mg/kg | 593 ± 79 | 57.86 | 49.92 | * |
| DB1001-X2, 3 mg/kg | 258 ± 33 | 24.06 | 86.52 | **** |
| DB1001-X2, 10 mg/kg | 74 ± 5 | 7.05 | 106.59 | **** |

| | | | | |
|---|---|---|---|---|
| 1. Tumour volume is expressed as mean ± standard error; 2. Tumour growth inhibition is reflected by T/C (T/C (%) = T₂₈/ V₂₈ ×100) and TGI (TGI (%) = [1-(T₂₈-T₀)/ (V₂₈-V₀)] ×100); 3. The *p* value is calculated based on the tumour volume (**** *indicates p* < *0.0001,* * *indicates p < 0.05*)*.* | | | | |

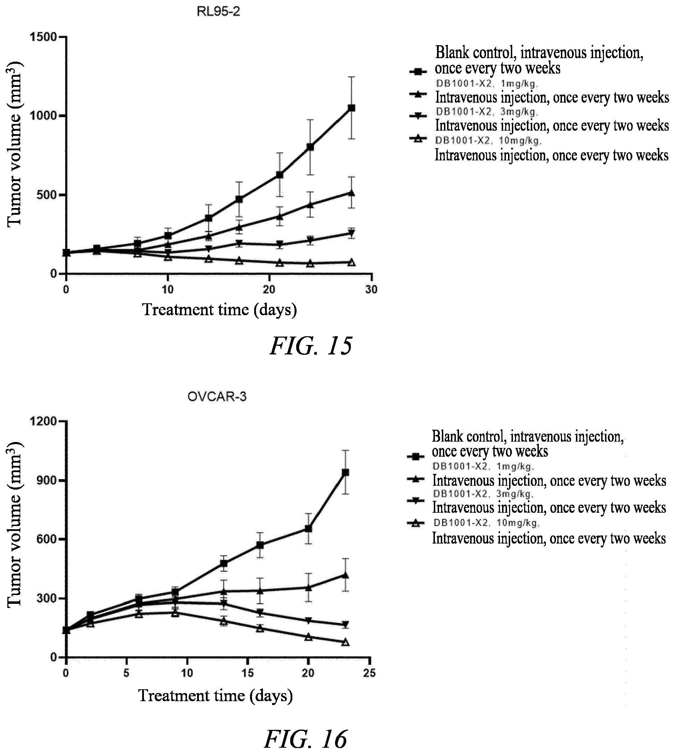

The experimental results are as shown in FIG. 15 and Table 16. The antibody-drug conjugate DB1001-X2 exhibits significant dose-dependent tumour inhibitory activity after administration.

### Example 19: Pharmacodynamic evaluation of antibody-drug conjugate in tumour-bearing mice (human ovarian cancer cells OVCAR-3 with low expression of B7H4)

To investigate the inhibitory effect of DB1001-X2 on the tumours formed *in vivo,* wherein the anti-tumour effects of DB1001-X2 were assessed after transplanted tumours were established in mice with human ovarian cancer cells OVCAR-3 positive for low B7H4 expression.
1. Test drugs and materials
   Blank control group (control group): Physiological saline
   DB1001-X2 (treatment group): 1 mg/kg
   DB1001-X2 (treatment group): 3 mg/kg
   DB1001-X2 (treatment group): 10 mg/kg
2. Formulation method: all samples were diluted and formulated with physiological saline.
3. Experimental animals: 6-8-week-old female BALB/c Nude mice, purchased from Jicui Gempharmatech Co., Ltd.
4. Experimental method:
   6-8-week-old female BALB/c Nude mice were inoculated subcutaneously on the right back with 1 × 10⁷ OVCAR-3 cells (0.2 ml/mouse, the cells were resuspended in a 1 : 1 PBS plus matrigel). When the tumour grew to about 142.85 mm³, the tumour-bearing mice were randomly grouped using StudyDirectorTM. On the same day (day 0), the mice were injected intravenously (i.v.) with the test article once every two weeks for a total of 2 injections (doses: DB1001-X2: 1 mg/kg, 3 mg/kg and 10 mg/kg, respectively). On day 27 after grouping, the experiment reached the endpoint, the tumour volumes and body weights were measured twice a week, and the data were recorded.

There were 5 mice in each control group or treatment group. The tumour inhibition rate was calculated by measuring the tumour volume.

The tumour volume calculation formula is: V = 0.5 *a* × *b²*, where *a* and *b* represent the long and short diameters of the tumour, respectively. The tumour inhibition effect of the compound was evaluated by T/C (%). T/C (%) is an indicator reflecting tumour growth inhibition in percentage, with T and C representing the mean tumour volume on a certain day in the administration group and the control group, respectively. The tumour growth inhibition rate was calculated using the following formula: TGI (%) = [1-(Tᵢ-T₀)/ (Vᵢ-V₀)] × 100, where Tᵢ is the mean tumour volume on a certain day in the administration group, T₀ is the mean tumour volume at the beginning of administration in the same administration group; Vᵢ is the mean tumour volume on a certain day (the same day as Tᵢ) in the vehicle control group, and V₀ is the mean tumour volume at the beginning of administration in the vehicle control group.

Comparisons between two groups of samples were made using an independent samples T-Test, and the data were analysed using SPSS, wherein P < 0.05 indicated significant difference. The data was plotted using GraphPad Prism software.

**Table 17. Evaluation of tumour inhibition efficacy of test articles in subcutaneous xenograft tumour models of OVCAR-3 cell**

| (Calculated based on tumour volume at day 27 after administration) | | | | |
|---|---|---|---|---|
| Group | Tumour volume (mm³) ¹ | T/C (%) ² | TGI (%) ² | p value ³ |
| Blank control group, | 1144.84 ± 144.07 | | | |
| DB1001-X2, 1 mg/kg | 390.93 ± 85.40 | 34.15 | 65.85 | 0.0132 |
| DB1001-X2, 3 mg/kg | 122.94 ± 17.80 | 10.74 | 89.26 | 0.0000534 |
| DB1001-X2, 10 mg/kg | 47.72 ± 7.95 | 4.17 | 95.83 | 5.14e-10 |

| | | | | |
|---|---|---|---|---|
| 1. Tumour volume is expressed as mean ± standard error; 2. Tumour growth inhibition is reflected by T/C (T/C (%) = T₂₇/ V₂₇ ×100) and TGI (TGI (%) = [1-(T₂₇-T₀)/ (V₂₇-V₀)] × 100); 3. The *p* value is calculated based on the tumour volume (*p <* 0.05 indicates statistical difference, p < 0.01 indicates significant difference). | | | | |

The experimental results are as shown in FIG. 16 and Table 17. The antibody-drug conjugate DB1001-X2 exhibits significant dose-dependent tumour inhibitory activity after administration.

Although the specific embodiments of the present invention have been described above, it will be understood by those skilled in the art that these are merely illustrative, and that various alterations or modifications can be made to these embodiments without departing from the principle and essence of the present invention. Therefore, the scope of protection of the present invention is defined by the appended claims.

## Claims

1. An anti-B7H4 antibody-drug conjugate, an isomer thereof, a pharmaceutically acceptable salt thereof or a mixture thereof, **characterised in that** the structure of the anti-B7H4
antibody-drug conjugate is shown as formula (I-1): 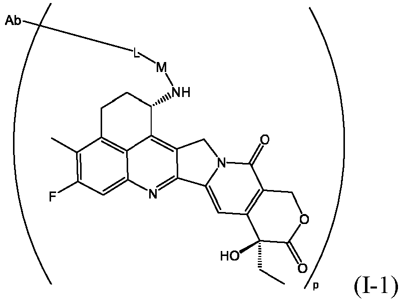
wherein,
M is -L²-L¹-C(O)-;
L² is -O- or -S-;
L¹ is -(C(R^{1a})(R^{1b}))ₘ-CH₂-, saturated C₃-C₆ cycloalkyl or 3- to 6-membered saturated heterocyclyl, wherein the saturated C₃-C₆ cycloalkyl and 3- to 6-membered saturated heterocyclyl are each independently and optionally substituted with one or more R^{2a};
m is selected from 1, 2, 3 or 4; the 3- to 6-membered saturated heterocyclyl has 1-3 heteroatoms selected from N, O and S;
each R^{1a} is independently selected from hydrogen, halogen, hydroxyl, amino and C₁-C₆ alkyl, wherein the C₁-C₆ alkyl is optionally substituted with one or more R;
R^{1b} and R^{2a} are each independently selected from hydrogen, halogen, hydroxyl, amino and C₁-C₆ alkyl, wherein the C₁-C₆ alkyl is optionally substituted with one or more R;
each R is independently hydrogen or halogen;
L is a linker unit;
p represents the average connection number, and p is an integer or decimal selected from 1 to 10;
Ab is an anti-B7H4 antibody or an antigen-binding fragment thereof.

2. The anti-B7H4 antibody-drug conjugate, the isomer thereof, the pharmaceutically acceptable salt thereof or the mixture thereof according to claim 1, **characterised in that** the anti-B7H4 antibody or the antigen-binding fragment thereof comprises a heavy chain variable region and a light chain variable region, the heavy chain variable region comprises HCDR1, HCDR2 and HCDR3 having the amino acid sequences as shown in SEQ ID NO:1, SEQ ID NO:2 and SEQ ID NO:3, respectively, and the light chain variable region comprises LCDR1, LCDR2 and LCDR3 having the amino acid sequences as shown in SEQ ID NO:4, SEQ ID NO:5 and SEQ ID NO:6, respectively.

3. The anti-B7H4 antibody-drug conjugate, the isomer thereof, the pharmaceutically acceptable salt thereof or the mixture thereof according to claim 1 or 2, **characterised in that** the anti-B7H4 antibody or the antigen-binding fragment thereof comprises: a heavy chain variable region with an amino acid sequence as shown in SEQ ID NO:7 or an amino acid sequence having at least 95%, 96%, 97%, 98% or 99% identity to SEQ ID NO:7, and a light chain variable region with an amino acid sequence as shown in SEQ ID NO:8 or an amino acid sequence having at least 95%, 96%, 97%, 98% or 99% identity to SEQ ID NO:8.

4. The anti-B7H4 antibody-drug conjugate, the isomer thereof, the pharmaceutically acceptable salt thereof or the mixture thereof according to claim 1 or 2, **characterised in that** the anti-B7H4 antibody or the antigen-binding fragment thereof is a murine antibody or a fragment thereof, a chimeric antibody or a fragment thereof, or a humanized antibody or a fragment thereof.

5. The anti-B7H4 antibody-drug conjugate, the isomer thereof, the pharmaceutically acceptable salt thereof or the mixture thereof according to claim 1 or 2, **characterised in that** the anti-B7H4 antibody or the antigen-binding fragment thereof is selected from Fab, Fab', Fab'-SH, Fv, scFv, F(ab')₂, sdAb, diabodies or linear antibodies.

6. The anti-B7H4 antibody-drug conjugate, the isomer thereof, the pharmaceutically acceptable salt thereof or the mixture thereof according to claim 1 or 2, **characterised in that** the antibody is an antibody in the form of IgG1, IgG2, IgG3 or IgG4.

7. The anti-B7H4 antibody-drug conjugate, the isomer thereof, the pharmaceutically acceptable salt thereof or the mixture thereof according to claim 1 or 2, **characterised in that** the anti-B7H4 antibody or the antigen-binding fragment thereof comprises: a heavy chain with an amino acid sequence as shown in SEQ ID NO: 9 or an amino acid sequence having at least 95%, 96%, 97%, 98% or 99% identity to SEQ ID NO: 9, and a light chain with an amino acid sequence as shown in SEQ ID NO: 10 or an amino acid sequence having at least 95%, 96%, 97%, 98% or 99% identity to SEQ ID NO: 10.

8. The anti-B7H4 antibody-drug conjugate, the isomer thereof, the pharmaceutically acceptable salt thereof or the mixture thereof according to any one of claims 1-7, **characterised in that** p is an integer or decimal from 3 to 8.

9. The anti-B7H4 antibody-drug conjugate, the isomer thereof, the pharmaceutically acceptable salt thereof or the mixture thereof according to any one of claims 1-7, **characterised in that** L¹ is -(C(R^{1a})(R^{1b}))ₘ-CH₂-; R^{1a} is selected from: hydrogen, halogen and C₁-C₆ alkyl; R^{1b} is selected from: hydrogen, halogen and C₁-C₆ alkyl.

10. The anti-B7H4 antibody-drug conjugate, the isomer thereof, the pharmaceutically acceptable salt thereof or the mixture thereof according to claim 9, **characterised in that** L¹ is -(C(R^{1a})(R^{1b}))ₘ-CH₂-; R^{1a} is -CH₃; R^{1b} is selected from: hydrogen and -CH₃.

11. The anti-B7H4 antibody-drug conjugate, the isomer thereof, the pharmaceutically acceptable salt thereof or the mixture thereof according to any one of claims 1-10, **characterised in that** L¹ is -(C(R^{1a})(R^{1b}))ₘ-CH₂-; m is 1 or 2.

12. The anti-B7H4 antibody-drug conjugate, the isomer thereof, the pharmaceutically acceptable salt thereof or the mixture thereof according to any one of claims 1-11, **characterised in that** L¹ is selected from:

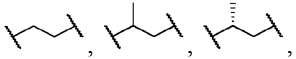

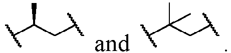

13. The anti-B7H4 antibody-drug conjugate, the isomer thereof, the pharmaceutically acceptable salt thereof or the mixture thereof according to any one of claims 1-7, **characterised in that** L¹ is saturated C₃-C₆ cycloalkyl or 3- to 6-membered saturated heterocyclyl, wherein the saturated C₃-C₆ cycloalkyl and 3- to 6-membered saturated heterocyclyl are each independently and optionally substituted with one or more R^{2a}, and each R^{2a} is independently selected from: hydrogen, halogen and C₁-C₆ alkyl.

14. The anti-B7H4 antibody-drug conjugate, the isomer thereof, the pharmaceutically acceptable salt thereof or the mixture thereof according to claim 13, **characterised in that** L¹ is saturated C₃-C₆ cycloalkyl optionally substituted with one or more R^{2a}; each R^{2a} is independently selected from: hydrogen, halogen and C₁-C₆ alkyl.

15. The anti-B7H4 antibody-drug conjugate, the isomer thereof, the pharmaceutically acceptable salt thereof or the mixture thereof according to claim 14, **characterised in that** L¹ is 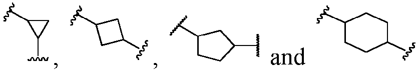 optionally substituted with 1, 2 or 3 R^{2a}; each R^{2a} is independently selected from: hydrogen, halogen and C₁-C₆ alkyl.

16. The anti-B7H4 antibody-drug conjugate, the isomer thereof, the pharmaceutically acceptable salt thereof or the mixture thereof according to any one of claims 1-15, **characterised in that** L¹ is selected from:

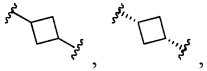

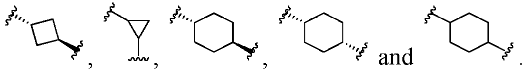

17. The anti-B7H4 antibody-drug conjugate, the isomer thereof, the pharmaceutically acceptable salt thereof or the mixture thereof according to any one of claims 1-16, **characterised in that**
M is -L²-L¹-C(O)-;
L² is -O-;
L¹ is -(C(R^{1a})(R^{1b}))ₘ-CH₂- or saturated C₃-C₆ cycloalkyl, wherein the saturated C₃-C₆ cycloalkyl is optionally substituted with 1, 2 or 3 R^{2a};
m is selected from 1 or 2;
each R^{1a} is independently selected from hydrogen, halogen and C₁-C₆ alkyl, wherein the C₁-C₆ alkyl is optionally substituted with one or more R;
R^{1b} and R^{2a} are each independently selected from hydrogen, halogen and C₁-C₆ alkyl, wherein the C₁-C₆ alkyl is optionally substituted with one or more R;
each R is independently hydrogen or halogen.

18. The anti-B7H4 antibody-drug conjugate, the isomer thereof, the pharmaceutically acceptable salt thereof or the mixture thereof according to claim 17, **characterised in that**
M is -L²-L¹-C(O)-;
L² is -O-;
L¹ is -(C(R^{1a})(R^{1b}))ₘ-CH₂-, or 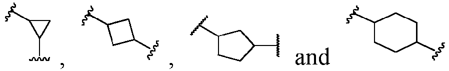 optionally substituted with 1, 2 or 3 R^{2a};
m is selected from 1 or 2;
each R^{1a} is independently selected from halogen and C₁-C₆ alkyl, wherein the C₁-C₆ alkyl is optionally substituted with one or more R;
R^{1b} and R^{2a} are each independently selected from hydrogen, halogen and C₁-C₆ alkyl, wherein the C₁-C₆ alkyl is optionally substituted with one or more R;
each R is independently hydrogen or halogen.

19. The anti-B7H4 antibody-drug conjugate, the isomer thereof, the pharmaceutically acceptable salt thereof or the mixture thereof according to any one of claims 1-18, **characterised in that** the -M- is selected from:

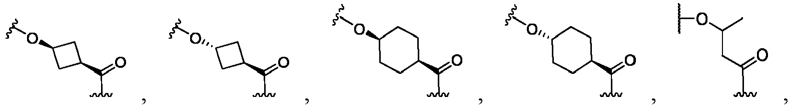

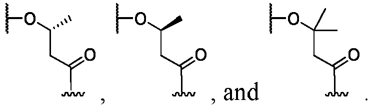

20. The anti-B7H4 antibody-drug conjugate, the isomer thereof, the pharmaceutically acceptable salt thereof or the mixture thereof according to any one of claims 1-19, **characterised in that** the L is -Lₐ-L_{b}-L_{c}-,
the -Lₐ- is 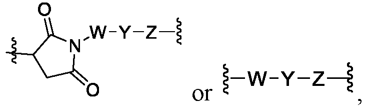
wherein, W is -(C(R^{wa})(R^{wb}))_{wn}-, Y is -(OCH₂CH₂)_{yn}-O_{yp}-, and Z is - (C(R^{za})(R^{zb}))_{zn},
wherein wn is 1, 2, 3 or 6,
0 or 1 methylene unit of W is each independently replaced with -Cyr-, - N(R^{wx})C(O)-, -C(O)N(R^{wx})-, or -C(O)-,
yn is 0, 4 or 8, yp is 0 or 1;
zn is 1, 2 or 3,
1 methylene unit of Z is each independently replaced with -Cyr-, -N(R^{zw})C(O)-, -C(O)N(R^{zx})-, or -C(O)-;
-Cyr- is 3- to 10-membered saturated cycloalkyl, and the -Cyr- is unsubstituted or independently substituted with 1 to 3 substituents R^{cx};
R^{wa}, R^{wb}, R^{za}, R^{zb}, R^{wx}, R^{zx}, and R^{cx} are each independently hydrogen, halogen, or -OR^{r}, or C₁₋₆ alkyl optionally substituted with R^{r},
wherein each R^{r} is independently hydrogen, halogen or C₁₋₆ alkyl;
the -L_{b}- represents a peptide residue composed of 2 to 4 amino acids, and the peptide residue of the -L_{b}- is a peptide residue formed by the amino acids selected from the following group: phenylalanine, glycine, alanine, valine, citrulline and lysine;
the -L_{c}- is 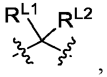
wherein R^{L1} and R^{L2} are each independently selected from the following group: hydrogen, halogen, -OH and C₁₋₆ alkyl.

21. The anti-B7H4 antibody-drug conjugate, the isomer thereof, the pharmaceutically acceptable salt thereof or the mixture thereof according to claim 20, **characterised in that** the -Lₐ- is

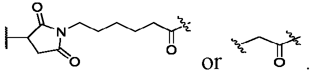

22. The anti-B7H4 antibody-drug conjugate, the isomer thereof, the pharmaceutically acceptable salt thereof or the mixture thereof according to claim 20, **characterised in that** the -L_{b}- is selected from the following group: 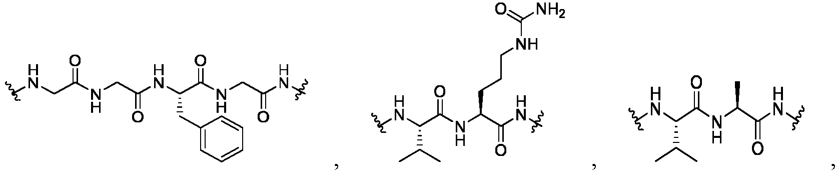 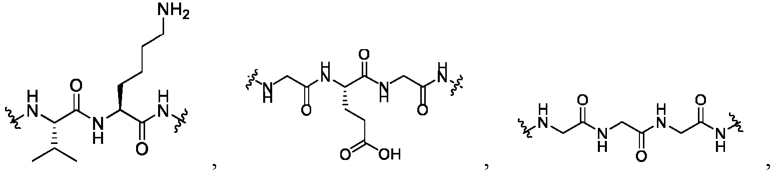 and 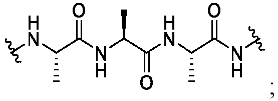 preferably, the -L_{b}- is

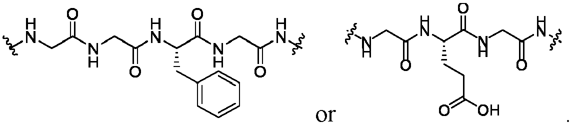

23. The anti-B7H4 antibody-drug conjugate, the isomer thereof, the pharmaceutically acceptable salt thereof or the mixture thereof according to claim 20, **characterised in that** the -L_{c}- is

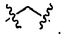

24. The anti-B7H4 antibody-drug conjugate, the isomer thereof, the pharmaceutically acceptable salt thereof or the mixture thereof according to any one of claims 20-23, **characterised in that** the L is

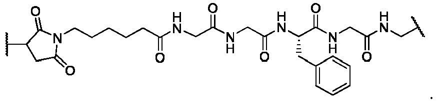

25. The anti-B7H4 antibody-drug conjugate, the isomer thereof, the pharmaceutically acceptable salt thereof or the mixture thereof according to any one of claims 1-24, **characterised in that** the structure of the anti-B7H4 antibody-drug conjugate is shown as formula (II-1) or (II-2): 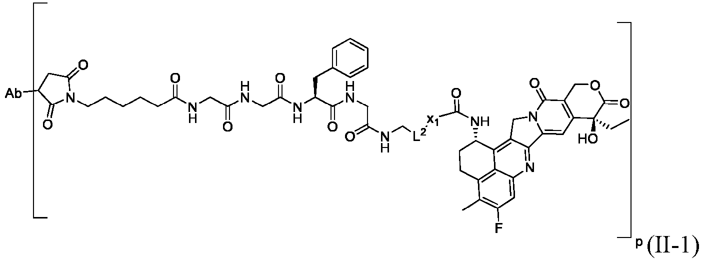 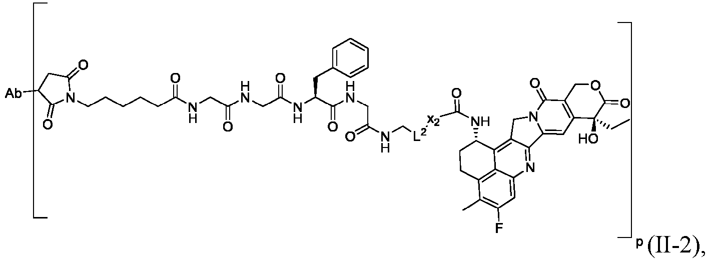 wherein,
p represents the average connection number, and p is an integer or decimal selected from 1 to 10, preferably an integer or decimal from 3 to 8;
Ab is as defined in any one of claims 1-7;
L² is -O- or -S-, preferably -O-;
X₁ is selected from saturated C₃-C₆ cycloalkyl optionally substituted with 1, 2 or 3 R^{2a}, preferably 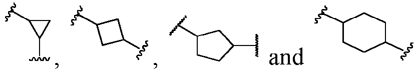
optionally substituted with 1, 2 or 3 R^{2a};
X₂ is selected from -(C(R^{1a})(R^{1b}))ₘ-CH₂-;
m is selected from 1 or 2;
R^{1a} is hydrogen or halogen, or C₁-C₆ alkyl optionally substituted with 1, 2 or 3 R;
R^{1b} and R^{2a} can each independently be hydrogen or halogen, or C₁-C₆ alkyl optionally substituted with 1, 2 or 3 R;
each R can independently be hydrogen or halogen.

26. The anti-B7H4 antibody-drug conjugate, the isomer thereof, the pharmaceutically acceptable salt thereof or the mixture thereof according to any one of claims 1-25, **characterised in that** the anti-B7H4 antibody-drug conjugate is selected from the following structural formulas: 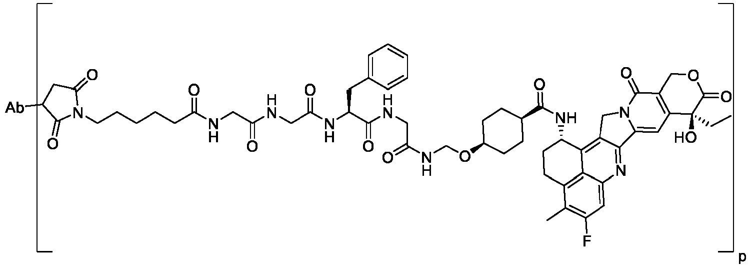 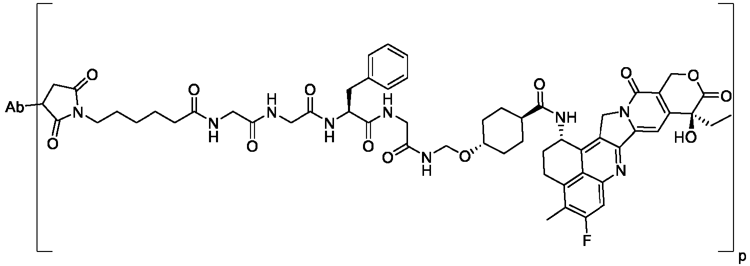 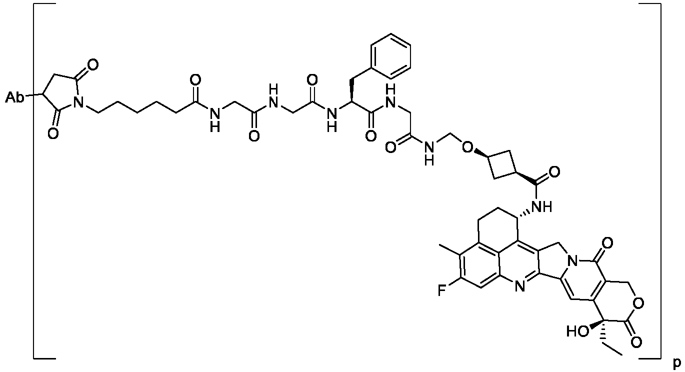 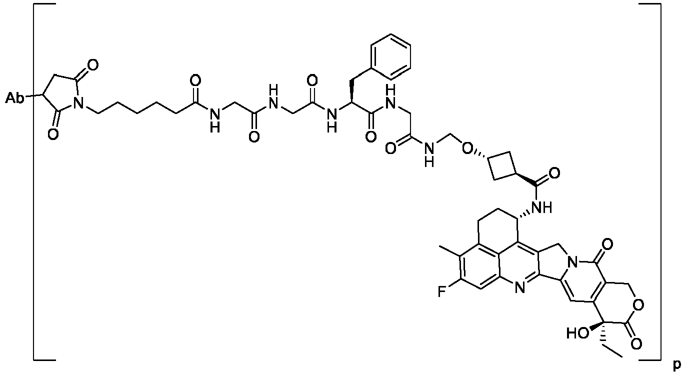 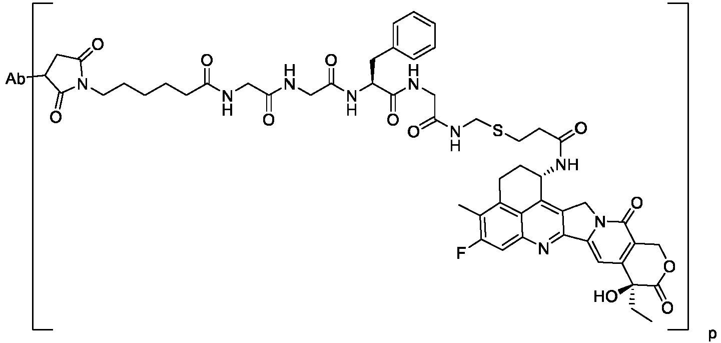 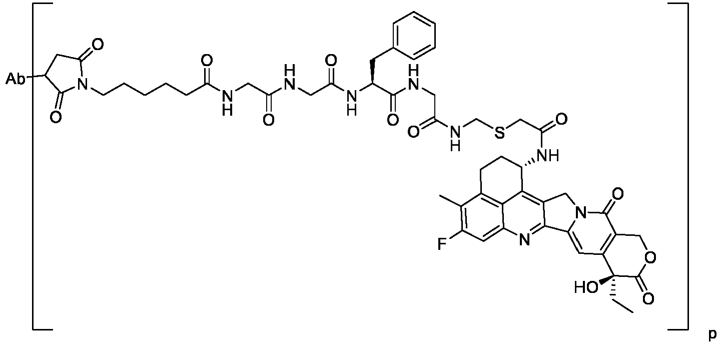 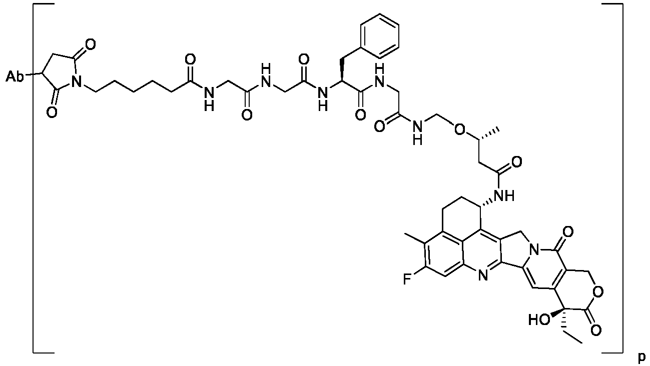 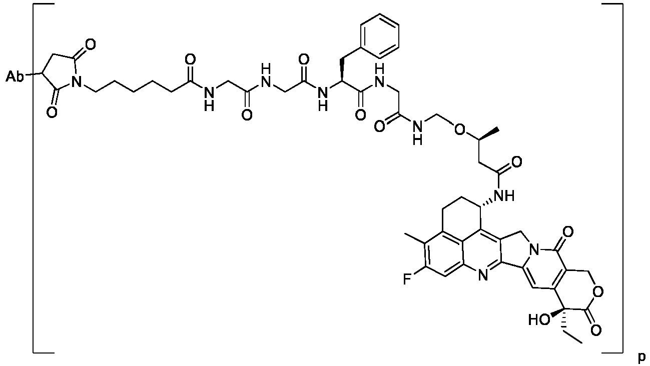 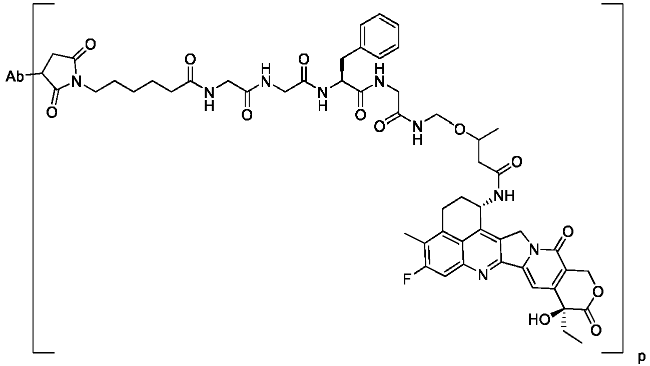 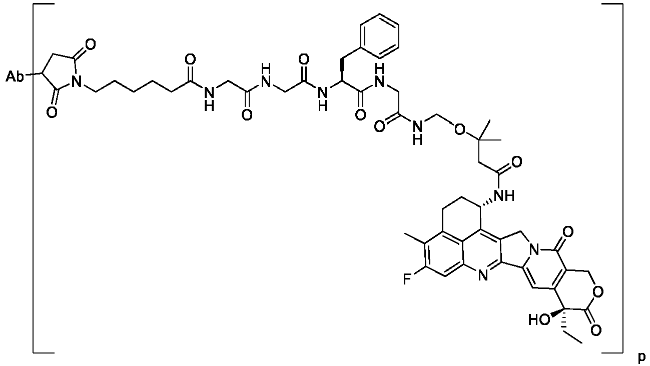 wherein,
p is as defined in claim 1 or 8;
Ab is as defined in any one of claims 1-7.

27. An anti-B7H4 antibody-drug conjugate, an isomer thereof, a pharmaceutically acceptable salt thereof or a mixture thereof, **characterised in that** the anti-B7H4 antibody-drug conjugate is selected from: 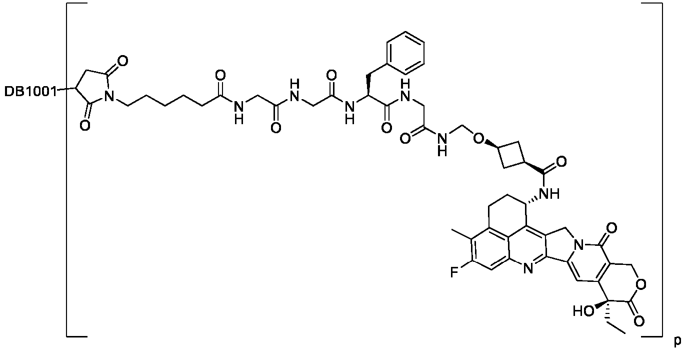 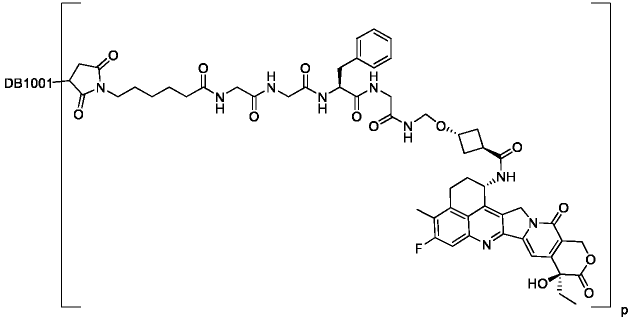 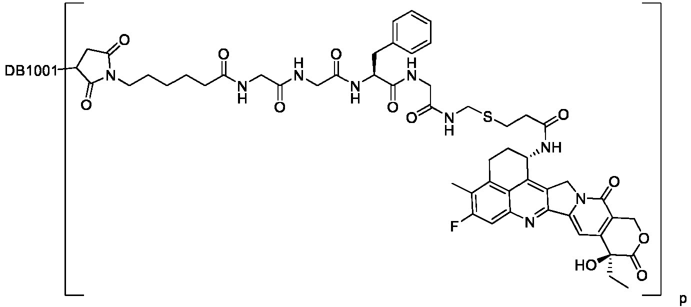 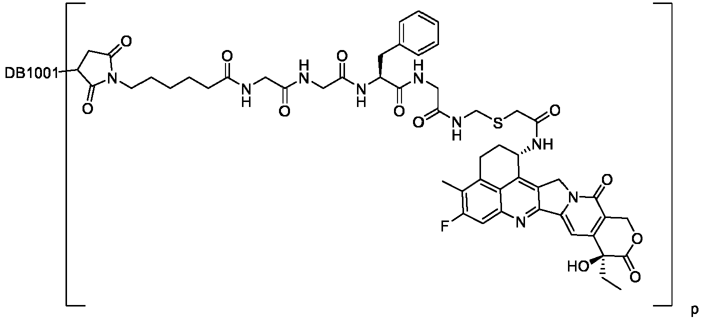 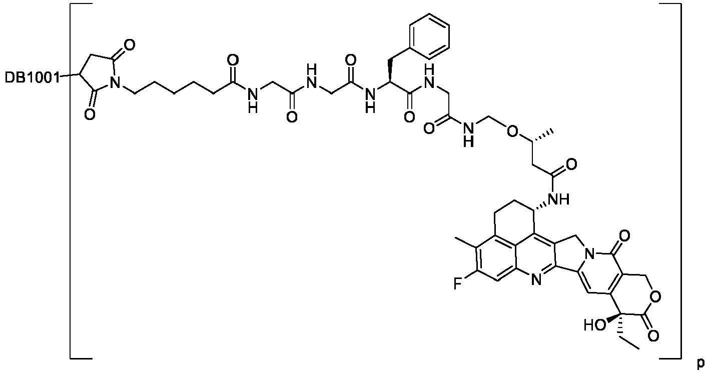 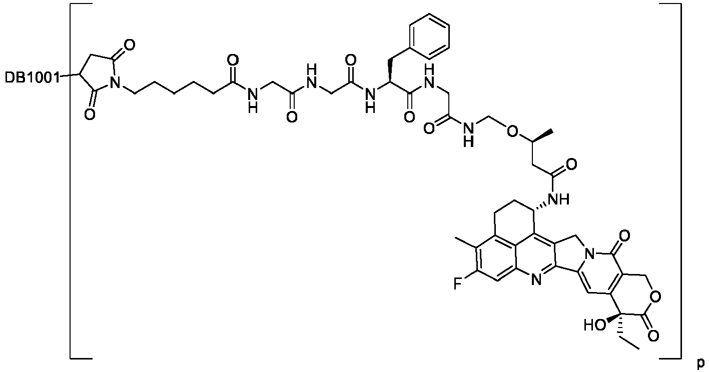 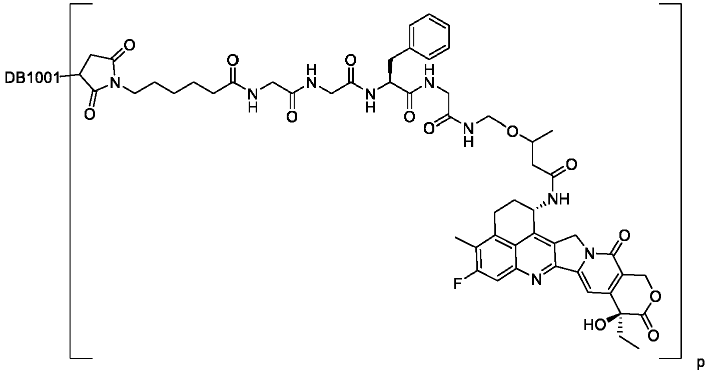 wherein,
p represents the average connection number, and p is an integer or decimal selected from 1 to 10, preferably an integer or decimal from 3 to 8.

28. A pharmaceutical composition, **characterised in that** the pharmaceutical composition comprises the anti-B7H4 antibody-drug conjugate, the isomer thereof, the pharmaceutically acceptable salt thereof or the mixture thereof according to any one of claims 1-27, and a pharmaceutically acceptable carrier or excipient.

29. Use of the anti-B7H4 antibody-drug conjugate, the isomer thereof, the pharmaceutically acceptable salt thereof or the mixture thereof according to any one of claims 1-27, or the pharmaceutical composition according to claim 28 in the preparation of a medicament for the treatment and/or prevention of a disease or condition mediated by B7H4, **characterised in that** the disease or condition is preferably cancer positive for B7H4 expression; more preferably, the cancer is selected from breast cancer, ovarian cancer and endometrial tumour.

30. A method for treating and/or preventing a disease or condition mediated by B7H4, **characterised in that** the method comprises: administering the anti-B7H4 antibody-drug conjugate, the isomer thereof, the pharmaceutically acceptable salt thereof or the mixture thereof according to any one of claims 1-27, or the pharmaceutical composition according to claim 28 to a subject in need thereof, wherein the disease or condition is preferably cancer positive for B7H4 expression; more preferably, the cancer is selected from breast cancer, ovarian cancer and endometrial tumour.
